(19) Europäisches Patentamt
European Patent Office
Office européen des brevets

(11) EP 1 368 462 B1

(12) **EUROPEAN PATENT SPECIFICATION**

(45) Date of publication and mention of the grant of the patent:
**01.08.2007 Bulletin 2007/31**

(21) Application number: **02705889.0**

(22) Date of filing: **22.01.2002**

(51) Int Cl.:
***C12N 9/16*** *(2006.01)*

(86) International application number:
**PCT/US2002/001783**

(87) International publication number:
**WO 2002/068619 (06.09.2002 Gazette 2002/36)**

(54) **BFIT (BROWN FAT INDUCIBLE THIOESTERASE) POLYPEPTIDES AND POLYNUCLEOTIDES AND THEIR USE**

BFIT (BROWN FAT INDUCIBLE THIOESTERASE) POLYPEPTIDE UND POLYNUKLEOTIDE UND IHRE VERWENDUNG

POLYPEPTIDES ET POLYNUCLEOTIDES BFIT (BROWN FAT INDUCIBLE THIOESTERASE) ET LEUR UTILISATION

(84) Designated Contracting States:
**AT BE CH CY DE DK ES FI FR GB GR IE IT LI LU MC NL PT SE TR**

(30) Priority: **22.01.2001 US 263362 P**

(43) Date of publication of application:
**10.12.2003 Bulletin 2003/50**

(73) Proprietors:
- **Curagen Corporation**
  **New Haven, CT 06511 (US)**
- **GENENTECH, INC.**
  **South San Francisco**
  **California 94080 (US)**

(72) Inventors:
- **LEWIN, David**
  **New Haven, CT 06511 (US)**
- **GODDARD, Audrey, D.**
  **San Francisco, CA 94127 (US)**
- **GRIMALDI, J., Christopher**
  **San Francisco, CA 94122 (US)**
- **CHUI, Clarissa, J.**
  **San Francisco, CA 94131 (US)**
- **ADAMS, Sean, H.**
  **Davis, CA 95618 (US)**

(74) Representative: **MacLean, Martin Robert et al**
**Mathys & Squire**
**120 Holborn**
**London EC1N 2SQ (GB)**

(56) References cited:
- **ISHIKAWA ET AL: "Prediction of the coding sequences of unidentified human genes. X. The complete sequences of 100 new cDNA clones from brain which can code for large proteins in vitro" DNA RESEARCH, vol. 5, 30 June 1998 (1998-06-30), pages 169-176, XP000980105 ISSN: 1340-2838**
- **DATABASE EBI [Online] 29 September 2000 (2000-09-29) ISOGAI ET AL: "Homo sapiens cDNA FLJ13875 fis, clone THYRO1001374, weakly similar to cytosolic acyl coenzyme A thioester hydrolase (EC 3.1.2.2.) " retrieved from EBI Database accession no. AK023937 XP002228848**
- **DATABASE EBI [Online] 15 July 1998 (1998-07-15) OHARA ET AL: "Homo sapiens mRNA for KIAA0707 protein, partial cds" retrieved from EBI Database accession no. AB014607 XP002228849**
- **ADAMS ET AL: "BFIT, a unique acyl-CoA thioesterase induced in thermogenic brown adipose tissue: cloning, organization of the human gene and assessment of a potential link to obesity" BIOCHEMICAL JOURNAL, vol. 360, 15 November 2001 (2001-11-15), pages 135-142, XP002228847**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 368 462 B1

## Description

Remarkable shifts in the energetic profile of brown adipose tissue (BAT) in mammals occur upon exposure to a cold environment. Non-shivering thermogenesis (NST) and a concomitant increase in fuel uptake and combustion is triggered in BAT, thus providing a source of metabolic heat in defense of body temperature. A catalyst of these events is uncoupling protein 1 (UCP1), which increases membrane proton permeability and thus accelerates the flux of fuel-derived reducing equivalents in the electron transport chain. Sympathetic outflow to BAT also Increases upon exposure to a cold environment, eliciting rapid biochemical changes supporting thermogenesis, such as the stimulation of lypolysis. Furthermore, these changes are augmented by genetic regulation for the manifestation of NST in BAT, such as the rapid and dramatic upregulation of expression of BAT UCP1. Chronic exposure to a cold environment induces proliferation and differentiation of BAT, which by definition require changes at the level of gene regulation. Similar patterns of thermogenic activation may also occur in response to high-fat diets, possibly as a means to combat excessive weight gain under these conditions.

Genes induced in BAT by exposure to a cold environment, but repressed by exposure to a warm environment, may encode proteins involved in the transition of BAT towards a more thermogenic state. Induction of the PPARγ-coactivator-1 (PGC-1) fits this pattern, and this BAT-abundant transcription regulator likely plays a central role in sparking changes important to the regulation of metabolism in a variety of tissues.

Other proteins, and the corresponding genes and antibodies, Involved in the transition of BAT to a more thermogenic state, would be useful for controlling and studying the thermogenic state of BAT, as well as controlling and studying weight gain and loss in mammals.

A partial putative 5' open gene, KIAA0707, has been predicted to encode a protein with a molecular weight of 68 kDa (Ishikawa et al., 1998) (GenBank Accession No. AB014607). The protein that is predicted to be encoded by KIAA0707 contains a StAR-related lipid-transfer (START) domain (where "StAR" stands for steroidogenic acute regulatory protein; START domains are believed to bind lipids) and two acyl-CoA-thioesterase domains (Ponting and Aravind, 1999)).

The presence of acyl-CoA thioesterase (hydrolase) activity has been recognized in a wide variety of tissues since at least the 1950s. Despite extensive biochemical characterization of thioesterase proteins over the last few decades, and the molecular identification of numerous functional family members, a complete understanding of the physiological role of most thioesterases has remained elusive. Efforts to clarify the biological functions of specific acyl-CoA thioesterases will require studies placing the enzymes into a physiological context.

Database EBI: AK023937, September 2000, describes a *homo sapiens* cDNA and polypeptide, weakly similar to cytosolic acyl coenzyme A thioester hydrolase.

Database EBI: AB014607, July 1998, describes *homo sapiens* mRNA and translated protein KIAA0707.

The present invention provides an isolated polypeptide comprising at least 97% sequence identity to the sequence SEQ ID NO: 6, wherein the polypeptide has thioesterase activity.

The present invention also provides a polynucleotide, or its compliment, encoding this polypeptides.

The present invention also provides an antibody that specifically binds to this these polypeptides.

Further aspects of the present invention are defined in the claims.

Figure 1 illustrates quantitative expression analysis of mRNA samples derived from cold-challenged, control, and warm-challenged mice.

Figure 2 shows the amino acid sequence of human and murine BFIT variants.

Figure 3A shows the BFIT mRNA abundance in BAT of mice from different temperature environments.

Figure 3B shows the BFIT mRNA level in different mouse tissues.

Figures 4A, 4B and 4C show the BFIT mRNA abundance in BAT from different mouse strains fed a high-fat diet.

Figure 5 is a schematic representation of the human .BFIT genomic organization.

Figures 6A, 6B and 6C show the abundance of BFIT1 and BFIT2 mRNA in different human tissues and hibernoma.

Figures 7A depicts thioesterase activity of partially-purified recombinant hBFIT1.

Figure 7B depicts thioesterase activity of partially-purified recombinant hBFIT2.

DETAILED DESCRIPTION

A gene encoding a BAT-abundant acyl-CoA thioesterase, termed "BFIT" (brown fat inducible thioesterase) has been discovered. This gene has been found to be induced or repressed by cold- and warm-exposure, respectively.

The important role of BFIT in supporting enhanced metabolism is evidenced by its cold-induction in BAT, thioesterase and lipid-binding domains, co-localization with chromosomal sites associated with body fat in humans and mice, and a significantly depressed BAT expression level in obesity-prone mice as compared to their lean counterparts. Based on these facts, BFIT acts to increase energy expenditure and fatty acid oxidation.

Murine BFIT, termed "mBFIT2", is homologous to the human KIAA0707 (Ishikawa et al., 1998) (hereinafter termed "hBFIT1"). A novel splice variant of hBFIT1, termed "hBFIT2", is orthologous to mBFIT2. The term "BFIT" means hBFIT1 and all polypeptides having at least about 80% sequence identity to hBFIT1, including mBFIT2 and hBFIT2. The sequence of HBFIT1 is SEQ ID NO:2, the sequence of hBFIT2 is SEQ ID NO:4, and the sequence of mBFIT2 is SEQ ID NO:6. The cDNA sequence encoding hBFIT1 is SEQ ID NO:1, the cDNA sequence encoding hBFIT2 is SEQ ID NO: 3, and the cDNA sequence encoding mBFIT2 is SEQ ID NO:5.

BFIT catalyzes hydrolytic thioester cleavage, and in the case of acyl-CoA as a substrate, yields non-esterified fatty acids (NEFAs) and CoASH. Modification of the cellular acyl-CoA profile may be the mechanism by which BFIT activity impacts tissue function; BFIT affects the metabolic state of BAT and other tissues, possibly through modulation of certain acyl-CoA profiles which affect intracellular signaling pathways. Thioesters are also known as thiol esters, and are compounds that include the -(C=O)-S- moiety, for example acyl-CoA thioesters, such an acyl-CoA containing 2-20, preferably 4-16, carbon atoms in the acyl moiety.

Increasing the expression of BFIT in a mammal, such as a human, for example through gene therapy, is effective to combat metabolic diseases, such as obesity, or to increase weight loss. Similarly, decreasing the expression of BFIT, for example through gene therapy or administration of antisense polynucleotides, is effective to combat metabolic diseases, such as cachexia, or to increase weight gain. The term "metabolic disease" means a condition in which a disruption of normal energy homeostasis is a causative or exacerbating factor underlying disease. For example, metabolic disease includes obesity, wasting disorders such cachexia (associated with, for example, HIV infection, sepsis and trauma, and cancer), and diabetes (including Type II diabetes and insulin resistance).

Because of the important role of BFIT in supporting enhanced metabolism, it will be useful to test for compounds having the property of increasing or decreasing BFIT activity. This increase in activity may come about in a variety of ways, for example: (1) by increasing or decreasing the copies of the gene in the cell (amplifiers and deamplifiers); (2) by increasing or decreasing transcription of the BFIT gene (transcription up-regulators and down-regulators); (3) by increasing or decreasing the translation of BFIT mRNA into protein (translation up-regulators and down-regulators); or (4) by increasing or decreasing the activity of BFIT itself (agonists and antagonists).

Compounds that are amplifiers and deamplifiers may be identified by contacting cells or organisms with the compound, and then measuring the amount of DNA present that encodes BFIT [Ausubel, 1987- #114]. Compounds that are transcription up-regulators and down-regulators may be identified by contacting cells or organisms with the compound, and then measuring the amount of mRNA produced that encodes BFIT [Ausubel, 1987-#114]. Compounds that are translation up-regulators and down-regulators may be identified by contacting cells or organisms with the compound, and then measuring the amount of BFIT polypeptide produced [Ausubel, 1987-#114]. Compounds that are agonists or antagonists may be identified by contacting cells or organisms with the compound, and then measuring thioester hydrolysis; this may also be carried out *in vitro* (Lee, K.Y. and H. Schulz 1979).

Compounds that are amplifiers, transcription up-regulators, translation up-regulators or agonists, are effective to combat metabolic diseases that can be ameliorated by increasing metabolic activity, such as obesity. Conversely, compounds that are deamplifiers, transcription down-regulators, translation down-regulators or antagonists, are effective to combat metabolic diseases that can be ameliorated by decreasing metabolic activity, such as cachexia, or to increase weight gain. Gene therapy is another way to up-regulate or down-regulate transcription and/or translation.

Both BFIT polypeptides and the polynucleotides can be used in clinical screens to test for metabolic disease etiology, or to assess the level of risk for these disorders. Tissue samples, especially BAT, of a patient can be examined for the amount BFIT polypeptide or BFIT mRNA present. When amounts significantly smaller or larger than normal are found, they will be indicative of metabolic disease, or risk of metabolic disease. Mutation of BFIT can have altered activity, and a patient with such a mutation may have a metabolic disease or be at risk for a metabolic disease. Finally, determining the amount of expression of BFIT in a mammal, in a tissue sample, or in a tissue culture, can be used to discover inducers or repressors of the gene.

"Percent (%) nucleic acid sequence identity" with respect to BFIT-encoding nucleic acid sequences identified herein is defined as the percentage of nucleotides in a candidate sequence that are identical with the nucleotides in the BFIT nucleic acid sequence of interest, after aligning the sequences and introducing gaps, if necessary, to achieve the maximum percent sequence identity. Alignment for purposes of determining % nucleic acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared.

When nucleotide sequences are aligned, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) can be calculated as follows:

$$\%\text{nucleic acid sequence identity} = W/Z \cdot 100$$

where
W is the number of nucleotides cored as identical matches by the sequence alignment program's or algorithm's alignment of C and D
and
Z is the total number of nucleotides in D.

When the length of nucleic acid sequence C is not equal to the length of nucleic acid sequence D, the % nucleic acid sequence identity of C to D will not equal the % nucleic acid sequence identity of D to C.

Percent nucleic acid sequence identity values may also be obtained by using the WU-BLAST-2 computer program (Altschul and Gish, 1996). Most of the WU-BLAST-2 search parameters are set to the default values. Those not set to default values, i.e., the adjustable parameters, are set with the following values: overlap span = 1, overlap fraction = 0.125, word threshold (T) = 11, and scoring matrix = BLOSUM62. When WU-BLAST-2 is employed, a % nucleic acid sequence identity value is determined by dividing (a) the number of matching identical nucleotides between the nucleic acid sequence of the BFIT polypeptide-encoding nucleic acid molecule of interest having a sequence derived from the sequence BFIT polypeptide-encoding nucleic acid and the comparison nucleic acid molecule of interest (i.e., the sequence against which the BFIT polypeptide-encoding nucleic acid molecule of interest is being compared which may be a variant BFIT polynucleotide) as determined by WU-BLAST-2 by (b) the total number of nucleotides of the BFIT polypeptide-encoding nucleic acid molecule of interest. For example, in the statement "an isolated nucleic acid molecule comprising a nucleic acid sequence A which has or having at least 80 % nucleic acid sequence identity to the nucleic acid sequence B", the nucleic acid sequence A is the comparison nucleic acid molecule of interest and the nucleic acid sequence B is the nucleic acid sequence of the BFIT polypeptide-encoding nucleic acid molecule of interest.

Percent nucleic acid sequence identity may also be determined using the sequence comparison program NCBI (National Center for Biotechnology Information)-BLAST2 (Altschul et al., 1997)). The NCBI-BLAST2 sequence comparison program is publicly available at www.ncbi.nlm.nih.gov/BLAST (BLAST home page); most recently, the specific program was located at www.ncbi.nlm.nih.gov/gorf/bl2.html; if these links should be broken, the National Center for Biotechnology (NCBI) homepage is www.ncbi.nlm.nih.gov. NCBI-BLAST2 uses several search parameters, wherein all of those search parameters are set to default values including, for example, unmask = yes, strand = all, expected occurrences = 10, minimum low complexity length = 15, multi-pass e-value = 0.01, constant for multi-pass = 25, dropoff for final gapped alignment = 25 and scoring matrix BLOSUM62.

In situations where NCBI-BLAST2 is employed for sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:

$$\%\text{nucleic acid sequence identity} = W/Z \cdot 100$$

where
W is the number of nucleotides cored as identical matches by the sequence alignment program's or algorithm's alignment of C and D
and
Z is the total number of nucleotides in D.

When the length of nucleic acid sequence C is not equal to the length of nucleic acid sequence D, the % nucleic acid sequence identity of C to D will not equal the % nucleic acid sequence identity of D to C.

BFIT variant polynucleotides are nucleic acid molecules that encode an active BFIT polypeptide and which are capable of hybridizing, preferably under stringent hybridization and wash conditions, to nucleotide sequences encoding a full-length BFIT polypeptide as disclosed herein. BFIT variant polypeptides may be those that are encoded by a BFIT variant polynucleotide.

The term "control sequences" refers to nucleotide sequences that enable expression of an operably linked coding sequence in a particular host organism. Prokaryotic control sequences include 1) a promoter, 2) optionally an operator sequence, and 3) a ribosome-binding site. Eukaryotic cells use 1) promoters, 2) polyadenylation signals, and 3) enhancers.

Nucleic acid is "operably linked" when in it is in a functional relationship with another nucleic acid sequence. For example, a nucleotide sequence for a pre-sequence or secretory leader is operably linked to DNA for a polypeptide if it is expressed as a pre-protein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The full- or partial length BFIT gene sequence may be used to "pull out" similar (homologous) sequences. Such hybridization probes can be used to identify homologous sequences that encode: 1) full-length BFIT cDNA from a cDNA library from any species (e.g. human, murine, feline, canine, bacterial, viral, retroviral, yeast, or cDNAs, 2) human cDNA, 3) variants within a species, and 4) homologues from other species.

In such experiments, the probe length is optimally about 20 to about 50 bases. Such probes can be designed by one of skill in the art to hybridize the desired sequences. For example, to identify a full-length cDNA, the probe would preferably comprise unique sequences. To find related sequences that may encode related genes, the probe may be designed to encode unique sequences or degenerate sequences. Unique regions may be determined without undue experimentation; sequences may also be from genomic sequences including promoters, enhancer elements and introns of BFIT gene sequence.

An example using such a probe would be a screen to isolate the coding region of a BFIT gene in another species. Using the known BFIT DNA sequence, a probe is designed and made of about 40 bases. Hybridization probes may be labeled by a variety of labels, including nucleic acids label with radionuclides such as $^{32}P$ or $^{35}S$, or enzymatic labels such as alkaline phosphatase coupled to the probe via avidin/biotin coupling systems. Labeled probes having a sequence complementary to that of the BFIT gene of the present invention can be used to screen libraries of cDNA, genomic DNA or mRNA of a desired species to determine which members of such libraries the probe hybridizes to. Hybridization techniques are well known to those of skill in the art.

Any EST sequences disclosed in the present application may similarly be employed as probes, using the methods disclosed herein.

If the BFIT coding sequence encodes a protein that binds another protein, the BFIT protein can be used as a probe to identify the interacting molecules. These approaches have functional applications. For example, such methods can identify inhibitors or agonists of the protein-protein binding interaction. Screening assays can identify compounds that mimic the biological activity of a native BFIT or a protein interacting with BFIT. Such screening assays will include those amenable to high-throughput screening of chemical libraries that may identify small molecule drug candidates, such as synthetic organic or inorganic compounds. The assays can be performed in a variety of formats, including protein-protein binding assays, biochemical screening assays, immunoassays and cell based assays, which are well characterized in the art.

If a BFIT polypeptide interacts with but does not bind another polypeptide, the identity of the interacting polypeptide can be identified by well known methods. These assays incorporate techniques such as cross-linking, co-immunoprecipitation and co-purification through gradients or chromatographic columns. Protein-protein interactions can also be monitored by the yeast two-hybrid system described by Fields and Song (1989), Chien et al. (1991) and as disclosed by Chevray and Nathans, (1991). Many transcriptional activators, such as yeast GAL4, consist of two physically discrete modular domains, one acting as the DNA-binding domain, the other one functioning as the transcription-activation domain. The yeast expression system described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GAL1-*lac*Z reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER™) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

Compounds that interfere with the interaction of a BFIT polypeptide or polynucleotide identified herein and other intra- or extracellular components can be tested as follows: usually a reaction mixture is prepared containing the polynucleotide or polypeptide of interest and the intra- or extracellular component under conditions and for a time allowing for the interaction and binding of the two products. To test the ability of a candidate compound to inhibit binding, the reaction is run in the absence and in the presence of the test compound. In addition, a placebo may be added to a third reaction mixture, to serve as positive control. The binding (complex formation) between the test compound and the intra- or extracellular component present in the mixture is monitored as described hereinabove. The formation of a complex in the control reaction(s) but not in the reaction mixture containing the test compound indicates that the test compound interferes with the interaction of the test compound and its reaction partner.

An "isolated" BFIT polypeptide-encoding nucleic acid is a nucleic acid molecule that is identified and separated from at least one contaminant nucleic acid molecule with which it is ordinarily associated in the natural source of the BFIT polypeptide nucleic acid. An isolated BFIT polypeptide nucleic acid molecule is other than in the form or setting in which it is found in nature. Isolated BFIT polypeptide nucleic acid molecules therefore are distinguished from the specific BFIT polypeptide nucleic acid molecule, as it exists in natural cells. However, an isolated BFIT polypeptide nucleic acid molecule includes BFIT polypeptide nucleic acid molecules contained in cells that ordinarily express the BFIT polypeptide where, for example, the nucleic acid molecule is in a chromosomal location different from that of natural cells, or as provided extra-chromosomally.

Methods of eukaryotic cell transfection and prokaryotic cell transformation, are well known in the art, and may be used to recombinantly produce BFIT protein. The choice of host cell will dictate the preferred technique for introducing the nucleic acid of interest. Table A, which is not meant to be limiting, summarizes many of the known techniques in the art. Introduction of nucleic acids into an organism may also be done with *ex vivo* techniques that use an *in vitro* method of transfection.

Table A: Methods to introduce nucleic acid into cells

| Cells | Methods | References | Notes |
|---|---|---|---|
| Prokaryotes (bacteria) | Calcium chloride | (Cohen et al., 1972; Hanahan, 1983; Mandel and Higa, 1970) | |
| | Electroporation | (Shigekawa and Dower, 1988) | |
| Eukaryotes<br>Mammalian cells | Calcium Phosphate transfection | *N*-(2-Hydroxyethyl) piperazine-*N'*-(2-ethanesulfonic acid (HEPES) buffered saline solution (Chen and Okayama, 1988;<br>Graham and van der Eb, 1973; Wigler et al., 1978)<br>BES (*N*,*N*-bis(2-hydroxyethyl)-2-aminoethanesulfonic acid) buffered solution (Ishiura et al., 1982) | Cells may be Cells may be glycerol or dimethylsulfoxide (DMSO) to increase transfection efficiency [Ausubel, 1987-#114]. |
| | Diethylaminoethyl (DEAE)-Dextran transfection | (Fujita et al., 1986; Lopata et al., 1984; Selden et al., 1986) | Most useful for transient, but not stable, transfections. Chloroquine can be used to increase efficiency. |
| | Electroporation | (Neumann et al., 1982; Potter, 1988; Potter et al., 1984; Wong and Neumann, 1982) | Especially useful for hard-to-transfect lymphocytes. |
| | Cationic lipid reagent transfection | (Elroy-Stein and Moss, 1990; Felgner et al., 1987; Rose et al., 1991; Whitt et al., 1990) | Applicable to both *in vivo* and *in vitro* transfection. |

(continued)

| Cells | Methods | References | Notes |
|---|---|---|---|
| | Retroviral | Production exemplified by (Cepko et al., 1984; Miller and Buttimore, 1986; Pear et al., 1993) Infection *in vitro* and *in vivo*: (Austin and Cepko, 1990; Bodine et al., 1991; Fekete and Cepko, 1993; Lemischka et al., 1986; Turner et al., 1990; Williams et al., 1984) | Lengthy process, many packaging lines available at ATCC. Applicable to both *in vivo* and *in vitro* transfection. |
| | Polybrene | (Chaney et al., 1986; Kawai and Nishizawa, 1984) | |
| | Microinjection | (Capecchi, 1980) | Can be used to establish cell lines carrying integrated copies of BFIT DNA sequences. |
| | Protoplast fusion | (Rassoulzadegan et al., 1982; Sandri-Goldin et al., 1981; Schaffner, 1980) | |
| Insect cells (*in vitro*) | Baculovirus systems | (Luckow, 1991; Miller, 1988 O'Reilly et al., 1992) | Useful for *in vitro* production of proteins with eukaryotic modifications. |
| Yeast | Electroporation | (Becker and Guarente, 1991) | |
| | Lithium acetate | (Gietz et al., 1998; Ito et al., 1983) | |
| | Spheroplast fusion | (Beggs, 1978; Hinnen et al., 1978 | Laborious, can produce aneuploids. |
| Plant cells (general reference: (Hansen and Wright, 1999)) | Agrobacterium transformation | (Bechtold and Pelletier, 1998; Escudero and Hohn, 1997; Hansen and Chilton, 1999; Touraev and al., 1997) | |
| | Biolistics (microprojectiles) | (Finer et al., 1999; Hansen and Chilton, 1999; Shillito, 1999) | |
| | Electroporation (protoplasts) | (Fromm et al., 1985; Ou-Lee et al., 1986; Rhodes et al., 1988; Saunders et al., 1989) al., 1989) May be combined with liposomes (Trick and al., 1997) | |
| | Polyethylene glycol (PEG) treatment | (Shillito, 1999) | |
| | Liposomes | May be combined with electroporation (Trick and al., 1997) | |

(continued)

| Cells | Methods | References | Notes |
|---|---|---|---|
| | *in planta* microinjection | (Leduc and al., 1996; Zhou and al., 1983) | |
| | Seed imbibition | (Trick and al., 1997) | |
| | Laser beam | (Hoffman, 1996) | |
| | Silicon carbide whiskers | (Thompson and al., 1995) | |

Any cell may be transfected or transformed, pending the development of a suitable method. Table B, which is not meant to be limiting, lists many examples of cells commonly used as hosts for DNA or the expression of DNA *in vitro.*

Table B: Examples of hosts for cloning or expressing nucleic acids (DNA) (not intended to be limiting)

| Organisms | Examples | Sources and References* |
|---|---|---|
| Prokaryotes<br>Enterobacteriaceae | *E. coli*<br>K 12 strain MM294<br>X1776<br>W3110<br>K5 772 | <br>ATCC 31,446<br>ATCC 31,537<br>ATCC 27,325<br>ATCC 53,635 |
| Enterobacteriaceae | *E. coli* | |
| | *Enterobacter* | |
| | *Erwinia* | |
| | *Klebsiella* | |
| | *Proteus* | |
| | *Salmonella* (*S. tyhpimurium*) | |
| | *Serratia* (*S. marcescans*) | |
| | *Shigella* | |
| | *Bacilli* (B. *subtilis* and *B. licheniformis*) | |
| | *Pseudomonas* (*P. aeruginosa*) | |
| | *Streptomyces* | |
| Eukaryotes | *Saccharomyces cerevisiae* | |
| | *Schizosaccharomyces pombe* | |
| Yeasts | *Kluyveromyces*<br>*K. lactis* MW98-8C, CBS683, CBS4574<br>*K. fragilis*<br>*K. bulgaricus*<br>*K. wickeramii*<br>*K. waltii*<br>*K. drosophilarum*<br>*K. thermotolerans*<br>*K. marxianus; yarrowia* | (Fleer et al., 1991)<br>(de Louvencourt et al., 1983)<br>ATCC 12,424<br>ATCC 16,045<br>ATCC 24,178<br>ATCC 56,500<br>ATCC 36,906<br><br>(EPO 402226, 1990) |
| | *Pichia pastoris* | (Sreekrishna et al., 1988) |

(continued)

| Organisms | Examples | Sources and References* |
|---|---|---|
| | *Candida* | |
| | *Trichoderma reesia* | |
| | *Neurospora crassa* | (Case et al., 1979) |
| | *Torulopsis* | |
| | *Rhodotorula* | |
| | *Schwanniomyces* (*S. occidentalis*) | |
| Filamentous Fungi | *Neurospora* | |
| | *Penicillium* | |
| | *Tolypocladium* | (WO 91/00357, 1991) |
| | *Aspergillus* (*A. nidulans* and *A. niger*) *nidulans* | (Kelly and Hynes, 1985; Tilburn et al., 1983; Yelton et al., 1984) |
| Invertebrate cells | *Drosophila* S2 | |
| | *Spodoptera* Sf9 | |
| Vertebrate cells | Chinese Hamster Ovary (CHO) | |
| | simian COS COS-7 | ATCC CRL 1651 |
| | HEK 293 | |
| *Not meant to be limiting | | |

Vectors act as tools to shuttle DNA between host cells or as a means to produce a large quantity of the DNA. Some vectors function only in prokaryotes, while others function in both prokaryotes and eukaryotes, enabling large-scale DNA preparation from prokaryotes to expression in a eukaryote. Inserting the DNA of interest, such as BFIT nucleotide sequence or a fragment, is accomplished by ligation techniques and/or mating protocols well-known to the skilled artisan. Such DNA is inserted such that its integration does not disrupt any necessary components of the vector. In the case of vectors that are used to express the inserted DNA protein, the introduced DNA is operably linked to the vector elements that govern its transcription and translation.

Vector choice is governed by the organism or cells being used and the desired fate of the vector. Vectors may replicate once in the target cells, or may be "suicide" vectors. In general, vectors comprise signal sequences, origins of replication, marker genes, enhancer elements, promoters, and transcription termination sequences. The choice of these elements depends on the organisms in which they will be used and are easily determined by one of skill in the art. Some of these elements may be conditional, such as an inducible or conditional promoter that is turned "on" when conditions are appropriate. Examples of such promoters include tissue-specific, which relegate expression to certain cell types, steroid-responsive, or heat-shock inducible. Some bacterial repression systems, such as the lac operon, have been exploited in mammalian cells. Vectors often have a selectable marker that facilitate identifying those cells that have taken up the exogenous nucleic acids. Many selectable markers are well known in the art for the use with prokaryotes, usually antibiotic-resistance genes or the use of autotrophy and auxotrophy. Table C, which is not meant to be limiting, lists often-used selectable markers for mammalian cell transfection.

Table C: Useful selectable markers for eukaryote cell transfection

| Selectable Marker | Selection | Action | Reference |
|---|---|---|---|
| Adenosine deaminase (ADA) | Media includes 9-β-D-xylofuranosyl adenine (Xyl-A) | Conversion of Xyl-A to Xyl-ATP, which incorporates into nucleic acids, killing cells. ADA detoxifies | (Kaufman et al., 1986) |

(continued)

| Selectable Marker | Selection | Action | Reference |
|---|---|---|---|
| Dihydrofolate reductase (DHFR) | Methotrexate (MTX) and dialyzed serum (purine-free media) | MTX competitive inhibitor of DHFR. In absence of exogenous purines, cells require DHFR a necessary enzyme in purine biosynthesis. | (Simonsen and Levinson, 1983) |
| Aminoglycoside phosphotransferase ("APH", "neo", "G418") | G418 | G418, an aminoglycoside detoxified by APH, interferes with ribosomal function translation. | (Southern and Berg, 1982) |
| Hygromycin-B-phosphotransferase (HPH) | hygromycin-B | Hygromycin-B, an aminocyclitol detoxified by HPH, disrupts protein translocation and promotes mistranslation. | (Palmer et al., 1987) |
| Thymidine kinase (TK) kinase | Forward selection (TK+) : Media (HAT) incorporates aminopterin. Reverse selection (TK-): Media incorporates 5-bromodeoxyuridine (BrdU). | Forward: Aminopterin forces cells to synthesze dTTP from thymidine, a pathway requiring TK. Reverse: TK phosphorylates BrdU, which incorporates into nucleic acids, killing cells. | (Littlefield, 1964) |

To monitor BFIT gene expression or to facilitate biochemical purification, BFIT nucleotide sequence can be fused to a heterologous peptide. These include reporter enzymes and epitope tags that are bound by specific antibodies. Table D, which is not meant to be limiting, lists some of the common fusions used to report gene expression.

Table D: Reporter proteins

| Reporter | *in vitro* | *in vivo* | Notes | Reference |
|---|---|---|---|---|
| Human growth hormone (hGH) | radioimmunoassay | none | Expensive, insensitive, narrow linear range. | (Selden et al., 1986) |
| β-glucuronidase (GUS) | colorimetric, fluorescent, or chemiluminescent | colorimetric (histochemical staining with X-gluc) | sensitive, broad linear range, non-iostopic. | (Gallagher, 1992) |
| Green fluorescent protein (GFP) and related molecules (RFP, BFP, etc.) | fluorescent | fluorescent | can be used in live cells; resists photobleaching | (Chalfie et al., 1994) |
| Luciferase (firefly) | bioluminsecent | bioluminescent | protein is unstable, difficult to reproduce, signal is brief | (de Wet et al., 1987) |
| Chloramphenicoal acetyltransferase (CAT) | chromatography, differential extraction, fluorescent, or immunoassay | none | expensive radioactive substrates, time-consuming, insensitive, narrow linear range | (Gorman et al., 1982) |

(continued)

| Reporter | *in vitro* | *in vivo* | Notes | Reference |
|---|---|---|---|---|
| β-galactosidase | colorimetric, fluorescence, chemiluminscence | colorimetric (histochemical staining with X-gal), bioluminescent in live cells | sensitive, broad linear range; some cells have high endogenous activity | (Alam and Cook, 1990) |
| Secrete alkaline phosphatase (SEAP) | colorimetric, bioluminescent, chemiluminescent | none | chemiluminscence assay is sensitive and broad linear range; some cells have endogenouse alkaline phosphatase activity | (Berger et al., 1988) |

"BFIT nucleotide variant" means a nucleic acid that encodes a BFIT polypeptide variant. Only those variants defined in the claims are part of this invention. "BFIT polypeptide variant" includes an active BFIT polypeptide as defined above or below having at least: 1) about 80% amino acid sequence identity with a full-length sequence BFIT polypeptide sequence as disclosed herein, 2) a BFIT polypeptide sequence lacking the signal peptide as disclosed herein, 3) an extracellular domain of a BFIT polypeptide, with or without the signal peptide, as disclosed herein, or 4) any other fragment of a full-length BFIT polypeptide sequence as disclosed herein. For example, BFIT polypeptide variants include BFIT polypeptides wherein one or more amino acid residues are added or deleted at the N- or C- terminus of the full-length amino acid sequence. A BFIT polypeptide variant will have at least about 80% amino acid sequence identity, preferably at least about 81 % amino acid sequence identity, more preferably at least about 82%, 83%, 84%, 85%, 86%, 87%, 88%, 89%, 90%, 91%, 92%, 93%, 94%, 95%, 96%, 97%, 98% amino acid sequence identity and most preferably at least about 99% amino acid sequence identity with a full-length sequence BFIT polypeptide sequence as disclosed herein. A BFIT polypeptide variant may have a sequence lacking the signal peptide as disclosed herein, an extracellular domain of a BFIT polypeptide, with or without the signal peptide, as disclosed herein or any other fragment of a full-length BFIT polypeptide sequence as disclosed herein. Variants do not encompass the native nucleotide sequence. Ordinarily, BFIT variant polypeptides are at least about 10 amino acids in length, often at least about 20 amino acids in length, more often at least about 30, 40, 50, 60, 70, 80, 90, 100, 150, 200, or 300 amino acids in length, or more.

"Percent (%) amino acid sequence identity" is defined as the percentage of amino acid residues that are identical with amino acid residues in the disclosed BFIT polypeptide sequence in a candidate sequence when the two sequences are aligned. To determine % amino acid identity, sequences are aligned and if necessary, gaps are introduced to achieve the maximum % sequence identity; conservative substitutions are not considered as part of the sequence identity. Amino acid sequence alignment procedures to determine percent identity are well known to those of skill in the art. Often publicly available computer software such as BLAST, BLAST2, ALIGN2 or Megalign (DNASTAR) software are used to align peptide sequences. Those skilled in the art can determine appropriate parameters for measuring alignment, including any algorithms needed to achieve maximal alignment over the full length of the sequences being compared.

When amino acid sequences are aligned, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) can be calculated as:

$$\%\text{amino acid sequence identity} = X/Y \cdot 100$$

where

X is the number of amino acid residues scored as identical matches by the sequence alignment program's or algorithm's alignment of A and B

and

Y is the total number of amino acid residues in B.

If the length of amino acid sequence A is not equal to the length of amino acid sequence B, the % amino acid sequence identity of A to B will not equal the % amino acid sequence identity of B to A.

Percent amino acid sequence identity values may be obtained using the WU-BLAST2 computer program (Altschul and Gish, 1996)). Most of the WU-BLAST2 search parameters are set to the default values. Those not set to default values, i.e., the adjustable parameters, are set with the following values: overlap span = 1, overlap fraction = 0.125, word threshold (T) = 11, and scoring matrix = BLOSUM62. When WU-BLAST2 is employed, a % amino acid sequence identity value is determined by dividing (a) the number of matching identical amino acid residues between the amino acid sequence of the BFIT polypeptide of interest having a sequence derived from the native BFIT polypeptide and the comparison amino acid sequence of interest (i.e., the sequence against which the BFIT polypeptide of interest is being compared which may be a BFIT variant polypeptide) as determined by WU-BLAST2 by (b) the total number of amino acid residues of the BFIT polypeptide of interest. For example, in the statement "a polypeptide comprising an the amino acid sequence A which has or having at least 80 % amino acid sequence identity to the amino acid sequence B", the amino acid sequence A is the comparison amino acid sequence of interest and the amino acid sequence B is the amino acid sequence of the BFIT polypeptide of interest.

Percent amino acid sequence identity may also be determined using the sequence comparison program NCBI (National Center for Biotechnology Information)-BLAST2 (Altschul et al., 1997)). The NCBI-BLAST2 sequence comparison program is publicly available at www.ncbi.nlm.nih.gov/BLAST (BLAST home page); most recently, the specific program was located at www.ncbi.nlm.nih.gov/gorf/bl2.html; if these links should be broken, the National Center for Biotechnology (NCBI) homepage is www.ncbi.nlm.nih.gov. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix BLOSUM62.

In situations where NCBI-BLAST2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$\%\text{amino acid sequence identity} = X/Y \cdot 100$$

where

X is the number of amino acid residues scored as identical matches by NCBI-BLAST2 program's alignment of A and B and

Y is the total number of amino acid residues in B.

When the length of amino acid sequence A is not equal to the length of amino acid sequence B, the % amino acid sequence identity of A to B will not equal the % amino acid sequence identity of B to A.

The term "positives" in the context of sequence comparison includes residues in the compared sequences that are not identical but have similar properties (e.g. as a result of conservative substitutions). For purposes herein, the % value of positives is determined by dividing (a) the number of amino acid residues scoring a positive value between the BFIT polypeptide amino acid sequence of interest having a sequence derived from the native BFIT polypeptide sequence and the comparison amino acid sequence of interest (i.e., the amino acid sequence against which the BFIT polypeptide sequence is being compared) as determined in the BLOSUM62 matrix of WU-BLAST2 by (b) the total number of amino acid residues of the BFIT polypeptide of interest. The ALIGN2 and NCBI-BLAST2 programs compare sequences, they consider not only identical amino acid residues, but also those having similar properties. Amino acid residues that score "positive" to an amino acid residue of interest are either identical to, or are a preferred substitution of, the amino acid residue of interest.

For amino acid sequence comparisons using ALIGN2 or NCBI-BLAST2, the % value of positives of a given amino acid sequence A to, with, or against a given amino acid sequence B (which can alternatively be phrased as a given amino acid sequence A that has or comprises a certain % positives to, with, or against a given amino acid sequence B) is calculated as follows:

$$\%\text{value of positives} = X/Y \cdot 100$$

where

X is the number of amino acid residues scored as positives by NCBI-BLAST2 or ALIGN2 programs' alignment of A and B and

Y is the total number of amino acid residues in B.

When the length of amino acid sequence A is not equal to the length of amino acid sequence B, the % positives of A to B will not equal the % positives of B to A.

BFIT fragments may be prepared by a number of techniques, including 1) chemical synthesis, 2) enzymatic digestion of BFIT, 3) recombinant DNA methods. Chemical synthesis methods are well known in the art, as are methods that employ proteases with specific cleavage sites. Endonuclease digestion or polymerase chain reaction (PCR) to produce segments of BFIT nucleic acid and then expressing the segments in a host organism, such as E. *coli* is an attractive method that allows one of skill in the art to generate an unlimited supply of well-defined fragments. Preferably, BFIT polypeptide fragments share at least one biological and/or immunological activity with the native BFIT polypeptide.

In particular embodiments, conservative substitutions of interest are shown in Table E, "Preferred substitutions." If such substitutions result in a change in biological activity, then more substantial changes, denominated exemplary substitutions in Table E, or as further described below in reference to amino acid classes, are introduced and the products screened.

| Table E: Preferred substitutions | | |
|---|---|---|
| Original residue | Exemplary substitutions | Preferred substitutions |
| Ala (A) | Val, Leu, Ile | Val |
| Arg (R) | Lys, Gln, Asn | Lys |
| Asn (N) | Gln, His, Lys, Arg | Gin |
| Asp (D) | Glu | Glu |
| Cys (C) | Ser | Ser |
| Gln (Q) | Asn | Asn |
| Glu (E) | Asp | Asp |
| Gly (G) | Pro, Ala | Ala |
| His (H) | Asn, Gln, Lys, Arg | Arg |
| Ile (I) | Leu, Val, Met, Ala, Phe | |
| | Norleucine | Leu |
| Leu (L) | Norleucine, Ile, Val | |
| | Met, Ala, Phe | Ile |
| Lys (K) | Arg, Gln, Asn | Arg |
| Met (M) | Leu, Phe, Ile | Leu |
| Phe (F) | Leu, Val, Ile, Ala, Tyr | Leu |
| BFIT (P) | Ala | Ala |
| Ser (S) | Thr | Thr |
| Thr (T) | Ser | Ser |
| Trp (W) | Tyr, Phe | Tyr |
| Tyr (Y) | Trp, Phe, Thr, Ser | Phe |
| Val (V) | Ile, Leu, Met, Phe | |
| | Ala, Norleucine | Leu |

Substitutions that effect 1) the structure of the polypeptide backbone, such as a β-sheet or α-helical conformation, 2) the charge or 3) hydrophobicity, or 4) the bulk of the side chain of the target site can modify BFIT polypeptide function or immunological identity. Residues are divided into groups based on common side-chain properties:

| Table F: Amino acid classes | |
|---|---|
| Class | Amino acids |
| hydrophobic | Norleucine, Met, Ala, Val, Leu, Ile |

(continued)

| Table F: Amino acid classes | |
|---|---|
| Class | Amino acids |
| neutral hydrophilic | Cys, Ser, Thr |
| acidic | Asp, Glu |
| basic | Asn, Gln, His, Lys, Arg |
| disrupt chain conformation | Gly, Pro |
| aromatic | Trp, Tyr, Phe |

Non-conservative substitutions entail exchanging a member of one of these classes for another class. Substitutions may be introduced into conservative substitution sites or more preferably into non-conserved sites.

The variations can be made using methods known in the art such as oligonucleotide-mediated (site-directed) mutagenesis, alanine scanning, and PCR mutagenesis. Site-directed mutagenesis (Carter et al., 1986; Zoller et al., 1987), cassette mutagenesis (Wells et al., 1985), restriction selection mutagenesis (Wells et al., 1986) or other known techniques can be performed on the cloned DNA to produce the BFIT variant DNA.

An "epitope tagged" polypeptide refers to a chimeric polypeptide fused to a "tag polypeptide". The tag polypeptide provides an epitope against which an antibody can be made, but is short enough to not interfere with polypeptide activity to which it is fused. To reduce cross-reactivity of such an antibody with other endogenous epitopes, the tag polypeptide is preferably unique. Suitable tag polypeptides generally have at least six amino acid residues and usually between about 8 and 50 amino acid residues (preferably, between about 8 and 20 amino acid residues). Examples of epitope tag sequences include FLAG and HA from *Influenza* A virus.

"Active" or "activity" refers to form(s) of a BFIT polypeptide that retain a biological and/or an immunological activity of native or naturally-occurring BFIT. Immunological activity refers to the ability to induce the production of an antibody against an antigenic epitope possessed by a native or naturally occurring BFIT whereas biological activity refers to a function, either inhibitory or stimulatory, caused by a native or naturally-occurring BFIT that excludes immunological activity. Biological activity includes, but not limited to, acyl-CoA thioesterase (hydrolase) activity.

An "isolated" polypeptide is one that has been identified and separated and/or recovered from a component of its natural environment. Contaminant components of its natural environment are materials that would typically interfere with diagnostic or therapeutic uses for the polypeptide, and may include enzymes, hormones, and other proteinaceous or non-proteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide *in situ* within recombinant cells, since at least one component of the BFIT polypeptide natural environment will not be present. Ordinarily, however, isolated polypeptide will be prepared by at least one purification step.

The term "antibody" is used in the broadest sense and comprises single anti-BFIT mAbs (including antagonist and neutralizing antibodies), anti-BFIT antibody compositions with poly-epitopic specificity, single chain anti-BFIT antibodies, and fragments of anti-BFIT antibodies. The term "monoclonal antibody" (mAb) is an antibody obtained from a population of substantially homogeneous antibodies, i.e., the individual antibodies comprising the population are identical except for possible naturally-occurring mutations that may be present in minor amounts.

The present invention provides antibodies that specifically bind to a polypeptide comprising at least 97% sequence identity, or at least 98% sequence identity to the sequence SEQ ID NO: 6, wherein said polypeptide has thioesterase activity. Exemplary antibodies include polyclonal, monoclonal, humanized, bi-specific, and heteroconjugate antibodies. These antibodies can be used to assay for the presences of BFIT polypeptide is a sample, for example through the use of an ELISA assay. Such an assay is useful, for example, to screen patient tissue samples and in screening assays looking for agonists or antagonist of BFIT.

Methods of preparing polyclonal antibodies are known to the skilled artisan. Polyclonal antibodies can be raised in a mammal, for example, by one or more injections of an immunogen and, if desired, an adjuvant. Typically, the immunogen and/or adjuvant are injected in the mammal by multiple subcutaneous or intraperitoneal injections. The immunogen may include the BFIT polypeptide or a fusion protein thereof. Examples of adjuvants include Freund's complete and MPL-TDM (monophosphoryl Lipid A, synthetic trehalose dicorynomycolate). It may be useful to conjugate the immunogen to a protein known to be immunogenic in the mammal being immunized. Examples of such immunogenic proteins are keyhole limpet hemocyanin (KLH), serum albumin, bovine thyroglobulin, and soybean trypsin inhibitor. One skilled in the art without undue experimentation may select the immunization protocol.

Alternatively, anti-BFIT antibodies may be monoclonal antibodies (mAbs). MAbs may be prepared using hybridoma methods, such as described by Kohler and Milstein (1975), A typical hybridoma method comprises at least 4 steps: 1) immunizing a host, or lymphocytes from a host, with the immunogen of interest; 2) harvesting the mAb secreting (or potentially secreting) lymphocytes, 3) fusing the lymphocytes to immortalized cells, and 4) selecting those cells that secrete the antl-BFIT mAb.

A mouse, hamster, or other appropriate host is immunized to elicit lymphocytes that produce or are capable of producing antibodies that will specifically bind to the immunogen. Alternatively, the lymphocytes may be immunized *in vitro.* If human cells are desired, peripheral blood lymphocytes (PBLs) are generally used; however, spleen cells or lymphocytes from other mammalian sources may also be used. The immunogen typically includes the BFIT polypeptide or a fusion protein thereof.

The lymphocytes are then fused with an immortalized cell line to form hybridoma cells, facilitated by a fusing agent such as polyethylene glycol (Goding, 1996). Rodent, bovine, or human myeloma cells immortalized by transformation may be used, or rat or mouse myeloma cell lines. Because pure populations of hybridoma cells and not unfused immortalized cells are preferred, the cells after fusion are grown in a suitable medium that contains one or more substances that inhibit the growth or survival of unfused, immortalized cells. A common technique uses parental cells that lack the enzyme hypoxanthine guanine phosphoribosyl transferase (HGPRT or HPRT). In this case, hypoxanthine, aminopterin and thymidine are added to the medium ("HAT medium") to prevent the growth of HGPRT-deficient cells while permitting hybridomas to grow.

Preferred immortalized cells fuse efficiently, can be isolated from mixed populations of cells by culturing in a medium such as HAT, and support stable and high-level expression of antibody after fusion. More preferred immortalized cell lines are murine myeloma lines, available from the American Type Culture Collection (Manassas, VA). Human myeloma and mouse-human heteromyeloma cell lines also have been described for the production of human mAbs (Kozbor, 1984; Schook, 1987).

Because hybridoma cells secrete antibody extracellularly, the culture media can be assayed for the presence of mAbs directed against BFIT (anti-BFIT mAbs). Immunoprecipitation or *in vitro* binding assays, such as radioimmunoassay (RIA) or enzyme-linked immunoabsorbent assay (ELISA), measure the binding specificity of mAbs. Such techniques are well known in the art. For example, Scatchard analysis (Munson and Rodbard, 1980) can be used to determine the binding affinity of a mAb.

Anti-BFIT secreting hybridoma cells may be isolated as single clones by limiting dilution procedures and subcultured by standard methods (Goding, *supra*). Suitable culture media include Dulbecco's Modified Eagle's Medium, RPMI-1640, or if desired, a protein-reduced or free and/or serum free medium (*e.g.*, Ultra DOMA PF or HL-1 from Biowhittaker; Walkersville, MD). Alternatively, the hybridoma cells may be grown *in vivo* as ascites in a mammal.

The mAbs may be isolated or purified from the culture medium or ascites fluid by conventional immunoglobulin purification procedures such as protein A-Sepharose, hydroxylapatite chromatography, gel electrophoresis, dialysis, ammonium sulfate precipitation or affinity chromatography.

The mAbs may also be made by recombinant DNA methods, such as those described in U.S. Patent No. 4,816,567. DNA encoding the mAbs of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of mAbs in the recombinant host cells. The DNA also may be modified, for example, by substituting the coding sequence for human heavy and light chain constant domains in place of the homologous murine sequences (U.S. Patent No. 4,816,567; Morrison et al., 1987), or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

The antibodies may be monovalent antibodies. Methods for preparing monovalent antibodies are well known in the art. For example, one method involves recombinant expression of immunoglobulin light chain and modified heavy chain. Heavy chain truncations generally at any point in the Fc region will prevent heavy chain crosslinking. Alternatively, the relevant cysteine residues are substituted with another amino acid residue or are deleted, preventing crosslinking. *In vitro* methods are also suitable for preparing monovalent antibodies. Antibodies can be digested to produce fragments, such as Fab fragments, using routine techniques known in the art.

Anti-BFIT antibodies may further comprise humanized antibodies or human antibodies. Humanized forms of non-human (e.g., murine) antibodies are chimeric immunoglobulins, immunoglobulin chains or fragments thereof (such as Fv, Fab, Fab', $F(ab')_2$ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit, having the desired specificity, affinity and capacity. In some instances, Fv framework residues of the human immunoglobulin are replaced by corresponding non-human residues. Humanized antibodies may also comprise residues that are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin (Jones et al., 1986; Riechmann et al., 1988; Presta, 1992).

Methods for humanizing non-human antibodies are well known in the art. Generally, a humanized antibody has one or more amino acid residues introduced into it from a non-human source. These non-human amino acid residues are often referred to as "import" residues, which are typically taken from an "import" variable domain. Humanization can be essentially performed by substituting rodent CDRs or CDR sequences for the corresponding sequences of a human antibody (Jones et al., 1986; Riechmann et al., 1988; Verhoeyen et al., 1988). Such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries (Hoogenboom and Winter, 1991; Marks et al., 1991). The techniques of Cole et al. (1985) and Boerner et al. (1991) also describe the preparation of human mAbs. Similarly, introducing human immunoglobulin genes into transgenic animals in which the endogenous immunoglobulin genes have been partially or completely inactivated can make human antibodies. Upon challenge, human antibody production is observed, which closely resembles that seen in humans in all respects, including gene rearrangement, assembly, and antibody repertoire. This approach is described in U.S. Patent Nos. 5,545,807; 5,545,806; 5,569,825; 5,625,126; 5,633,425; 5,661,016, and in Marks et al., (1992), Lonberg et al. (1994); Morrison (1994), Fishwild et al., (1996), Fishwild et al. (1996) and Lonberg and Huszar (1995).

Bi-specific antibodies are monoclonal, preferably human or humanized, that have binding specificities for at least two different antigens. In the present case, one of the binding specificities is for the BFIT, the other one is for any other antigen, preferably for a cell-surface protein or receptor or receptor subunit.

Methods for making bi-specific antibodies are known in the art. Traditionally, the recombinant production of bi-specific antibodies is based on the co-expression of two immunoglobulin heavy-chain/light-chain pairs, where the two heavy chains have different specificities (Milstein and Cuello, 1983). Because of the random assortment of immunoglobulin heavy and light chains, these hybridomas (quadromas) produce a potential mixture of ten different antibody molecules, of which only one has the correct bispecific structure. Affinity chromatography can purify the correct molecule. Similar procedures are disclosed in WO 93/08829 and Traunecker et al. (1991).

Antibody variable domains with the desired antibody-antigen combining sites can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bi-specific antibodies see, for example, Suresh et al. (1986).

The interface between a pair of antibody molecules can be engineered to maximize the percentage of heterodimers that are recovered from recombinant cell culture (WO 96/27011). The preferred interface comprises at least part of the CH3 region of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end products such as homodimers.

Bi-specific antibodies can be prepared as full length antibodies or antibody fragments (e.g. $F(ab')_2$ bi-specific antibodies). One technique to generate bi-specific antibodies exploits chemical linkage. Brennan et al. (1985) proteolytically cleave intact antibodies to generate $F(ab')_2$ fragments. These fragments are reduced in the presence of the dithiol complexing agent sodium arsenite to stabilize vicinal dithiols and prevent intermolecular disulfide formation. The generated Fab' fragments are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bi-specific antibody. The produced bi-specific antibodies can be used as agents for the selective immobilization of enzymes.

Fab' fragments may be directly recovered from E. *coli* and chemically coupled to form bi-specific antibodies. For example, Shalaby et al. (1992) described the production of a fully humanized bi-specific antibody $F(ab')_2$ molecule.

Each Fab' fragment was separately secreted from *E. coli* and directly coupled chemically *in vitro,* forming the bi-specific antibody. The resulting bi-specific antibody bound cells over expressing the ErbB2 receptor and normal human T cells, and triggered the lytic activity of human cytotoxic lymphocytes.

Various techniques for making and isolating bi-specific antibody fragments directly from recombinant cell culture have also been described. Kostelny et al. (1992) exploited leucine zippers to produce bi-specific antibodies. Leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also produce antibody homodimers. The "diabody" technology described by Hollinger et al. (1993) provides an alternative method to generate bi-specific antibody fragments. The fragments comprise a heavy-chain variable domain ($V_H$) connected to a light-chain variable domain ($V_L$) by a linker that is too short to allow pairing between the two domains on the same chain. The $V_H$ and $V_L$ domains of one fragment are forced to pair with the complementary $V_L$ and $V_H$ domains of another fragment, thereby forming two antigen-binding sites. Another strategy for making bi-specific antibody fragments by the use of single-chain Fv (sFv) dimers has also been reported (Gruber et al., 1994). Antibodies with more than two valencies are also contemplated, such as tri-specific antibodies (Tutt et al., 1991).

Exemplary bi-specific antibodies may bind to two different epitopes on a given BFIT polypeptide. Alternatively, cellular defense mechanisms can be restricted to a particular cell expressing the particular BFIT polypeptide: an anti-BFIT polypeptide arm may be combined with an arm that binds to a leukocyte triggering molecule, such as a T-cell receptor molecule (e.g. CD2, CD3, CD28, or B7), or to Fc receptors for IgG (FcγR), such as FcγRI (CD64), FcγRII (CD32) and FcγRIII (CD16). Bi-specific antibodies may also be used to target cytotoxic agents to cells that express a particular BFIT polypeptide. These antibodies possess a BFIT-binding arm and an arm which binds a cytotoxic agent or a radionuclide chelator, such as EOTUBE, DPTA, DOTA, or TETA. Another bi-specific antibody of interest binds the BFIT polypeptide and further binds tissue factor (TF).

Heteroconjugate antibodies are also within the scope of the present invention. Heteroconjugate antibodies are composed of two covalently joined antibodies. Such antibodies have, for example, been proposed to target immune system cells to unwanted cells (U.S. Patent No. 4,676,980) and for treatment of HIV infection (WO 91/00360; WO 92/200373; EP 03089). Antibodies prepared *in vitro* using known methods in synthetic protein chemistry, including those involving cross-linking agents, are contemplated. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

It may be desirable to modify the antibody of the invention with respect to effector function, so as to enhance the effectiveness of the antibody in treating a disease, such as cancer. For example, cysteine residue(s) may be introduced into the Fc region, thereby allowing interchain disulfide bond formation in this region. Such homodimeric antibodies may have improved internalization capability and/or increased complement-mediated cell killing and antibody-dependent cellular cytotoxicity (ADCC) (Caron et al., 1992; Shopes, 1992). Homodimeric antibodies with enhanced anti-tumor activity may also be prepared using hetero-bifunctional cross-linkers as described in Wolff et al. (1993). Alternatively, an antibody can be engineered having dual Fc regions and may thereby have enhanced complement lysis and ADCC capabilities (Stevenson et al., 1989).

Immunoconjugates may comprise an antibody conjugated to a cytotoxic agent such as a chemotherapeutic agent, toxin (e.g., an enzymatically active toxin of bacterial, fungal, plant, or animal origin, or fragments thereof), or a radioactive isotope (i.e., a radioconjugate).

Chemotherapeutic agents useful in the generation of such immunoconjugates have been described above. Useful enzymatically-active toxins and fragments include diphtheria A chain, non-binding active fragments of diphtheria toxin, exotoxin A chain from *Pseudomonas aeruginosa,* ricin A chain, abrin A chain, modeccin A chain, α-sarcin, *Aleurites fordii* proteins, dianthin proteins, *Phytolaca americana* proteins (PAPI, PAPII, and PAP-S), momordica charantia inhibitor, curcin, crotin, *Sapaonaria officinalis* Inhibitor, gelonin, mitogellin, restrictocin, phenomycin, enomycin, and the tricothecenes. A variety of radionuclides are available for the production of radioconjugated antibodies. Examples include $^{212}$ BI, $^{131}$I, $^{131}$In, $^{90}$Y, and $^{186}$Re.

Conjugates of the antibody and cytotoxic agent are made using a variety of bi-functional protein-coupling agents, such as N-succinimidyl-3-(2-pyridyldithiol) propionate (SPDP), iminothiolane (IT), bi-functional derivatives of imidoesters (such as dimethyl adipimidate HCl), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6- diisocyanate), and bis-active fluorine compounds (such as 1,5-difluoro-2,4-dinitrobenzene). For example, a ricin immunotoxin can be prepared as described by Vitetta et al. (1987). $^{14}$C-labeled 1-Isothiocyanatobenzyl-3-methyldiethylene triaminepentaacetic acid (MX-DTPA) is an exemplary chelating agent for conjugating radionuclide to the antibody (WO94/11026).

The antibody may be conjugated to a "receptor" (such as streptavidin) for utilization in tumor pre-targeting wherein the antibody-receptor conjugate is administered to the patient, followed by removal of unbound conjugate from the circulation using a clearing agent and then administration of a "ligand" (e.g., biotin) that is conjugated to a cytotoxic agent (e.g., a radionuclide).

Liposomes containing the antibody may also be formulated. Such immunoliposomes are prepared by methods known in the art, such as described by Epstein et al. (1985), Hwang et al. (1980), U.S. Patent Nos. 4,485,045 and 4,544,545. Liposomes with enhanced circulation time are disclosed in U.S. Patent No. 5,013,556.

Particularly useful liposomes can be generated by the reverse-phase evaporation method with a lipid composition comprising phosphatidylcholine, cholesterol, and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al. (1982) via a disulfide-interchange reaction. A chemotherapeutic agent (such as Doxorubicin) is optionally contained within the liposome (Gabizon et al., 1989). Other useful liposomes with different compositions may also be useful.

Gene expression may be measured at the mRNA level or at the protein level. To measure mRNA levels, conventional Northern blotting (Thomas, 1980), dot blotting, nuclear run-off assays or *in situ* hybridization may be used. These assays employ an appropriately labeled probe based on the sequences provided herein. Alternatively, antibodies may be employed that can recognize specific duplexes, including DNA duplexes, RNA duplexes, DNA-RNA hybrid duplexes or DNA-protein duplexes. The antibodies in turn may be labeled, and the assay may be carried out where the duplex is bound to a surface, so that upon the formation of duplex on the surface, the presence of antibody bound to the duplex can be detected.

Gene expression can be detected and measured at the protein level. Cells, tissue sections, cultured cells and/or culture media, or body fluids, can be used to quantify directly the expression of a gene product. Examples of useful techniques include immunohistochemical staining, immunoprecipitation and Western blotting; these exploit the antibodies of the invention.

"Antagonist" is used in the broadest sense and includes any molecule that partially or fully blocks, inhibits, or neutralizes a biological activity of a native BFIT polypeptide. Similarly, "agonist" is used in the broadest sense and includes any molecule that mimics a biological activity of a native BFIT polypeptide. Molecules that can act as agonists or antagonists include antibodies or antibody fragments, fragments or variants of native BFIT polypeptides, peptides, antisense oligonucleotides, small organic molecules, etc.

Expression cloning relies on the ability to detect bound ligand, such as BFIT polypeptide. A direct measure employs physical methods to detect bound BFIT. BFIT is labeled with a molecular and detectable "tag", such as a radionuclide or an enzyme that reacts with a substrate to produce a chemiluminescent or colored, insoluble product. Labeled BFIT is applied to the cells, the cells are processed for the detection method, and the label detected. Signal significantly greater than controls indicates specifically bound BFIT polypeptide.

A physical approach for identification of proteins which interact with BFIT relies on the ability to cross-link labeled BFIT polypeptide to an interacting protein(s). Cell membrane or extract preparations that express the interacting protein are exposed to labeled BFIT polypeptide, proteins are cross-linked to neighboring proteins by reagents well-known in the art, such as *N*-hydroxysuccinimide esters-maleimide crosslinkers (e.g., *m*-maleimidobenzoyl-*N*-hydroxysuccinimide ester or succinimidyl 4-(*p*-maleimidophenyl)butyrate) or imidoesters crosslinkers (e.g. dimethyl adipimidate 2 HCI or dimethyl suberimidate 2 HCl). Cross-linked proteins are extracted, resolved (such as by sodium dodecyl sulfate polyacrylamide gel electrophoresis) and the BFIT-labeled complex detected appropriately for the label used. The labeled complex is then excised, resolved into peptide fragments, and subjected to protein micro-sequencing. The resulting amino acid sequence can be used to design degenerate oligonucleotide probes to screen a cDNA library, identifying the gene encoding the putative interacting protein.

Preferably, expression cloning is employed. Polyadenylated RNA is prepared from a cell responsive to the BFIT polypeptide and a cDNA library prepared. The cDNAs are divided into pools and transfected into COS fibroblasts or other cells that are usually unresponsive to the BFIT polypeptide. Those cDNA pools that render cells responsive to BFIT polypeptide indicate the presence of a cDNA encoding a BFIT interacting protein or other BFIT-sensitive molecule. Positive pools are subdivided into smaller pools, and the process is repeated until the sequence(s) that renders the cells responsive to BFIT polypeptides is isolated as a single clone encoding a putative BFIT interacting protein.

To assay for antagonists or agonists, the BFIT polypeptide may be added to a cell along with the compound to be screened for a particular activity. If the compound inhibits the activity of interest in the presence of the BFIT polypeptide, that compound is an antagonist to the BFIT polypeptide. If the compound increases the activity of interest in the presence of the BFIT polypeptide, that compound is an agonist to the BFIT polypeptide.

Any class of molecule that inhibits BFIT polypeptide cellular effects is a candidate antagonist. Likewise any class of molecule that enhances BFIT polypeptide cellular effects is a candidate agonist. Screening techniques well known to those skilled in the art can identify these molecules. Examples of antagonists and agonists include: 1) small organic and inorganic compounds, 2) small peptides, 3) antibodies and derivatives, 4) polypeptides closely related to BFIT, 5) antisense DNA and RNA, 6) ribozymes, and 7) triple DNA helices.

Agonists and antagonists may be small molecules that bind to the BFIT polypeptide active site, or other relevant part of the polypeptide or any corresponding interacting protein and inhibit the biological activity of the BFIT polypeptide are antagonists. Examples of small molecules include small peptides, peptide-like molecules, preferably soluble, and synthetic non-peptidyl organic or inorganic compounds.

Any antibody that affects BFIT polypeptide's effects on cells is a candidate antagonist or agonist. Examples of antibody antagonists or agonists include polyclonal, monoclonal, single-chain, anti-idiotypic, chimeric antibodies, or humanized versions of such antibodies or fragments. Antibodies may be from any species in which an immune response can be raised. Humanized antibodies are also contemplated.

Alternatively, a potential antagonist or agonist may be a closely related protein, for example, a mutated form of the BFIT polypeptide that recognizes the substrate but has no activity, thereby competitively inhibiting BFIT polypeptide action.

Antisense RNA or DNA constructs can be effective antagonists. Antisense RNA or DNA molecules block function by inhibiting translation by hybridizing to targeted mRNA. Antisense technology can be used to control gene expression through triple-helix formation or antisense DNA or RNA, both of which are based on polynucleotide binding to DNA or RNA. For example, the 5' coding portion of the BFIT polynucleotide sequence is used to design an antisense RNA oligonucleotide of from about 10 to 40 base pairs in length. A DNA oligonucleotide is designed to be complementary to a region of the gene involved in transcription [triple helix - see Lee et al. (1979); Cooney et al., (1988); (Dervan, 1991)], thereby preventing transcription and the production of the BFIT polypeptide. The antisense RNA oligonucleotide hybridizes to the mRNA *in vivo* and blocks translation of the mRNA molecule into the BFIT polypeptide (Okano (1991); Oligodeoxynucleotides as Antisense Inhibitors of Gene Expression (CRC Press: Boca Raton, FL, 1988)). The above described oligonucleotides can also be delivered to cells such that the antisense RNA or DNA may be expressed *in vivo* to inhibit production of the BFIT polypeptide. When antisense DNA is used, oligodeoxyribonucleotides derived from the translation-initiation site, e.g., between about -10 and +10 positions of the target gene nucleotide sequence, are preferred.

Antisense or sense oligonucleotides bind to target nucleic acid sequences, forming duplexes that block transcription or translation of the target sequence. These duplexes inhibition BFIT protein expression by enhanced degradation of the duplexes, premature termination of transcription or translation, or by other means.

Antisense or sense oligonucleotides are single-stranded nucleic acids, either RNA or DNA, which can bind target BFIT mRNA (sense) or BFIT DNA (antisense) sequences. Antisense or sense oligonucleotides comprise a fragment of the BFIT DNA coding region of at least about 14 nucleotides, preferably from about 14 to 30 nucleotides. In general, antisense RNA or DNA molecules can comprise at least 5, 10, 15, 20, 25, 30, 35, 40, 45, 50, 55, 60, 65, 70, 75, 80, 85, 90, 95, 100 bases in length or more. Among others, Stein and Cohen (1988) and van der Krol et al. (1988) describe methods to derive antisense or a sense oligonucleotides from a given cDNA sequence.

Modifications of antisense and sense oligonucleotides can augment their effectiveness. Modified sugar-phosphodiester bonds or other sugar linkages, such as described in WO 91/06629, increase *in vivo* stability by conferring resistance to endogenous nucleases without disrupting binding specificity to target sequences. Other modifications include covalently linked organic moieties, such as those described in WO 90/10048, and other moieties, such as poly-(L)-lysine, that increase antisense and sense oligonucleotide affinities for a target nucleic acid sequence. Other attachments to sense and antisense oligonucleotides include metal complexes or intercalating (e.g. ellipticine) and alkylating agents. These attachments can modify binding specificities of the antisense or sense oligonucleotide for the target nucleotide sequence.

To introduce antisense or sense oligonucleotides into target cells (cells containing the target nucleic acid sequence), any gene transfer method may be used and are well known to those of skill in the art. Examples of gene transfer methods include 1) biological, such as gene transfer vectors like Epstein-Barr virus or conjugating the exogenous DNA to a ligand-binding molecule (liposomes), 2) physical, such as electroporation, and 3) chemical, such as $CaPO_4$ precipitation and oligonucleotide-lipid complexes.

In a preferred procedure, an antisense or sense oligonucleotide is inserted into a suitable gene transfer retroviral vector. A cell containing the target nucleic acid sequence is contacted with the recombinant retroviral vector, either *in vivo* or *ex vivo.* Examples of suitable retroviral vectors include those derived from the murine retrovirus M-MuLV, N2 (a retrovirus derived from M-MuLV), or the double copy vectors designated DCT5A, DCT5B and DCT5C (described in WO 90/13641).

A procedure that specifies target cells in a mixed population of cells exploits cell surface receptors that are specific to the target cells. Antisense and sense oligonucleotides are conjugated to a ligand-binding molecule, as described in WO 91/04753. Ligands are chosen for receptors that are specific to the target cells. Examples of suitable ligand-binding molecules include cell surface receptors, growth factors, cytokines, or other ligands that bind to cell surface receptors or molecules. Preferably, conjugation of the ligand-binding molecule does not substantially interfere with the ability of the receptors or molecule to bind the ligand-binding molecule conjugate, or block entry of the sense or antisense oligonucleotide or its conjugated version into the cell.

Liposomes efficiently transfer sense or an antisense oligonucleotide to cells, such as described in WO 90/10448. The sense or antisense oligonucleotide-lipid complex is preferably dissociated within the cell by an endogenous lipase.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques (Rossi, 1994; WO 97/33551).

To inhibit transcription, triple-helix nucleic acids that are single-stranded and comprise deoxynucleotides are useful antagonists. These oligonucleotides are designed such that triple-helix formation via Hoogsteen base-pairing rules is promoted, generally requiring stretches of purines or pyrimidines (WO 97/33551).

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONICS.

In a gene therapy approach, introduced genes are expressed *in vivo* to achieve a therapeutic affect. For example, an introduced gene may encode a functional protein to replace a defective protein encoded from a mutated gene. Nucleic acids encoding the BFIT polypeptides may be useful in gene therapy.

Gene therapies may be transient and permanent. To achieve a permanent effect, the design of the therapy recurrently administers the introduced therapeutic nucleic acid. Individuals who suffer from a disease or condition caused by a mutant gene can be effectively treated in this way. To achieve a transient effect, the design of the therapy administers the therapeutic nucleic acid once or several times, but the nucleic acid does not persist in the individual over time. This approach is effective in treating conditions that are not chronic, such as an infection, or can be used to ameliorate a condition, such as inducing repair in damaged tissues.

Antisense RNAs and DNAs can be used as therapeutic agents to block the expression of certain genes *in vivo.* While cells inefficiently import short antisense oligonucleotides resulting in low intracellular concentrations, gene expression can still be inhibited (Zamecnik et al., 1986). Substituting negatively charged phosphodiester groups by uncharged groups on oligonucleotides may increase the efficiency of cell uptake of therapeutic nucleic acids.

Many techniques are available for introducing nucleic acids into viable cells, the choice depending on whether the nucleic acid is transferred to cells *in vitro* or *in vivo.* Suitable *in vitro* techniques include methods that use liposomes, electroporation, microinjection, cell fusion, DEAE-dextran, calcium phosphate precipitation, etc. Preferred *in vivo* gene transfer techniques include transfection with viral (typically retroviral) vectors and viral coat protein-liposome mediated transfection (Dzau et al., 1993). Other vectors that are currently useful are summarized by Romano et al. (2000). In some situations, only certain cells will be desirable targets of gene therapies. Nucleic acids can be coupled with an agent that targets the desired cells, such as an antibody specific for a cell surface membrane protein on the target cell, or a ligand for a receptor on the target cell, etc. For example, molecules that bind specifically to the target cell could be incorporated into the outer layer of a liposome that contains the nucleic acid. An example of proteins that would confer such targeting include those that would bind a cell surface receptor that is regularly endocytosed (to facilitate uptake; Wu et al., 1987; Wagner et al., 1990), antibodies for proteins that are internalized, and proteins that target intracellular localization and enhance intracellular half-life. The technique of receptor-mediated endocytosis is described, for example, by. For review of gene marking and gene therapy protocols see Anderson et al. (1992).

## EXAMPLES

*Animals & Tissue Collection.* All experiments were done in accordance with the guidelines set forth by the Institutional Animal Care and Use Committee at Genentech. Differential gene expression experiments were carried out using newly-weaned male FVB-N mice (TACONIC, Germantown, N.Y.), received at 3 wk of age and housed at 2 mice/cage until tissue harvest at 6 wk of age. Mice were fed standard rodent chow *ad libitum* (Chow 5010, RALSTON PURINA, St. Louis, MO), and housed on a 12:12 light/dark cycle (lights on 06:00). Control and Cold-Challenged mice were housed at an ambient temperature ($T_a$) of 22°C during this period, whereas Warm-Acclimated mice were housed at 33°C, within their thermoneutral zone (TNZ). Cold-Challenged mice were transferred to a 4°C room for 48 hr prior to tissue harvest. Following $CO_2$-induced euthanasia in the afternoon, interscapular BAT was excised, cleaned of visible blood vessels, white adipose tissue (WAT), and connective tissue, and snap-frozen for subsequent RNA preparation. For differential expression, 3 independent BAT samples/treatment were generated for analysis; each sample was composed of BAT pooled from 10 mice. Follow-up studies examining the timecourse of cold-inducibility of BAT genes were carried out on Cold-Challenged mice exposed to 4°C (1, 6, and 24 hr), whereas studies examining the tissue distribution and cold-inducibility across tissues employed pooled samples (at least 3/tissue) from additional mice.

Some experiments employed a high-fat diet regimen to compare BAT gene expression differences in model systems of obesity-prone versus obesity-resistant mice. The diet, containing 58% of calories as fat, was patterned after that formulated by R.D. Suwit (Surwit et al., 1995). Male mice received at 4wk of age were immediately placed on the diet, with interscapular BAT harvest occurring at 7 wk of age. The first study compared expression in AKR/J vs. SWR/J mice, whereas the second study compared expression in C57BL/6J vs. A/J mice (JACKSON LABS, Bar Harbor, ME). Additional studies compared expression in 9 wk old male ob/ob mice vs. lean Ob/? controls, fed standard rodent chow *ad libitum.*

*Example 1: Differential Gene Expression Analysis*

Interscapular BAT mRNA samples were prepared, reverse-transcribed, and subjected to CURAGEN Quantitative Expression Analysis (QEA). The details of QEA analysis are described elsewhere (Shimkets et al., 1999). Analysis focused on identification of genes regulated at least 2-fold by changes in $T_a$. Follow-up analyses, using quantitative real-time RT-PCR (TaqMan) under conditions described previously (Yu et al., 2000), employed specific primers and FAM/TAMRA-labeled probes recognizing mBFIT or hBFIT as follows (all are 5'□3'): Mouse BFIT:

fwdTGAAGGATACCGGAACCCC (SEQ ID NO 7);
probeCGGAGGTGCAGATGAGCCAGCTG (SEQ ID NO 8);
revTACTGCCCTGCCACACCAA (SEQ ID NO 9).

Human BFIT1: fwdTGCTGGGTTAGGGTCTCCCT (SEQ ID NO 10); probeACTGAGCTGGTCTCGGCAAGTGGC (SEQ ID NO 11); revTCTATTCCTGGGGGCTCGA (SEQ ID NO 12).

Human BFIT2: fwdTCTCTTGGACAACCGGAATGA (SEQ ID NO 13); probeTGGCCCCCAGCCTCCAGACC (SEQ ID NO 14); revTCTAGATGCCCTCAGTGGCC (SEQ ID NO 15).

All quantitative RT-PCR data were normalized using 18S RNA abundance to account for loading differences, using commercially-available primer/probe sets (PE APPLIED BIOSYSTEMS, Foster City, CA). Human tissue mRNAs analyzed by real-time quantitative RT-PCR were derived from the following sources: a human tissue mRNA panel (CLONTECH, Palo Alto, CA), white adipose tissue (ZENBIO, Research Triangle Park, NC), or a human hibernoma sample derived from a 52 yr old female and procured from the University of Michigan.

*Example* 2: *Radiation Hybrid Analysis.*

The human BFIT gene localization was carried out using the BFIT-specific oligo sets described above. Real-time RT-PCR was performed on the Stanford G3 Radiation Hybrid Panel of 83 human/hamster somatic clones. Positive clones were identified as having a signal above background and with a cycle threshold below 33 on the PCR amplification plot. The data were submitted to the Stanford server (http://www-shgc.stanford.edu/RH/G3index.html), and results were returned with linkage on Chromosome 1 with a LOD score of 15.7. Cytogenetic localization was obtained through the Genome Database (http://www.gdb.org). The mouse BFIT chromosomal locus was identified through radiation hybrid analysis of 93 hybrids of the Mouse/Hamster T31 Radiation Hybrid Panel (Research Genetics, Huntsville, AL), employing PCR primers designed from the g1i0-182 fragment sequence (see Results) as follows (5'□3'):

fwdGTAAGAAGGGAGCCTGGGAG (SEQ ID NO 16) and
revTCTAGACCACCCTTTCTCCG (SEQ ID NO 17). A collective set of scores (0=no amplification; 1=amplification; 2=uncertain) were used to assess the radiation hybrid vectors, and analyses were performed in duplicate. Chromosomal placement of the mBFIT gene was accomplished using the Whitehead Institute/Massachusetts Institute of Technology Center for Genome Research website (http://www.genome.wi.mit.edu/cgi-bin/mouse-_rh/rhmap-auto/rhmapper.cgi). The LOD score for linkage was 15.

*Statistics.* Comparisons between any two treatments were made using a Student's t-test, whereas statistical differences across temperature treatments were assessed using a Newman-Keuls treatment with $p<0.05$ considered statistically significant. All values presented are means ± SEM of between 3-5 samples per treatment unless otherwise noted.

*Example 3: Identification of BAT*

A 182 bp cDNA fragment (g1i0-182) corresponding to an mRNA upregulated in the BAT of Cold-Challenged mice was identified through Quantitative Expression Analysis, shown in Figure 1. In this figure, BAT mRNA samples derived from 6 wk old male Cold-Challenged mice (4°C, 48 hr), controls (22°C), or Warm-Acclimated mice (33°C, 3 wk) were subjected to a QEA analysis designed to uncover temperature-sensitive genes. Peak height corresponds to the abundance of a ~182 bp cDNA fragment, which in turn represents the abundance of a specific mRNA species in the original sample. The abundance of the fragment (later determined to represent murine BFIT) is highest in the BAT from cold mice (A), lower in controls (B), and negligible in warm mice (C). The bp values on the X axis are derived from gel location.

Sequencing of this short fragment suggested that it represented a portion of the 3' untranslated region (UTR) of a novel gene, as it lacked homology with genes or proteins in the public database, and did not display an obvious open reading frame (ORF). The fragment sequence was utilized in strategies to obtain extended sequences from clones derived from an FVB/N murine BAT cDNA library. A number of clones contained a 594 amino acid ORF with high homology (81% identity, 83% similarity) to a hypothetical human acyl-CoA thioesterase KIAA0707 (GenBank Accession No. AB014607), predicted to encode a protein with a molecular weight of 68 kDa (Figure 2). None of the isolated clones contained an in-frame Stop codon upstream of the first Met, but contig analysis identified matching overlapping murine ESTs (W64130 & AA051663) which extended the sequence further 5' to an in-frame Stop. Furthermore, the human genomic region containing the entire ORF of KIAA0707 (see below) also contains an in-frame Stop prior to the Met.

Interestingly, there appeared to be significant sequence divergence in the carboxy-terminus of the murine protein compared to KIAA0707, beginning at residue 544 of the human protein (Figure 2). This result raised the possibilities that alternative splice variants exist for the human gene, or that a separate gene may encode a protein orthologous to the version found in mouse BAT. The divergent mouse carboxy terminal sequence was therefore used to search the public database for orthologous human ESTs, two of which were identified and found to include a Stop codon toward their 3' end (ESTs AA587744 & AI732328). Employing a reverse primer specific to this region (5'CCAGACCCTCTAGATGCCCTCA3'; SEQ ID NO 18) and a forward primer designed from the AB014607 sequence (5'ATGATCCAGAATGTCGGAAATCAC3'; SEQ ID NO 19), a 1794 bp cDNA fragment containing the entire ORF of this variant was generated by PCR using human hibernoma (a benign tumor with characteristics resembling BAT) cDNA as a template, and subcloned into the pcDNA3.1/V5-His TOPO TA vector (INVITROGEN, Carlsbad, CA). A 1878 bp cDNA encoding the entire ORF of KIAA0707 was also PCR-generated and cloned from hibernoma cDNA, using the AB014607 forward primer and the reverse primer (5'AGACACCTGAAACCTTATCATGAGCC3'; SEQ ID NO 20). The amino-terminal sequences of the proteins encoded by these cDNAs matched that of KIAA0707, but the sequences diverged at the carboxy-terminus, analogous with the mouse sequence compared to KIAA0707 (Figure 2). Both forms of the protein possess two acyl-CoA thioesterase domains and a START lipid-binding domain, indicative of involvement with lipid metabolism. Thus, based on domain structure and the expression pattern in the mouse, KIAA0707 protein was renamed "hBFIT1," with the novel orthologous variants termed "hBFIT2" and "mBFIT2" in humans and mice, respectively. hBFIT1 was found to share the highest homology (30-35% identity; 50-55% similarity) with a variety of acyl-CoA thioesterases identified in *Chlamydophila* and *Bacillus* (i.e., AP002547_137 & AP001509_236, respectively), but also appeared related to the human or rat thioesterase CTE-2 (CTE2_HUMAN & CTE2_RAT, respectively) at 22% identity and 41% similarity.

Figure 2 shows alignment of BFIT sequences from human (hBFIT) and mouse (mBFIT), and indicates the strong conservation of BFIT across species, and the presence of alternative splicing events. The divergence of hBFI1 and hBFIT2 sequences at the carboxy terminus is due to alternative splicing of the human BFIT gene. Bars denote G-X-H motifs conserved in some thioesterase proteins. The following Pfam domains were identified: START lipid-binding domain (residues 384-590 & 384-571 in hBFIT1 and hBFIT2, respectively); two acyl-CoA thioesterase domains (residues 29-166 and residues 199-341 in both hBFIT variants).

Figure 3 shows that BFIT is strongly and rapidly induced in the BAT of mice exposed to cold, and repressed by warm temperature. In Figure 3A, BFIT mRNA level in BAT was induced by 24 hr of cold exposure (4°C) in adult male mice, and remained elevated through at least 48 hr in the cold (*p<0.05 compared to control). In contrast, acclimation to a thermoneutral environment (33°C, 3 wk) depressed expression by ~70% (**p<0.01 vs. control, p<0.05 vs. others). Figure 3B indicates BFIT mRNA is most abundant in mouse BAT, and its cold-inducibility is strikingly apparent in this tissue. The figure depicts expression in pooled mRNAs from each tissue noted, and represent different samples than those in (A). Expression levels after cold-exposure, control conditions, or upon warm acclimation are indicated by three bars (left-to-right) for white adipose tissue (WAT), whole brain, liver, and hindlimb skeletal muscle (SKM); kidney, heart, and testis values were derived from control mice.

*Example 4: BfIT Association with Chromosomal Markers Associated with Body Fat*

Radiation hybrid analyses were carried out to identify the chromosomal localizations of hBFIT and mBFIT. Interestingly, murine BFIT was co-localized with the markers D4Mit168 and D4Mit43 on Chromosome 4, which rest in the region defined by the quantitative trait locus (QTL) Dietary-obese 1 (*Do1*) as described (West et al., 1994). This QTL was discovered in a genetic analysis of obesity-prone AKR/J mice versus obesity-resistant SWR/J mice. *Do1* was hypothesized to contain a gene which plays a role in driving the large differences in body fat content between the strains, observed after feeding a high-fat diet (West et al., 1994). Human BFIT was localized to a syntenic region of Chromosome 1 (1 p32.3), near the Stanford Human Genome Center marker SHGC-58180 (between D1S509 & D1S3255). This marker is in proximity to a potential human *Do1* region linked to body fatness in the Québec Family Study cohort (includes the markers D1S193, D1S200, & D1S476) (Chagnon et al., 1997).

The chromosomal position of BFIT, its involvement with lipid metabolism, and its high abundance and induction in thermogenic BAT pointed to BFIT as a candidate gene defining the *Do1* locus. To test this postulate, a comparison was made of the full-length ORF of mBFIT2 in AKR/J and SWR/J mice, using BAT cDNAs from each strain as templates in multiple, independent PCR reactions. The fragments obtained were subcloned into pcDNA3.1/V5-His-TOPO and sequenced. The cDNA and protein sequences corresponding to the mBFIT2 ORF did not differ between strains, and matched that obtained previously from FVB/N mice (Figure 2). Because a putative strain-related difference in the BFIT gene sequence could occur outside the coding region and hence lead to expression differences among strains, we explored whether BAT expression of BFIT was altered in AKR/J versus SWR/J mice fed a high-fat diet for 3 wk. This treatment led to a substantial difference in body fat accretion between strains, as judged by the 4-fold higher epididymal fat pad weight (as a % of body wt) in AKR/J mice (4.0 ± 0.29%) compared to SWR/J mice (1.0 ± 0.11 %)($p<0.01$)(also see (West et al., 1994). The abundance of BFIT was significantly ($p<0.001$) elevated ~1.6-fold in the BAT of SWR/J mice compared to obesity-prone AKR/J mice (Figure 4A). Similar patterns emerged from additional lean/obese comparisons of BFIT expression in different established models. For instance, a comparison between obesity-resistant A/J versus obesity-prone C57BL/6J mice (Surwit et al., 1995) fed a high-fat diet 3 wk revealed a 2-fold higher expression of BFIT in the A/J mice (Figure 4B) ($p<0.001$). Furthermore, BFIT expression in the BAT of non-obese Ob/? mice was 2.5-fold elevated compared to obese ob/ob mice (Figure 4C) ($p<0.001$).

*Example 5: Organization of the Human BFIT Gene, & Evidence for Tissue-Specific Regulation of hBFIT Splicing Events*

The presence of two hBFIT cDNA variants and a single chromosomal location for BFIT suggested the presence of alternative splicing events in the hBFIT gene. A ~155 kb assembly encompassing the hBFIT genomic region was made *in silico,* using information contained in two human unordered, nondirectional Phase I genomic clones from the public database (AC025990 & AC011736) which contained the hBFIT1/hBFIT2 ORFs. hBFIT variants arise from alternative splicing events, as depicted schematically in Figure 5: hBFIT1 is predicted to arise from 17 exons, compared to the 16 exons giving rise to BFIT2. This figure illustrates exons (upward ticks) encoding hBFIT1 and hBFIT2 splice variants, plus intervening introns represented by the horizontal line. Both variants share the initial 15 exons of the gene, but hBFIT2 is generated from an alternate exon (see bottom arrow) not utilized in hBFIT1 transcription. In contrast, hBFIT1 is generated from the use of two downstream exons; (see top arrow) not used to generate the hBFIT2 sequence.

In humans, an interesting tissue-specificity of hBFIT splicing patterns emerged from quantitative real-time RT-PCR assays employing variant-specific primers and probes. Figure 6 shows the broad expression of BFIT in humans, and tissue-specific regulation of gene-splicing events. Shown in Figure 6A, BFIT1 mRNA, expressed as a percentage of that determined in hibernoma (a benign tumor with characteristics of BAT), was especially abundant in heart, but was also apparent in numerous other tissues (bars are identified on the bottom panel). Shown in Figure 6B, BFIT2 mRNA distribution was more confined versus BFIT1, but nevertheless expressed in numerous tissues. Shown in Figure 6C, the ratio of BFIT splice variants was quite variable, with tissue-specific patterns indicative of differential regulation of transcription. Values represent the percentage of total BFIT mRNA (BFIT1 plus BFIT2 mRNAs) attributable to either variant in each tissue examined (e.g. hibernoma predominantly expressed BFIT2, represented by the crosshatched bar).

Regulation of this gene was further analyzed in a follow-up experiment designed to dissect whether repression of the BFIT gene in Warm-Acclimated mice (Figure 3A) was attributed to specific temperature signals or due to diminished food intake (warm mice ate ~50% less versus controls). Expression was checked in a group of mice kept 3 wk at 22°C, but meal-fed the same amount of chow as Warm-Acclimated mice. Surprisingly, BFIT expression in food-restricted mice was induced to 204±47% of control values (n=3, $p<0.05$; range 186-455%), similar in magnitude to observations in Cold-Challenged mice (Figure 3A). These results indicate that the warmth-related repression of the gene was due to physiological signals specific to ambient temperature. Furthermore, the data suggest that factors common to both long-term food restriction and acute cold-exposure trigger the gene, or that separate condition-specific cohorts of regulators impact the gene in the same direction. Lipolysis is increased in the BAT of cold-exposed mice, and perhaps increased non-esterified fatty acid (NEFA) availability played a part in BFIT induction under cold and food-restricted conditions.

*Example 6: Isolafion of cDNA Clones Encoding Mouse BFIT Gene*

The Mouse BFIT is a novel gene encoding a 1782 bp (594 amino acid (~67 kDa)) open reading frame (ORF). The total length of the isolated mouse BFIT gene was 2699 kb; the vector pRK5D used was 5.1 kb, thus adding to a total length of 7.8 kb of the DNA molecule amplified by inverse polymerase chain reaction (IPCR) and isolated.

Mouse BFIT gene was cloned as follows. Two adjacent 5' phosphorylated primers, e6.bat.snr1 and e6.bat.snf1, were designed on opposite strands with melting temperatures of 70°C and 69°C respectively. These primers were used in an inverse PCR reaction. The PCR primer sequences (5' to 3') were as follows:

e6.bat.snr1 >pGCCACTGAGTCAGAGTCCAGCCAAC (SEQ ID NO 21) and e6.bat.snf1 >pCCAGCCTGCAGCTGGAAGGAC (SEQ ID NO 22).

In a 50μl reaction, the following reagents were added: 50ng of a mouse brown adipose tissue cDNA library in the vector pRK5D, which was propagated in a methylation positive bacteria; 50 picomoles of each PCR primer; 10nmoles of each deoxynucleotide triphosphate, 5μl of Pfu 10x buffer (Stratagene, La Jolla CA), and 1μl of Pfu Turbo (Stratagene, La Jolla CA). The plasmid vector pRK5 (4,661 bp) has been described (EP 307,247 published 15 March 1989); pRK5D (5117 bp) is a derivative of pRK5.

PCR conditions were one cycle at 94°C for 3 minutes, then 20 cycles of 94°C for 30 seconds, 65°C for 30 seconds, 72°C for 13 minutes. The PCR reaction generated a linear 5' phosphorylated amplicon that contained the Toll 6 cDNA insert plus the pRK5D vector.

Next, 10μl) of the completed PCR reaction was ligated in a 100μl reaction containing the following other reagents: 10μl 10x T4 DNA ligase buffer (New England BioLabs, Beverly, MA), 4μl T4 DNA ligase (New England BioLabs, Beverly, MA), and 76μl H2O. The ligation was incubated at ambient temperature for 1 hour.

After 1 hour of ligation, 2μl of the restriction enzyme Dpnl (New England BioLabs, Beverly, MA) was added to the ligation reaction and the digestion was allowed to continue for 1 hour at 37°C. Dpnl will specifically digest methylated but not unmethylated DNA; therefore, the original mouse brown adipose tissue cDNA library which was used as a template will be digested, leaving only the mouse BFIT/vector amplicon intact. After the completion of the digestion the sample was cleaned using the QIAquick PCR purification kit (Qiagen, Valencia, CA), eluted in 30μl of elution buffer or H2O, and then ethanol precipitated. The pellet was resuspended in 2μl of H2O and the entire sample was then used to transform bacteria. DH10B ElectroMAX competent bacteria (Life Technologies, Rockville, MD) by electroporation. The transformed bacteria were plated on Luria broth agar plates and colonies were grown overnight at 37°C.

The next day, the colonies were lifted onto a nylon membrane, denatured, renatured and probed with a 32P-ATP kinase-labeled, mouse BFIT specific probe. The sequence of the mouse BFIT specific probe was e6.bat.p1 >AAATCCACATGGCTGTTCCCAGCAGTGCTGTGGTACACTGTGACAGT (SEQ ID NO 23). Positive colonies were sequenced to confirm that point mutations were not introduced by the PCR reaction.

*Example 7: Thioesterase Assay*

Human BFIT proteins were produced in *E. coli* transformed with appropriate vectors containing the BFIT protein-encoding nucleotide sequences. Figure 7A depicts thioesterase activity of partially-purified recombinant hBFIT1 and Figure 7B depicts partially-purified recombinant hBFIT2. Thioesterase activity is defined as the amount of coenzyme-A (CoASH) released from various acyl-CoA thioester substrates per minute per mg of protein. Both enzymes displayed the highest activity against C16-CoA, an acyl-CoA with 16 carbons on the fatty acid backbone, with progressively diminished activities against C12-CoA and C4-CoA. Measurement of release of coenzyme A into the medium was measured spectrophotometrically (Lee, K.Y. and H. Schulz 1979).

*Deposit of Material*

The following materials have been deposited with the American Type

Culture Collection, 10801 University Blvd., Manassas, VA 20110-2209, USA (ATCC):

| Material | ATCC Dep. No. | Deposit Date |
|---|---|---|
| pR1853.hBFIT1 | | January 17,2001 |
| pR1853.hBFIT2 | | January 17,2001 |

This deposit was made under the provisions of the Budapest Treaty on the International Recognition of the Deposit of Microorganisms for the Purpose of Patent Procedure and the Regulations thereunder (Budapest Treaty). This assures maintenance of a viable culture of the deposit for 30 years from the date of deposit.

The assignee of the present application has agreed that if a culture of the materials on deposit should die or be lost or destroyed when cultivated under suitable conditions, the materials will be promptly replaced on notification with another of the same. Availability of the deposited material is not to be construed as a license to practice the invention in contravention of the rights granted under the authority of any government in accordance with its patent laws.

REFERENCES:

EPO 402226. 1990. Transformation vectors for yeast *Yarrowia.*
WO 91/00357. 1991. New strain with filamentous fungi mutants, process for the production of recombinant proteins using said strain, and strains and proteins.
Alam, J., and J.L. Cook. 1990. Reporter genes: Application to the study of mammalian gene transcription. Anal. Biochem. 188:245-254.
Altschul, S.F., and W. Gish. 1996. Local alignment statistics. Methods Enzymol. 266:460-80.
Altschul, S.F., T.L. Madden, A.A. Schaffer, J. Zhang, et al. 1997. Gapped BLAST and PSI-BLAST: a new generation of protein database search programs. Nucleic Acids Res. 25:3389-402.
Austin, C.P., and C.L. Cepko. 1990. Cellular migration patterns in the developing mouse cerebral cortex. Development. 110:713-732.
Bechtold, N., and G. Pelletier. 1998. In planta Agrobacterium-mediated transformation of adult Arabidopsis thaliana plants by vacuum infiltration. Methods Mol Biol. 82:259-66.
Becker, D.M., and L. Guarente. 1991. High-efficiency transformation of yeast by electroporation. Methods Enzymol. 194:182-187.
Beggs, J.D. 1978. Transformation of yeast by a replicating hybrid plasmid. Nature. 275:104-109.
Berger, J., J. Hauber, R. Hauber, R. Geiger, et al. 1988. Secreted placental alkaline phosphatase: A powerful new qunatitative indicator of gene expression in eukaryotic cells. Gene. 66:1-10.
Bodine, D.M., K.T. McDonagh, N.E. Seidel, and A.W. Nienhuis. 1991. Survival and retrovirus infection of murine hematopoietic stem cells in vitro: effects of 5-FU and method of infection. Exp. Hematol. 19:206-212.
Capecchi, M.R. 1980. High efficiency transformation by direct microinjection of DNA into cultured mammalian cells. Cell. 22:479.
Case, M.E., M. Schweizer, S.R. Kushner, and N.H. Giles. 1979. Efficient transformation of Neurospora crassa by utilizing hybrid plasmid DNA. Proc Natl Acad Sci U S A. 76:5259-63.
Cepko, C.L., B.E. Roberts, and R.E. Mulligan. 1984. Construction and applications of a highly transmissible murine retrovirus shuttle vector. Cell. 37:1053-1062.
Chagnon, Y.C., L. Perusse, M. Lamothe, M. Chagnon, et al. 1997. Suggestive linkages between markers on human 1 p32-p22 and body fat and insulin levels in the Quebec Family Study. Obes Res. 5:115-21.
Chalfie, M., Y. tu, G. Euskirchen, W.W. Ward, et al. 1994. Green fluorescent protein as a marker for gene expression. Science. 263:802-805.
Chaney, W.G., D.R. Howard, J.W. Pollard, S. Sallustio, et al. 1986. Highfrequency transfection of CHO cells using Polybrene. Somatic Cell Mol. Genet. 12:237.
Chen, C., and H. Okayama. 1988. Calcium phosphate-mediated gene transfer: A highly efficient system for stably transforming cells with plasmid DNA. BioTechniques. 6:632-638.
Cohen, S.M.N., A.C.Y. Chang, and L. Hsu. 1972. Nonchromosomal antibiotic resistance in bacteria: Genetic transformation of Escherichia coli by R-factor DNA. Proc. Natl. Acad. Sci. USA. 69:2110.
de Louvencourt, L., H. Fukuhara, H. Heslot, and M. Wesolowski. 1983. Transformation of Kluyveromyces lactis by killer plasmid DNA. J Bacteriol. 154:737-42.
de Wet, J.R., K.V. Wood, M. DeLuca, D.R. Helinski, et al. 1987. Sturcture and expression in mammalian cells. Mol. Cell Biol. 7:725-737.
Dervan, P.B. 1991. Characterization of protein-DNA complexes by affinity cleaving. Methods Enzymol. 208:497-515.
Elroy-Stein, O., and B. Moss. 1990. Cytoplasmic expression system based on constitutive synthesis of bacteriophage T7 RNA polymerase in mammalian cells. Proc. Natl. Acad. Sci. USA. 87:6743-6747.
Escudero, J., and B. Hohn. 1997. Transfer and integration of T-DNA without cell injury in the host plant. Plant Cell. 9:2135-2142.
Fekete, D.M., and C.L. Cepko. 1993. Retroviral infection coupled with tissue transplantation limits gene transfer in the chick embryo. Proc. Natl. Acad. Sci. USA. 90:2350-2354.
Felgner, P.L., T.R. Gadek, M. Holm, R. Roman, et al. 1987. Lipofectin: A highly efficient, lipid-mediated DNA/ transfection procedure. Proc. Natl. Acad. Sci. USA. 84:7413-7417.
Finer, J.J., K.R. Finer, and T. Ponappa. 1999. Particle bombardment-mediated transformation. Current Topics in microbiology and immunology. 240:59-80.
Fleer, R., P. Yeh, N. Amellal, I. Maury, et al. 1991. Stable multicopy vectors for high-level secretion of recombinant human serum albumin by Kluyveromyces yeasts. Biotechnology (N Y). 9:968-75.
Fromm, M., L.P. Taylor, and V. Walbot. 1985. Expression of genes transferred into monocot and dicot plant cells by electroporation. Proc. Natl. Acad. Sci. USA. 82:5824-5828.
Fujita, T., H. Shubiya, T. Ohashi, K. Yamanishi, et al. 1986. Regulation of human interleukin-2 gene: Functional DNA sequences in the 5' flanking region for the gene expression in activated T lymphocytes. Cell. 46:401-407.
Gallagher, S.R. 1992. GUS protocols: Using the GUS gene as a reporter of gene expression. Academic Press, San Diego, CA.

Gietz, R.D., R.A. Woods, P. Manivasakam, and R.H. Schiestl. 1998. Growth and transformation of Saccharomyces cerevisiae. In Cells: A laboratory manual. Vol. I. D. Spector, R. Goldman, and L. Leinwand, editors. Cold Spring Harbor Press, Cold Spring Harbor, NY.
Gorman, C.M., L.F. Moffat, and B.H. Howard. 1982. Recombinant genomes which express chloramphenicol acetyltransferase in mammalian cells. Mol. Cell. Biol. 2:1044-1051.
Graham, F.L., and A.J. van der Eb. 1973. A new technique for the assay of infectivity of human adenovirus 5 DNA. Virology. 52:456-.
Hanahan, D. 1983. Studies on transformation of Escherichia coli with plasmids. J. Mol. Biol. 166:557-580.
Hansen, G., and M.-D. Chilton. 1999. Lessons in gene transfer to plants by a gifted microbe. Curr. Top. Microbiol. Immunol. 240:21-57.
Hansen, G., and M.S. Wright. 1999. Recent advances in the transformation of plants. Trends Plant Sci. 4:226-231.
Hinnen, A., J.B. Hicks, and G.R. Fink. 1978. Transformation of yeast. Proc. Natl. Acad. Sci. USA. 75:1929-1933.
Hoffman, F. 1996. Laser microbeams for the manipulation of plant cells and subcellular structures. Plant Sci. 113:1-11.
Ishikawa, K., T. Nagase, M. Suyama, N. Miyajima, et al. 1998. Prediction of the coding sequences of unidentified human genes. X. The complete sequences of 100 new cDNA clones from brain which can code for large proteins in vitro. DNA Res. 5:169-76.
Ishiura, M., S. Hirose, T. Uchida, Y. Hamada, et al. 1982. Phage particle-mediated gene transfer to cultured mammalian cells. Molecular and Cellular Biology. 2:607-616.
Ito, H., Y. Fukuda, K. Murata, and A. Kimura. 1983. Transformation of intact yeast cells treated with alkali cations. J. Bacteriol. 153:163-168.
Kaufman, R.J., P. Murtha, D.E. Ingolia, C.-Y. Yeung, et al. 1986. Selection and amplification of heterologous genes encoding adenosine deaminase in mammalian cells. Proc. Natl. Acad. Sci. USA. 83:3136-3140.
Kawai, S., and M. Nishizawa. 1984. New procedure for DNA transfection with polycation and dimethyl sulfoxide. Mol. Cell. Biol. 4:1172.
Kelly, J.M., and M.J. Hynes. 1985. Transformation of Aspergillus niger by the amdS gene of Aspergillus nidulans. Embo J. 4:475-9.
Leduc, N., and e. al. 1996. Isolated maize zygotes mimic in vivo embryogenic development and express microinjected genes when cultured in vitro. Dev. Biol. 10:190-203.
Lemischka, I.R., D.H. Raulet, and R.C. Mulligan. 1986. Developmental potential and dynamic behavior of hematopoietic stem cells. Cell. 45:917-927.
Littlefield, J.W. 1964. Selection of hybrids from matings of fibroblasts in vitro and their presumed recombinants. Science. 145:709-710.
Lopata, M.A., D.W. Cleveland, and B. Sollner-Webb. 1984. High-level expression of a chloramphenicol acetyltransferase gene by DEAEdextran-mediated DNA traansfection couled with a dimethylsulfoxide or glycerol shock treatment. Nucleic Acids Research. 12:5707.
Luckow, V.A. 1991. Cloning and expression of heterologous genes in insect cells with baculovirus vectors. In Recombinant DNA technology and applications. A. Prokop, R.K. Bajpai, and C. Ho, editors. McGraw-Hill, New York. 97-152.
Mandel, M., and A. Higa. 1970. Calcium-dependent bacteriophage DNA infection. J. Mol. biol. 53:159-162.
Miller, A.D., and C. Buttimore. 1986. Redesign of retrovirus packaging cell lines to avoid recombination leading to helper virus production. Mol. Cell biol. 6:2895-2902.
Miller, L.K. 1988. Baculoviruses as gene expression vectors. Annu. Rev. Microbiol. 42:177-199.
Neumann, E., M. Schaefer-Ridder, Y. Wang, and P.H. Hofschneider. 1982. Gene transfer into mouse lyoma cells by electroporation in high electric fields. EMBO J. 1:841-845.
O'Reilly, D.R., L.K. Miller, and V.A. Luckow. 1992. Baculovirus expression vectors. W.H. Freeman and Company, New York.
Ou-Lee, T.M., R. Turgeon, and R. Wu. 1986. Uptake and expression of a foreign gene linked to either a plant virus or Drosophila promoter in protoplasts of rice, wheat and sorghum. Proc. Natl. Acad. Sci. USA. 83:6815-6819.
Palmer, T.D., R.A. Hock, W.R.A. osborne, and A.D. Miller. 1987. Efficient retrovirus-mediated transfer and expression of a human adenosine deaminase gene in diploid skin fibroblasts from an adenosie-deficient human. Proc. Natl. Acad. Sci. USA. 84:1055-1059.
Pear, W., G. Nolan, M. Scott, and D. Baltimore. 1993. Production of high-titer helper-free retroviruses by transient transfection. Proc. Natl. Acad. Sci. USA. 90:8392-8396.
Ponting, C.P., and L. Aravind. 1999. START: a lipid-binding domain in StAR, HD-ZIP and signalling proteins. Trends Biochem Sci. 24:130-2. 4_00001362 4_00001362.
Potter, H. 1988. Electroporation in biology: Methods, applications, and instrumentation. Analytical Biochemistry. 174:361-373.
Potter, H., L. Weir, and P. Leder. 1984. Enhancer-dependent expression of human kappa immunoglobulin genes introduced into mouse pre-B lymphocytes by electroporation. Proc. Natl. Acad. Sci. USA. 81:7161-7165.
Rassoulzadegan, M., B. Binetruy, and F. Cuzin. 1982. High frequency of gene transfer after fusion between bacteria and eukaryotic cells. Nature. 295:257.
Rhodes, C.A., D.A. Pierce, I.J. Mettler, D. Mascarenhas, et al. 1988. Genetically transformed maize plants from protoplasts. Science. 240:204-207.
Rose, J.K., L. Buonocore, and M. Whitt. 1991. A new cationic liposome reagent mediating nearly quantitative transfection of animal cells. BioTechniques. 10:520-525.
Sandri-Goldin, R.M., A.L. Goldin, J.C. Glorioso, and M. Levine. 1981. Highfrequency transfer of cloned herpes simjplex virus type I sequences to mammalian cells by protoplast fusion. Mol. Cell. Biol. 1:7453-752.
Saunders, J.A., B.F. Matthews, and P.D. Miller. 1989. Plant gene transfer using electrofusion and electroporation. In Electroporation and electrofusion in cell biology. E. Neumann, A.E. Sowers, and C.A. Jordan, editors. Plenum Press, New York. 343-354.
Schaffner, W. 1980. Direct transfer of cloned genes from bacteria to mammalian cells. Proc. Natl. Acad. Sci. USA. 77:2163.
Selden, R.F., K. Burke-Howie, M.E. Rowe, H.M. Goodman, et al. 1986. Human growth hormone as a reporter gene in regulation studies employing transient gene expression. Molecular and Cellular Biololgy. 6:3173-3179.
Shigekawa, K., and W.J. Dower. 1988. Electroporation of eukaryotes and prokaryotes: A general approach to the introduction of macomolecules into cells. BioTechniques. 6:742-751.
Shillito, R. 1999. Methods of genetic transformations: Electroporation and polyethylene glycol treatment. In Molecular improvement of cereal crop. I. Vasil, editor. Kluwer, Dordrecht, The Netherlands. 9-20.
Shimkets, R.A., D.G. Lowe, J.T. Tai, P. Sehl, et al. 1999. Gene expression analysis by transcript profiling coupled to a gene database query. Nat Biotechnol. 17:798-803. java/Propub/biotech/nbt0899_798.fulltext java/Propub/biotech/nbt0899_798.abstract.
Simonsen, C.C., and A.D. Levinson. 1983. Isolation and expression of an altered mouse dihydrofolate reductase cDNA. Proc. Natl. Acad. Sci. USA. 80:2495-2499.
Southern, P.J., and P. Berg. 1982. Transformation of mammalian cells to antibiotic resistanced with a bacterial gene under control of the SV40 early region promoter. J. Mol. Appl. Gen. 1:327-341.
Sreekrishna, K., R.H. Potenz, J.A. Cruze, W.R. McCombie, et al. 1988. High level expression of heterologous proteins in methylotrophic yeast Pichia pastoris. J Basic Microbiol. 28:265-78.
Surwit, R.S., M.N. Feinglos, J. Rodin, A. Sutherland, et al. 1995. Differential effects of fat and sucrose on the development of obesity and diabetes in C57BL/6J and A/J mice. Metabolism. 44:645-51.
Thompson, J.A., and e. al. 1995. Maize transformation utilizing silicon carbide whiskers: A review. Euphytica. 85: 75-80.
Tilburn, J., C. Scazzocchio, G.G. Taylor, J.H. Zabicky-Zissman, et al. 1983. Transformation by integration in Aspergillus nidulans. Gene. 26:205-21.
Touraev, A., and e. al. 1997. Plant male germ line transformation. Plant J. 12:949-956.
Trick, H.N., and e. al. 1997. Recent advances in soybean transformation. Plant Tissue Cult. Biotechnol. 3:9-26.
Turner, D.L., E.Y. Snyder, and C.L. Cepko. 1990. Lineage-independent determinationh of cell type in the embryonic mouse retina. Neuron. 4:833-845.
West, D.B., J. Waguespack, B. York, J. Goudey-Lefevre, et al. 1994. Genetics of dietary obesity in AKR/J x SWR/J mice: segregation of the trait and identification of a linked locus on chromosome 4. Mamm Genome. 5:546-52.
Whitt, M.A., L. Buonocore, J.K. Rose, V. Ciccarone, et al. 1990. TransfectACE reagent promotes transient transfection frequencies greater than 90%. Focus. 13:8-12.
Wigler, M., A. Pellicer, S. Silversttein, and R. Axel. 1978. Biochemical transfer of single-copy eucaryotic genes using total cellular DNA as donor. Cell. 14:725.
Williams, D.A., I.R. Lemischka, D.G. Nathan, and R.C. Mulligan. 1984. Introduction of a new genetic material into pluripotent haematopoietic stem cells of the mouse. Nature. 310:476-480.
Wong, T.K., and E. Neumann. 1982. Electric field mediated gene transfer. Biochemical and Biophysical Research Communications. 107:584-587.
Yelton, M.M., J.E. Hamer, and W.E. Timberlake. 1984. Transformation of Aspergillus nidulans by using a trpC plasmid. Proc Natl Acad Sci U S A. 81:1470-4.
Yu, X.X., J.L. Barger, B.B. Boyer, M.D. Brand, et al. 2000. Impact of endotoxin on UCP homolog mRNA abundance, thermoregulation, and mitochondrial proton leak kinetics. Am J Physiol Endocrinol Metab. 279:E433-46.
Zhou, G., and e. al. 1983. Introduction of exogenous DNA into cotton embryos. Methods Enzymol. 101:433-481.

## Claims

1. An isolated polypeptide comprising at least 97% sequence identity to the sequence SEQ ID NO:6, wherein the polypeptide has thioesterase activity.

2. A polypeptide according to Claim 1, having at least 98% sequence identity to the sequence SEQ ID NO:6.

3. An isolated polynucleotide encoding a polypeptide according to either of Claims 1-2, or a complement of said polynucleotide.

**4.** An antibody that specifically binds to a polypeptide according to either of Claims 1-2.

**5.** Use of a polynucleotide encoding a polypeptide having at least 92% sequence identity to SEQ ID NO:2 or SEQ ID NO:4, or a complement of said polynucleotide, for the manufacture of a medicament for treating obesity or diabetes.

**6.** Use of a polynucleotide encoding a polypeptide according to either of Claims 1-2, or a complement of said polynucleotide, for the manufacture of a medicament for treating obesity or diabetes.

**7.** Use of a polynucleotide having at least 95% sequence identity to the sequence SEQ ID NO:1 or SEQ ID NO:3, or a complement thereof, wherein the polynucleotide encodes a polypeptide with thioesterase activity, for the manufacture of a medicament for treating obesity or diabetes.

**8.** Use of a polynucleotide having at least 95% sequence identity to the sequence SEQ ID NO:5, or a complement thereof, wherein the polynucleotide encodes a polypeptide with thioesterase activity, for the manufacture of a medicament for treating obesity or diabetes.

**9.** Use according to Claim 7, wherein said polynucleotide has at least 98% sequence identity to the sequence SEQ ID NO:1 or SEQ ID NO:3, or a complement thereof.

**10.** Use according to Claim 8, wherein said polynucleotide has at least 98% sequence identity to the sequence SEQ ID NO:5, or a complement thereof.

**11.** Use according to Claim 5, wherein the polypeptide comprises a sequence of SEQ ID NO:2 or SEQ ID NO:4.

**12.** Use according to Claim 7, wherein said polynucleotide comprises a sequence of SEQ ID NO:1 or SEQ ID NO:3.

**13.** A method of quantifying an amount of BFIT in a BAT sample, comprising contacting the sample with an antibody that specifically binds to a polypeptide according to Claim 1 or 2, or to a polypeptide consisting of an amino acid sequence of SEQ ID NO: 2 or SEQ ID NO: 4.

**14.** A method according to Claim 13, further comprising measuring the amount of said antibody bound to BFIT in said composition.

**15.** A method of measuring BFIT transcription up-regulation or down-regulation activity of a compound, comprising:

(i) contacting an isolated brown adipose cell or tissue with the compound, and
(ii) determining whether transcription of a BFIT polynucleotide encoding a polypeptide that has at least 95% sequence identity to SEQ ID NO:2, SEQ ID NO:4, or SEQ ID NO:6 and has thioesterase activity, in the cell or tissue, is increased or decreased in the presence of the compound as compared to transcription in the absence of the compound.

**16.** A method according to Claim 15, wherein the polypeptide comprises a sequence of SEQ ID NO:2, SEQ ID NO:4, or SEQ ID NO:6.

**17.** A method according to Claim 15, wherein determination of the transcription of the polynucleotide is conducted using primers and real time RTPCR.

**18.** A method according to Claim 15, wherein said thioester is an acyl-CoA thioester.

**19.** A method according to Claim 15, wherein the transcription of the polynucleotide is determined using a probe and quantitating the amount of probe bound to the polynucleotide.

**20.** A vector comprising a polynucleotide encoding a polypeptide according to either of Claims 1-2, or a complement of said polynucleotide.

**21.** A cell comprising a vector according to Claim 20.

**22.** An *in vitro* method of screening a patient for predisposition to obesity, diabetes or cachexia, comprising:

measuring expression of a BFIT polynucleotide encoding a polypeptide having at least 92% sequence identity to SEQ ID NO:2 or SEQ ID NO:4 in a sample comprising brown adipose cells (BAT) from the patient, and determining whether the expression of the BFIT polynucleotide is changed as compared to a control BAT sample wherein the change in BFIT polynucleotide expression is indicative of a predisposition to obesity, diabetes or cachexia.

**23.** An *in vitro* method of screening a patient for predisposition to obesity, cachexia or diabetes, comprising:

measuring expression of a BFIT polynucleotide encoding a polypeptide according to either of Claims 1-2 in a sample comprising brown adipose cells (BAT) from the patient, and determining whether the expression of the BFIT polynucleotide is changed as compared to a control BAT sample wherein the change in BFIT expression is indicative of a predisposition to obesity, cachexia or diabetes.

**24.** A method according to Claim 22, wherein the BFIT gene encodes a polypeptide comprising a sequence of SEQ ID NO:2 or SEQ ID NO:4.

**25.** A method according to Claim 22 or 23, wherein said measuring BFIT polynucleotide expression is measuring an amount of the polypeptide.

**26.** A method according to Claim 22 or 23, wherein said measuring BFIT polynucleotide expression is measuring an amount of mRNA encoding the polypeptide.

**27.** An *in vitro* method of screening a brown adipose tissue sample for a BFIT mutation, comprising:

comparing a BFIT gene in the sample comprising brown adipose cells with a polynucleotide comprising SEQ ID NO:1 or SEQ ID NO:3, or a complement thereof, and determining whether the sequence of the BFIT gene in the sample differs from that of SEQ ID NO:1 or SEQ ID NO:3, wherein the sample is a tissue sample from a patient having obesity, cachexia or diabetes.

**28.** An *in vitro* method of screening a brown adipose tissue sample for a BFIT mutation, comprising:

comparing a BFIT gene in the sample comprising brown adipose cells with a polynucleotide comprising SEQ ID NO:5: or a complement thereof, and determining whether the sequence of the BFIT gene in the sample differs from that of SEQ ID NO:5, wherein the sample is a tissue sample from a patient having obesity, cachexia or diabetes.

**29.** Use of a polypeptide comprising at least 95% sequence identity to an amino acid sequence of SEQ ID NO:2 or SEQ ID NO:4, wherein the polypeptide has thioesterase activity, for the manufacture of a medicament for treating obesity or diabetes.

**30.** Use according to Claim 29, wherein the polypeptide comprises a sequence of SEQ ID NO:2 or SEQ ID NO:4.

**31.** Use according to Claim 30, wherein the polypeptide consists of a sequence of SEQ ID NO:4.

**32.** Use of a polypeptide comprising at least 95% sequence identity to an amino acid sequence of SEQ ID NO:6, wherein the polypeptide has thioesterase activity, for the manufacture of a medicament for treating obesity or diabetes.

**33.** A method for monitoring treatment of obesity, diabetes or cachexia in a patient, comprising:

a) measuring expression of a brown fat inducible thioesterase (BFIT) polynucleotide encoding a BFIT polypeptide having at least 92% amino acid sequence identity with SEQ ID NO:2, SEQ ID NO:4, or SEQ ID NO:6, wherein the polypeptide has thioesterase activity, in a tissue sample comprising brown adipose cells from the patient; and
b) comparing expression of the BFIT polynucleotide to a control sample comprising brown adipose cells, wherein a change in the expression of the BFIT polynucleotide as compared to the control sample is indicative of treatment of obesity, diabetes or cachexia.

**34.** A method according to Claim 33, wherein said monitoring is detecting increased expression of the BFIT polynucleotide as compared to the control sample, wherein increased expression of BFIT polynucleotide is indicative of treatment of obesity or diabetes.

**35.** A method according to Claim 33, wherein said monitoring is detecting decreased expression of the BFIT polynucleotide as compared to the control sample, wherein decreased expression of BFIT polynucleotide is indicative of treatment of cachexia.

**36.** A method according to any of Claims 33-35, wherein the BFIT polynucleotide comprises an nucleic acid sequence of SEQ ID N0:1, SEQ ID NO:3, or SEQ ID NO:5.

**37.** A method according to any of Claims 33-35, wherein the BFIT polynucleotide comprises at least 95% sequence identity with an amino acid sequence of SEQ ID NO:2, SEQ ID NO:4, or SEQ ID NO:6.

**38.** A method according to any of Claims 33-35, wherein the BFIT polynucleotide comprises SEQ ID NO:2, SEQ ID NO: 4, or SEQ ID NO:6.

**39.** A method according to any of Claims 33-38, wherein measuring expression of a BFIT polynucleotide comprises detecting and measuring mRNA encoding a BFIT polypeptide.

**40.** A method according to any of Claims 33-38, wherein measuring expression of a BFIT polynucleotide comprises detecting and measuring BFIT polypeptide.

**41.** An *in vitro* method of screening a patient for cachexia, or diabetes, comprising:

measuring expression of a BFIT polynucleotide encoding a polypeptide having at least 92% sequence identity to SEQ ID NO:2 or SEQ ID NO:4 in a sample comprising brown adipose cells (BAT) from the patient, and determining whether the expression of the BFIT polynucleotide is changed as compared to a control BAT sample, wherein the change in BFIT polynucleotide expression is indicative of cachexia or diabetes.

**42.** An *in vitro* method of screening a patient for cachexia, or diabetes, comprising:

measuring expression of a BFIT polynucleotide encoding a polypeptide according to either of Claims 1-2 in a sample comprising brown adipose cells (BAT) from the patient, and determining whether the expression of the BFIT polynucleotide expression is changed as compared to a control BAT sample, wherein the change in BFIT polynucleotide expression is indicative of cachexia or diabetes.

**43.** A method according to Claims 41 or 42, wherein said measuring BFIT polynucleotide expression is measuring an amount of the polypeptide.

**44.** A method according to Claim 41 or 42, wherein said measuring BFIT polynucleotide expression is measuring an amount of mRNA encoding the polypeptide.

**45.** A method of detecting BFIT in a cell or tissue sample comprising brown adipose cells, comprising contacting the sample with an antibody that specifically binds to a polypeptide according to Claim 1 or 2, or to a polypeptide consisting of an amino acid sequence of SEQ ID NO: 2 or SEQ ID NO: 4.

**46.** A method according to Claim 45, further comprising measuring the amount of said antibody bound to BFIT in said sample.

## Patentansprüche

**1.** Isoliertes Polypeptid umfassend mindestens 97 % Sequenzidentität mit der Sequenz SEQ ID NO: 6, wobei das Polypeptid Thioesterase-Aktivität aufweist.

**2.** Polypeptid nach Anspruch 1, welches mindestens 98 % Sequenzidentität mit der Sequenz SEQ ID NO: 6 aufweist.

**3.** Isoliertes Polynucleotid codierend ein Polypeptid nach einem der Ansprüche 1 oder 2 oder ein Komplement des Polnucleotids.

4. Antikörper, welcher spezifisch an ein Polypeptid nach einem der Ansprüche 1 oder 2 bindet.

5. Verwendung eines Polynucleotids codierend ein Polypeptid, welches mindestens 92 % Sequenzidentität mit SEQ ID NO: 2 oder SEQ ID NO: 4 aufweist oder eines Komplements des Polynucleotids zur Herstellung eines Medikaments zur Behandlung von Fettleibigkeit oder Diabetes.

6. Verwendung eines Polynucleotids codierend ein Polypeptid nach einem der Ansprüche 1 oder 2 oder eines Komplements des Polynucleotids zur Herstellung eines Medikaments zur Behandlung von Fettleibigkeit oder Diabetes.

7. Verwendung eines Polynucleotids, welches mindestens 95 % Sequenzidentität mit der Sequenz SEQ ID NO: 1 oder SEQ ID NO: 3 aufweist oder eines Komplements davon, wobei das Polynucleotid ein Polypeptid mit Thioesterase-Aktivität codiert, zur Herstellung eines Medikaments zur Behandlung von Fettleibigkeit oder Diabetes.

8. Verwendung eines Polynucleotids, welches mindestens 95 % Sequenzidentität mit der Sequenz SEQ ID NO: 5 aufweist oder eines Komplements davon, wobei das Polynucleotid ein Polypeptid mit Thioesterase-Aktivität codiert, zur Herstellung eines Medikaments zur Behandlung von Fettleibigkeit oder Diabetes.

9. Verwendung nach Anspruch 7, wobei das Polynucleotid mindestens 98 % Sequenzidentität mit der Sequenz des SEQ ID NO: 1 oder SEQ ID NO: 3 aufweist oder einem Komplement davon.

10. Verwendung nach Anspruch 8, wobei das Polynucleotid mindestens 98 % Sequenzidentität mit der Sequenz SEQ ID NO: 5 aufweist oder einem Komplement davon.

11. Verwendung nach Anspruch 5, wobei das Polypeptid eine Sequenz aus SEQ ID NO: 2 oder SEQ ID NO: 4 umfasst.

12. Verwendung nach Anspruch 7, wobei das Polynucleotid eine Sequenz aus SEQ ID NO: 1 oder SEQ ID NO: 3 umfasst.

13. Verfahren zur Quanitfizierung einer Menge von BFIT in einer BAT-Probe umfassend Kontaktieren der Probe mit einem Antikörper, welcher spezifisch an ein Polypeptid nach Anspruch 1 oder 2 bindet oder an ein Polypeptid bestehend aus einer Aminosäuresequenz aus SEQ ID NO: 2 oder SEQ ID NO: 4.

14. Verfahren nach Anspruch 13, weiterhin umfassend die Messung der Menge an Antikörper, welche an BFIT in der Zusammensetzung gebunden ist.

15. Verfahren zur Messung von BFIT Transkription Upregulations- oder Downregulations-Aktivität einer Verbindung, umfassend:

(i) Kontaktieren einer isolierten braunen Fettzelle oder Gewebe mit der Verbindung, und
(ii) Bestimmen, ob Transkription eines BFIT Polynucleotids codierend ein Polypeptid, welches mindestens 95 % Sequenzidentität mit SEQ ID NO: 2, SEQ ID NO: 4 oder SEQ ID NO: 6 aufweist und eine Thioesterase-Aktivität in der Zelle oder dem Gewebe aufweist, zunimmt oder abnimmt in Gegenwart der Verbindung, verglichen mit der Transkription in Abwesenheit der Verbindung.

16. Verfahren nach Anspruch 15, wobei das Polypeptid eine Sequenz aus SEQ ID NO: 2, SEQ ID NO: 4 oder SEQ ID NO: 6 umfasst.

17. Verfahren nach Anspruch 15, wobei die Bestimmung der Transkription des Polynucleotids durch Verwendung von Primern und Real time RT-PCR durchgeführt wird.

18. Verfahren nach Anspruch 15, wobei der Thioester ein Acyl-CoA-Thioster ist.

19. Verfahren nach Anspruch 15, wobei die Transkription des Polynucleotids durch Verwendung einer Sonde und Quantifizierung der Menge der an das Polypeptid gebundenen Sonde bestimmt wird.

20. Vector umfassend ein Polynucleotid, welches ein Polypeptid nach einem der Ansprüche 1 oder 2 codiert oder ein Komplement des Polynucleotids.

21. Zelle umfassend einen Vector nach Anspruch 20.

**22.** *In vitro*-Verfahren zum Testen eines Patienten auf Prädisposition zu Fettleibigkeit, Kachexie oder Diabetes, umfassend:

Messen der Expression eines BFIT-Polynucleotids codierend ein Polypeptid, welches mindestens 92 % Sequenzidentität mit SEQ ID NO: 2 oder SEQ ID NO:4 in einer Probe aufweist, welche braune Fettzellen (brown adipose cells, BAT) von dem Patienten umfasst, und Bestimmen, ob die Expression des BFIT Polynucleotids verändert wird im Vergleich mit einer Kontrollprobe brauner Fettzellen, wobei die Veränderung in der Polynucleotid-Expression indikativ für eine Prädisposition zu Fettleibigkeit, Diabetes oder Kachexie ist.

**23.** In vitro-Verfahren zum Testen eines Patienten auf Predispisition zu Fettleibigkeit, Diabetes oder Kachexie, umfassend:

Messen der Expression eines BFIT-Polynucleotids codierend ein Polypeptid nach einem der Ansprüche 1 oder 2 in einer Probe, welche braune Fettzellen von dem Patienten umfasst, und Bestimmen, ob die Expression des BFIT-Polynucleotids verändert wird im Vergleich mit einer Kontrollprobe brauner Fettzellen, wobei die Veränderung in der Polynucleotid-Expression indikativ für eine Prädisposition zu Fettleibigkeit, Kachexie oder Diabetes ist.

**24.** Verfahren nach Anspruch 22, wobei das BFIT-Gen ein Polypeptid codiert, welches eine Sequenz aus SEQ ID NO: 2 oder SEQ ID NO: 4 umfasst.

**25.** Verfahren nach Anspruch 22 oder 23, wobei das Messen der BFIT-Expression ein Messen einer Menge des Polypeptids ist.

**26.** Verfahren nach Anspruch 22 oder 23, wobei das Messen der BFIT-Expression ein Messen einer Menge der das Polypeptid codierenden mRNA ist.

**27.** *In Vitro*-Verfahren zum Testen einer braunen Fettgewebe-Probe auf eine BFIT-Mutation umfassend:

Vergleichen eines BFIT-Gens in der braune Fettzellen umfassenden Probe mit einem Polynucleotid umfassend SEQ ID NO: 1 oder SEQ ID NO: 3 oder einem Komplement davon, und Bestimmen, ob sich die Sequenz des BFIT-Gens von derjenigen von SEQ ID NO: 1 oder SEQ-ID NO: 3 unterscheidet, wobei die Probe eine Gewebeprobe eines Fettleibigkeit, Kachexie oder Diabetes aufweisenden Patienten ist.

**28.** *In Vitro*-Verfahren zum Testen einer braunen Fettgewebe-Probe auf eine BFIT-Mutation umfassend:

Vergleichen eines BFIT-Gens in der braune Fettzellen umfassenden Probe mit einem Polynucleotid umfassend SEQ ID NO: 5 oder einem Komplement davon, und Bestimmen, ob die Sequenz des BFIT-Gens in der Probe sich von derjenigen von SEQ ID NO: 5 unterscheidet, wobei die Probe eine Gewebeprobe eines Fettleibigkeit, Kachexie oder Diabetes aufweisenden Patienten ist.

**29.** Verwendung eines Polypeptids umfassend mindestens 95 % Sequenzidentität mit einer Aminosäuresequenz von SEQ ID NO: 2 oder SEQ ID NO: 4, wobei das Polypeptid Thioesterase-Aktivität aufweist, zur Herstellung eines Medikaments zur Behandlung von Fettleibigkeit oder Diabetes.

**30.** Verwendung nach Anspruch 29, wobei das Polypeptid eine Sequenz von SEQ ID NO: 2 oder SEQ ID NO: 4 umfasst.

**31.** Verwendung nach Anspruch 30, wobei das Polypeptid aus einer Sequenz von SEQ ID NO: 4 besteht.

**32.** Verwendung eines Polypeptids umfassend mindestens 95 % Sequenzidentität mit einer Aminosäuresequenz von SEQ ID NO: 6, wobei das Polypeptid Thioesterase-Aktivität aufweist, zur Herstellung eines Medikaments zur Behandlung von Fettleibigkeit oder Diabetes.

**33.** Verfahren zur Überwachung der Behandlung von Fettleibigkeit, Diabetes oder Kachexie eines Patienten, umfassend:

a) Messen der Expression eines brown fat inducible thioesterase (BFIT) Polynucleotids codierend ein BFIT-Polypeptid, welches mindestens 92 % Aminosäure-Sequenzidentität mit SEQ ID NO: 2, SEQ ID NO: 4 oder SEQ ID NO: 6 aufweist, wobei das Polypeptid Thioesterase-Aktivität aufweist, in einer Gewebeprobe umfassend braune Fettzellen von dem Patienten; und
b) Vergleichen der Expression des BFIT Polynucleotids mit einer Kontrollprobe umfassend braune Fettzellen, wobei eine Veränderung in der Expression des BFIT-Polynucleotids verglichen mit der Kontrollprobe indikativ für eine Behandlung von Fettleibigkeit, Diabetes oder Kachexie ist.

**34.** Verfahren nach Anspruch 33, wobei die Überwachung das Detektieren einer zunehmenden Expression des BFIT-Polynucleotids im Vergleich zur Kontrollprobe ist, wobei zunehmende Expression von BFIT-Polynucleotid indikativ für eine Behandlung von Fettleibigkeit oder Diabetes ist.

**35.** Verfahren nach Anspruch 33, wobei eine Überwachung das Detektieren einer abnehmenden Expression des BFIT-Polynucleotids im Vergleich zur Kontrollprobe ist, wobei abnehmende Expression von BFIT-Polynucleotid indikativ für eine Behandlung von Kachexie ist.

**36.** Verfahren nach einem der Ansprüche 33 - 35, wobei das BFIT-Polynucleotid eine Nucleinsäuresequenz aus SEQ ID NO: 1, SEQ ID NO: 3 oder SEQ ID NO: 5 umfasst.

**37.** Verfahren nach einem der Ansprüche 33 - 35, wobei das BFIT-Polynucleotid mindestens 95 % Sequenzidentät mit einer Aminosäuresequenz aus SEQ ID NO: 2, SEQ ID NO: 4 oder SEQ ID NO: 6 umfasst.

**38.** Verfahren nach einem der Ansprüche 33 - 35, wobei das BFIT-Polynucleotid SEQ ID NO: 2, SEQ ID NO: 4 oder SEQ ID NO: 6 umfasst.

**39.** Verfahren nach einem der Ansprüche 33 - 38, wobei das Messen der Expression eines BFIT-Polynucleotids das Detektieren und Messen von BFIT-Polypeptid codierender mRNA umfasst.

**40.** Verfahren nach einem der Ansprüche 33 - 38, wobei das Messen der Expression eines BFIT-Polynucleotids das Detektieren und Messen von BFIT-Polypeptid umfasst.

**41.** *In Vitro*-Verfahren zum Testen eines Patienten auf Kachexie oder Diabetes umfassend:

Messen der Expression eines BFIT-Polynucleotids codierend ein Polypeptid, welches mindestens 92 % Sequenzidentität mit SEQ ID NO: 2 oder SEQ ID NO: 4 in einer Probe aufweist, welche braune Fettzellen (brown adipose cells, BAT) von dem Patienten umfasst,
und Bestimmen, ob die Expression des BFIT-Polynucleotids sich im Vergleich zu einer Kontroll-BAT-Probe verändert, wobei die Veränderung in der BFIT-Polynucleotid Expression indikativ für Kachexie oder Diabetes ist.

**42.** In-Vitro-Verfahren zum Testen eines Patienten auf Kachexie oder Diabetes umfassend:

Messen der Expression eines BFIT-Polynucleotids codierend ein Polypeptid nach einem der Ansprüche 1 - 2 in einer Probe umfassend braune Fettzellen (brown adipose cells, BAT) von dem Patienten, und
Bestimmen, ob die Expression der BFIT Polynucleotid Expression sich im Vergleich zu einer Kontroll-BAT-Probe verändert, wobei die Änderung in der BFIT Polynucleotid Expression indikativ für Kachexie oder Diabetes ist.

**43.** Verfahren nach Anspruch 41 oder 42, wobei das Messen der BFIT-Polynucleotid Expression das Messen einer Menge von Polypeptid ist.

**44.** Verfahren nach Anspruch 41 oder 42, wobei das Messen der BFIT-Polynucleotid Expression das Messen einer Menge der das Polypeptid kodierenden mRNA ist.

**45.** Verfahren zur Detektion von BFIT in einer Zell- oder Gewebeprobe umfassend braune Fettzellen, umfassend Kontaktieren der Probe mit einem Antikörper, welcher spezifisch an ein Polypeptid nach Anspruch 1 oder 2 bindet oder an ein Polypeptid bestehend aus einer Aminosäuresequenz aus SEQ ID NO: 2 oder SEQ ID NO: 4.

**46.** Verfahren nach Anspruch 45, weiterhin umfassend das Messen der Menge des an BFIT gebundenen Antikörpers in der Probe.

**Revendications**

1. Polypeptide isolé comprenant une identité de séquence d'au moins 97 % avec la séquence SEQ ID NO : 6, où le polypeptide a une activité estérase.

2. Polypeptide selon la revendication 1, ayant une identité de séquence d'au moins 98 % avec la séquence SEQ ID NO : 6.

3. Polynucléotide isolé codant pour un polypeptide selon l'une ou l'autre des revendications 1 et 2, ou un complémentaire dudit polynucléotide.

4. Anticorps qui se lie spécifiquement à un polypeptide selon l'une ou l'autre des revendications 1 et 2.

5. Utilisation d'un polynucléotide codant pour un polypeptide ayant une identité de séquence d'au moins 92 % avec SEQ ID NO : 2 ou SEQ ID NO : 4, ou d'un complémentaire dudit polynucléotide, pour la fabrication d'un médicament destiné au traitement de l'obésité ou du diabète.

6. Utilisation d'un polynucléotide codant pour un polypeptide selon l'une ou l'autre des revendications 1 et 2, ou d'un complémentaire dudit polynucléotide, pour la fabrication d'un médicament destiné au traitement de l'obésité ou du diabète.

7. Utilisation d'un polynucléotide ayant une identité de séquence d'au moins 95 % avec la séquence SEQ ID NO : 1 ou SEQ ID NO : 3, ou d'un complémentaire de celui-ci, où le polynucléotide code pour un polypeptide avec une activité thioestérase, pour la fabrication d'un médicament destiné au traitement de l'obésité ou du diabète.

8. Utilisation d'un polynucléotide ayant une identité de séquence d'au moins 95 % avec la séquence SEQ ID NO : 5, ou d'un complémentaire de celui-ci, où le polynucléotide code pour un polypeptide avec une activité thioestérase, pour la fabrication d'un médicament destiné au traitement de l'obésité ou du diabète.

9. Utilisation selon la revendication 7, où ledit polynucléotide a une identité de séquence d'au moins 98 % avec la séquence SEQ ID NO : 1 ou SEQ ID NO : 3, ou d'un complémentaire de celui-ci.

10. Utilisation selon la revendication 8, où ledit polynucléotide a une identité de séquence d'au moins 98 % avec la séquence SEQ ID NO : 5, ou d'un complémentaire de celui-ci.

11. Utilisation selon la revendication 5, où le polypeptide comprend une séquence de SEQ ID NO : 2 ou SEQ ID NO : 4.

12. Utilisation selon la revendication 7, où le polynucléotide comprend une séquence de SEQ ID NO : 1 ou SEQ ID NO : 3.

13. Procédé de quantification d'une quantité de BFIT dans un échantillon de TAB, comprenant la mise en contact de l'échantillon avec un anticorps qui se lie spécifiquement à un polypeptide selon la revendication 1 ou 2, ou à un polypeptide constitué d'une séquence d'acides aminés de SEQ ID NO : 2 ou SEQ ID NO : 4.

14. Procédé selon la revendication 13, comprenant en outre la mesure de la quantité dudit anticorps lié à la BFIT dans ladite composition.

15. Procédé de mesure de l'activité de régulation à la hausse ou de régulation à la baisse de la transcription de la BFIT d'un composé, comprenant :

    (i) la mise en contact d'une cellule ou d'un tissu adipeux brun isolé avec le composé, et
    (ii) la détermination de si la transcription d'un polynucléotide de BFIT codant pour un polypeptide qui a une identité de séquence d'au moins 95 % avec SEQ ID NO : 2, SEQ ID NO : 4 ou SEQ ID N0 : 6 et a une activité thioestérase, dans la cellule ou le tissu, est augmentée ou diminuée en présence du composé par comparaison à la transcription en l'absence du composé.

16. Procédé selon la revendication 15, où le polypeptide comprend une séquence de SEQ ID NO : 2, SEQ ID NO : 4 ou SEQ ID NO : 6.

**17.** Procédé selon la revendication 15, où la détermination de la transcription du polynucléotide est conduite en utilisant des amorces et une RT-PCR en temps réel.

**18.** Procédé selon la revendication 15, où ledit thioester est un acyl-CoA thioester.

**19.** Procédé selon la revendication 15, où la transcription du polynucléotide est déterminée en utilisant une sonde et en quantifiant la quantité de sonde liée au polynucléotide.

**20.** Vecteur comprenant un polynucléotide codant pour un polypeptide selon l'une ou l'autre des revendications 1 et 2, ou un complémentaire dudit polynucléotide.

**21.** Cellule comprenant un vecteur selon la revendication 20.

**22.** Procédé *in vitro* de dépistage d'un patient pour une prédisposition à l'obésité, au diabète ou à la cachexie, comprenant :

la mesure de l'expression d'un polynucléotide de BFIT codant pour un polypeptide ayant une identité de séquence d'au moins 92 % avec SEQ ID NO : 2 ou SEQ ID NO : 4 dans un échantillon comprenant des cellules adipeuses brunes (TAB) provenant du patient, et la détermination de si l'expression du polynucléotide de BFIT est changée par comparaison à un échantillon de TAB contrôle où le changement de l'expression du polynucléotide de BFIT est indicatif d'une prédisposition à l'obésité, au diabète ou à la cachexie.

**23.** Procédé *in vitro* de dépistage d'un patient pour une prédisposition à l'obésité, à la cachexie ou au diabète, comprenant :

la mesure de l'expression d'un polynucléotide de BFIT codant pour un polypeptide selon l'une ou l'autre des revendications 1 et 2 dans un échantillon comprenant des cellules adipeuses brunes (TAB) provenant du patient, et la détermination de si l'expression du polynucléotide de BFIT est changée par comparaison à un échantillon de TAB contrôle où le changement de l'expression de BFIT est indicatif d'une prédisposition à l'obésité, à la cachexie ou au diabète.

**24.** Procédé selon la revendication 22, où le gène de BFIT code pour un polypeptide comprenant une séquence de SEQ ID NO : 2 ou SEQ ID NO : 4.

**25.** Procédé selon la revendication 22 ou 23, où ladite mesure de l'expression du polynucléotide de BFIT mesure une quantité du polypeptide.

**26.** Procédé selon la revendication 22 ou 23, où ladite mesure de l'expression du polynucléotide de BFIT mesure une quantité d'ARNm codant pour le polypeptide.

**27.** Procédé *in vitro* de criblage d'un échantillon de tissu adipeux brun pour une mutation de la BFIT, comprenant :

la comparaison d'un gène de BFIT dans l'échantillon comprenant des cellules adipeuses brunes avec un polynucléotide comprenant SEQ ID NO : 1 ou SEQ ID NO : 3, ou un complémentaire de celui-ci, et la détermination de si la séquence du gène de BFIT dans l'échantillon diffère de celle de SEQ ID NO : 1 ou SEQ ID NO : 3, où l'échantillon est un échantillon tissulaire provenant d'un patient souffrant d'obésité, de cachexie ou de diabète.

**28.** Procédé *in vitro* de criblage d'un échantillon de tissu adipeux brun pour une mutation de la BFIT, comprenant:

la comparaison d'un gène de BFIT dans l'échantillon comprenant des cellules adipeuses brunes avec un polynucléotide comprenant SEQ ID NO : 5, ou un complémentaire de celui-ci, et la détermination de si la séquence du gène de BFIT dans l'échantillon diffère de celle de SEQ ID NO : 5, où l'échantillon est un échantillon tissulaire provenant d'un patient souffrant d'obésité, de cachexie ou de diabète.

**29.** Utilisation d'un polypeptide comprenant une identité de séquence d'au moins 95 % avec une séquence d'acides aminés de SEQ ID NO : 2 ou SEQ ID NO : 4, où le polypeptide a une activité thioestérase, pour la fabrication d'un médicament destiné au traitement de l'obésité ou du diabète.

**30.** Utilisation selon la revendication 29, où le polypeptide comprend une séquence de SEQ ID NO : 2 ou SEQ ID NO : 4.

**31.** Utilisation selon la revendication 30, où le polypeptide est constitué d'une séquence de SEQ ID NO : 4.

**32.** Utilisation d'un polypeptide comprenant une identité de séquence d'au moins 95 % avec une séquence d'acides aminés de SEQ ID NO : 6, où le polypeptide a une activité thioestérase, pour la fabrication d'un médicament destiné au traitement de l'obésité ou du diabète.

**33.** Procédé de contrôle d'un traitement de l'obésité, du diabète ou de la cachexie chez un patient, comprenant :

a) la mesure de l'expression d'un polynucléotide de thioestérase inductible par le tissu adipeux brun (BFIT) codant pour un polypeptide de BFIT ayant une identité de séquence d'acides aminés d'au moins 92 % avec SEQ ID NO : 2, SEQ ID NO : 4 ou SEQ ID NO : 6, où le polypeptide a une activité thioestérase, dans un échantillon tissulaire comprenant des cellules adipeuses brunes provenant du patient ; et
b) la comparaison de l'expression du polynucléotide de BFIT avec un échantillon contrôle comprenant des cellules adipeuses brunes, où un changement de l'expression du polynucléotide de BFIT par comparaison à l'échantillon contrôle est indicatif d'un traitement de l'obésité, du diabète ou de la cachexie.

**34.** Procédé selon la revendication 33, où ledit contrôle détecte une augmentation de l'expression du polynucléotide de BFIT par comparaison avec l'échantillon contrôle, où une augmentation de l'expression du polynucléotide de BFIT est indicative d'un traitement de l'obésité ou du diabète.

**35.** Procédé selon la revendication 33, où ledit contrôle détecte une diminution de l'expression du polynucléotide de BFIT par comparaison avec l'échantillon contrôle, où une diminution de l'expression du polynucléotide de BFIT est indicative d'un traitement de la cachexie.

**36.** Procédé selon l'une quelconque des revendications 33 à 35, où le polynucléotide de BFIT comprend une séquence d'acide nucléique de SEQ ID NO : 1, SEQ ID NO : 3 ou SEQ ID NO : 5.

**37.** Procédé selon l'une quelconque des revendications 33 à 35, où le polynucléotide de BFIT comprend une identité de séquence d'au moins 95 % avec une séquence d'acides aminés de SEQ ID NO : 2, SEQ ID NO : 4 ou SEQ ID NO : 6.

**38.** Procédé selon l'une quelconque des revendications 33 à 35, où le polynucléotide de BFIT comprend SEQ ID NO : 2, SEQ ID NO : 4 ou SEQ ID NO : 6.

**39.** Procédé selon l'une quelconque des revendications 33 à 38, où la mesure de l'expression d'un polynucléotide de BFIT comprend la détection et la mesure de l'ARNm codant pour un polypeptide de BFIT.

**40.** Procédé selon l'une quelconque des revendications 33 à 38, où la mesure de l'expression d'un polynucléotide de BFIT comprend la détection et la mesure du polypeptide de BFIT.

**41.** Procédé *in vitro* de dépistage d'un patient pour la cachexie ou le diabète, comprenant :

la mesure de l'expression d'un polynucléotide de BFIT codant pour un polypeptide ayant une identité de séquence d'au moins 92 % avec SEQ ID NO : 2, SEQ ID NO : 4 dans un échantillon comprenant des cellules adipeuses brunes (TAB) provenant du patient, et la détermination de si l'expression du polynucléotide de BFIT est changée par comparaison avec un échantillon de TAB contrôle, où le changement de l'expression du polynucléotide de BFIT est indicatif de cachexie ou de diabète.

**42.** Procédé *in vitro* de dépistage d'un patient pour la cachexie ou le diabète, comprenant :

la mesure de l'expression d'un polynucléotide de BFIT codant pour un polypeptide selon l'une ou l'autre des revendications 1 et 2 dans un échantillon comprenant des cellules adipeuses brunes (TAB) provenant du patient, et la détermination de si l'expression du polynucléotide de BFIT est changée par comparaison avec un échantillon de TAB contrôle, où le changement de l'expression du polynucléotide de BFIT est indicatif de cachexie ou de diabète.

**43.** Procédé selon la revendication 41 ou 42, où ladite mesure de l'expression du polynucléotide de BFIT mesure une quantité du polypeptide.

**44.** Procédé selon la revendication 41 ou 42, où ladite mesure de l'expression du polynucléotide de BFIT mesure une quantité d'ARNm codant pour le polypeptide.

**45.** Procédé de détection de BFIT dans un échantillon cellulaire ou tissulaire comprenant des cellules adipeuses brunes, comprenant la mise en contact de l'échantillon avec un anticorps qui se lie spécifiquement à un polypeptide selon la revendication 1 ou 2, ou à un polypeptide constitué d'une séquence d'acides aminés de SEQ ID NO : 2 ou SEQ ID NO : 4.

**46.** Procédé selon la revendication 45, comprenant en outre la mesure de la quantité dudit anticorps lié à la BFIT dans ledit échantillon.

Figure 1

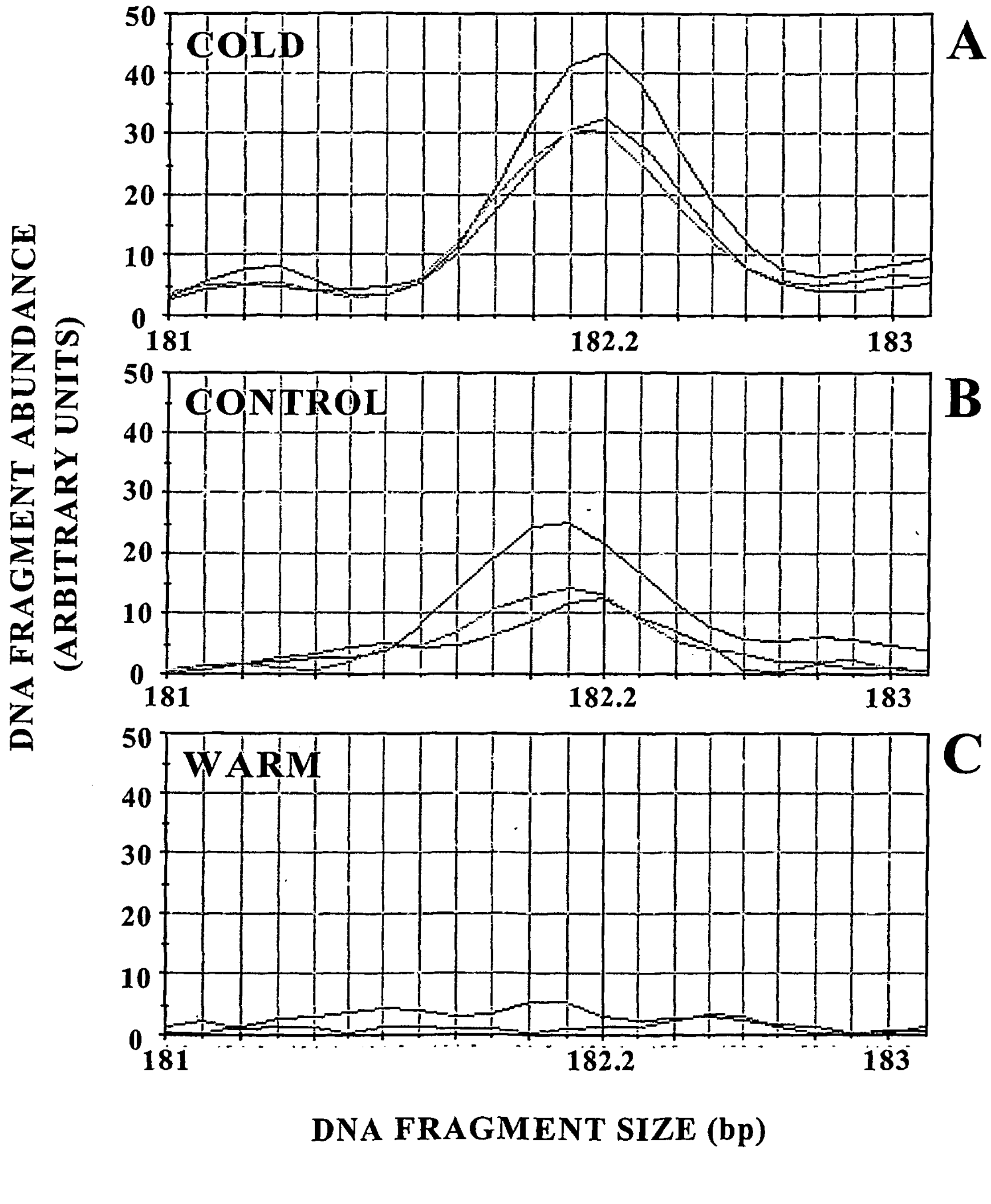
COLD
A
CONTROL
B
WARM
C
DNA FRAGMENT ABUNDANCE (ARBITRARY UNITS)
0
10
20
30
40
50
181
182.2
183
DNA FRAGMENT SIZE (bp)

# Figure 2

```
hBFIT1    1  MIQNVGNHLRRGLASVFSNRTSRK--SALRAGNDSAMADGEGYRNPTEVQ
hBFIT2    1  MIQNVGNHLRRGLASVFSNRTSRK--SALRAGNDSAMADGEGYRNPTEVQ
mBFIT2    1  MIQNVGNHLRRGFASMFSNRTSRKSISHPESGDPPTMAEGEGYRNPTEVQ

hBFIT1   49  MSQLVLPCHTNQRGELSVGQLLKWIDTTACLSAERHAGCPCVTASMDDIY
hBFIT2   49  MSQLVLPCHTNQRGELSVGQLLKWIDTTACLSAERHAGCPCVTASMDDIY
mBFIT2   51  MSQLVLPCHTNHRGELSIGQLLKWIDTTACLSAERHAGCPCVTASMDDIY

hBFIT1   99  FEHTISVGQVVNIKAKVNRAFNSSMEVGIQVASEDLCSEKQWNVCKALAT
hBFIT2   99  FEHTISVGQVVNIKAKVNRAFNSSMEVGIQVASEDLCSEKQWNVCKALAT
mBFIT2  101  FDHTISVGQVVNIKAKVNRAFNSSMEVGIQVVSEDLCSEKQWSVCKALAT

hBFIT1  149  FVARREITKVKLKQITPRTEEEKMEHSVAAERRRMRLVYADTIKDLLANC
hBFIT2  149  FVARREITKVKLKQITPRTEEEKMEHSVAAERRRMRLVYADTIKDLLANC
mBFIT2  151  FVAHRELSKVKLKQVIPLTEEEKTEHGVAAERRRMRLVYADTIKDLLTHC

hBFIT1  199  AIQGDLESRDCSRMVPAEKTRVESVELVLPPHANHQGNTFGGQIMAWMEN
hBFIT2  199  AIQGDLESRDCSRMVPAEKTRVESVELVLPPHANHQGNTFGGQIMAWMEN
mBFIT2  201  VIQDDLD-KDCSNMVPAEKTRVESVELVLPPHANHQGNTFGGQIMAWMEN

hBFIT1  249  VATIAASRLCRAHPTLKAIEMFHFRGPSQVGDRLVLKAIVNNAFKHSMEV
hBFIT2  249  VATIAASRLCRAHPTLKAIEMFHFRGPSQVGDRLVLKAIVNNAFKHSMEV
mBFIT2  250  VATIAASRLCHAHPTLKAIEMFHFRGPSQVGDRLVLKAIVNNAFKHSMEV

hBFIT1  299  GVCVEAYRQEAETHRRHINSAFMTFVVLDADDQPQLLPWIRPQPGDGERR
hBFIT2  299  GVCVEAYRQEAETHRRHINSAFMTFVVLDADDQPQLLPWIRPQPGDGERR
mBFIT2  300  GVCVKAYRQEAETQRRHINSAFMTFVVLDKDDQPQKLPWIRPQPGEGERR

hBFIT1  349  YREASARKKIRLDRKYIVSCKQTEVPLSVPWDPSNQVYLSYNNVSSLKML
hBFIT2  349  YREASARKKIRLDRKYIVSCKQTEVPLSVPWDPSNQVYLSYNNVSSLKML
mBFIT2  350  YREASARKKIRLDRKYLVSCKQAEVALSVPWDPSNQVYLSYYNVSSLKTL

hBFIT1  399  VAKDNWVLSSEISQVRLYTLEDDKFLSFHMEMVVHVDAAQAFLLLSDLRQ
hBFIT2  399  VAKDNWVLSSEISQVRLYTLEDDKFLSFHMEMVVHVDAAQAFLLLSDLRQ
mBFIT2  400  MAKDNWVLSVEISEVRLYILEED-FLSFHLEMVVNVDAAQVFQLLSDLRR

hBFIT1  449  RPEWDKHYRSVELVQQVDEDDAIYHVTSPALGGHTKPQDFVILASRRKPC
hBFIT2  449  RPEWDKHYRSVELVQQVDEDDAIYHVTSPALGGHTKPQDFVILASRRKPC
mBFIT2  449  RPEWDKHYRSVELVQQVDEDDAIYHVISPALSGNTKPQDFVILASRRKPC

hBFIT1  499  DNGDPYVIALRSVTLPTHRETPEYRRGETLCSGFCLWREGDQLTKCCWVR
hBFIT2  499  DNGDPYVIALRSVTLPTHRETPEYRRGETLCSGFCLWREGDQLTKVSYYN
mBFIT2  499  DNGDPYVIALRSVTLPTHHETPEYQRGETLCSGFCLWREGDQMTKVSYYN

hBFIT1  549  VSLTELVSASGFYSWGLESRSKGRRSDGWNGKLAGGHLSTLKAIPVAKIN
hBFIT2  549  QATPGVLNYVTTNVAGLSSEFYTTFKACEQFLLDNRNDLAPSLQTL----
mBFIT2  549  QATPGFLNYVTTNVSGLSSEFYNTFKACESFLLDNRNDLAPSLQTL----

hBFIT1  599  SRFGYLQDT
```

Figure 3

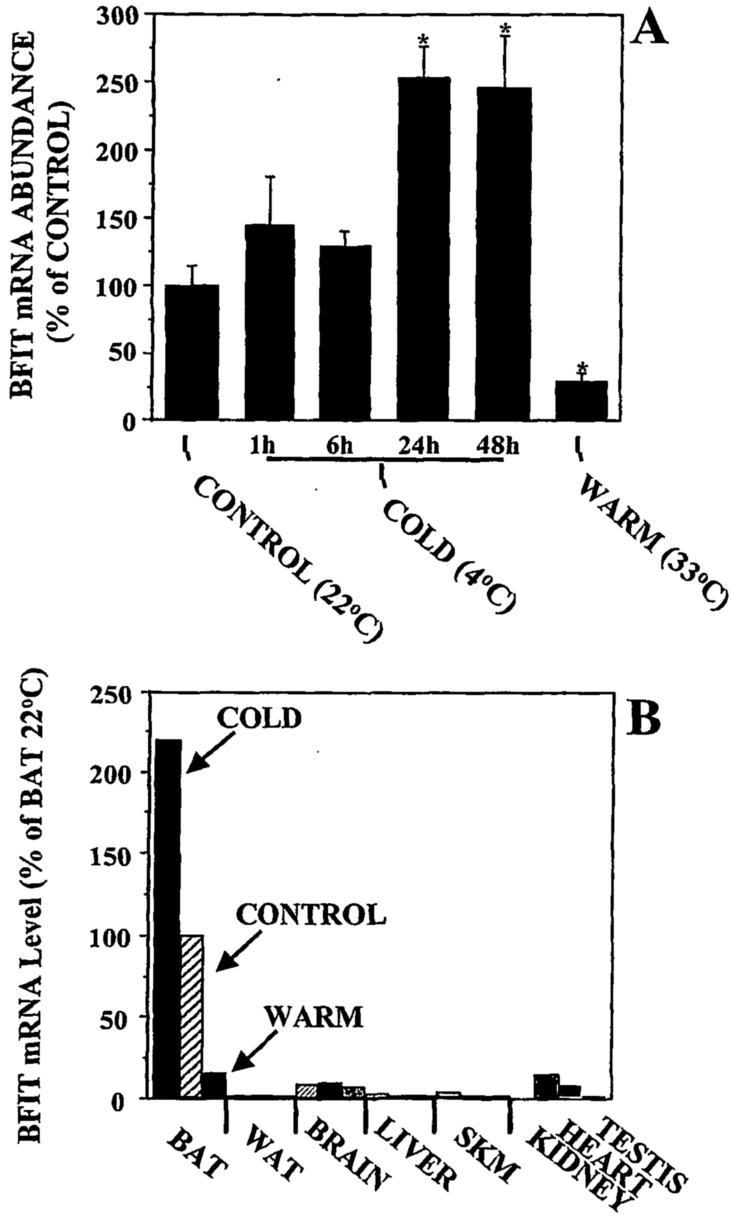
A
BFIT mRNA ABUNDANCE (% of CONTROL)
300
250
200
150
100
50
0
1h
6h
24h
48h
CONTROL (22°C)
COLD (4°C)
WARM (33°C)
B
BFIT mRNA Level (% of BAT 22°C)
250
200
150
100
50
0
COLD
CONTROL
WARM
BAT
WAT
BRAIN
LIVER
SKM
KIDNEY
HEART
TESTIS

Figure 4

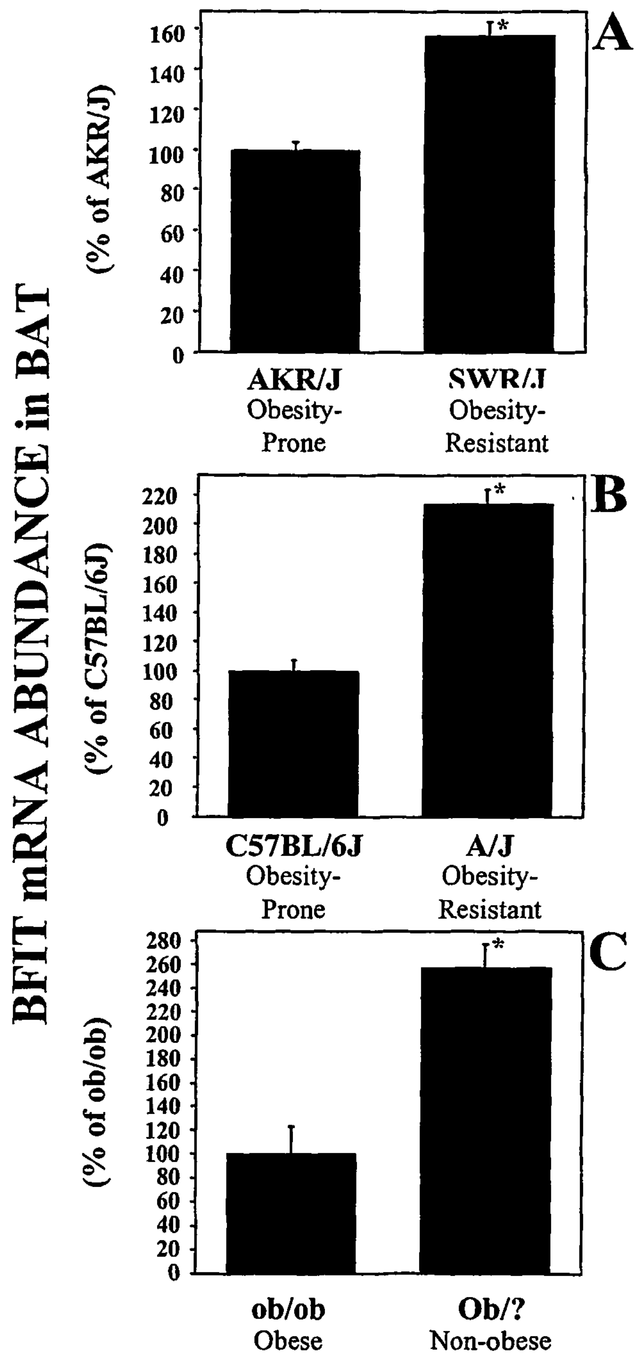
BFIT mRNA ABUNDANCE in BAT
A
(% of AKR/J)
160
140
120
100
80
60
40
20
0
*
AKR/J
Obesity-
Prone
SWR/J
Obesity-
Resistant
B
(% of C57BL/6J)
220
200
180
160
140
120
100
80
60
40
20
0
*
C57BL/6J
Obesity-
Prone
A/J
Obesity-
Resistant
C
(% of ob/ob)
280
260
240
220
200
180
160
140
120
100
80
60
40
20
0
*
ob/ob
Obese
Ob/?
Non-obese

Figure 5

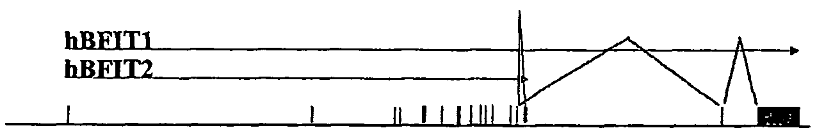

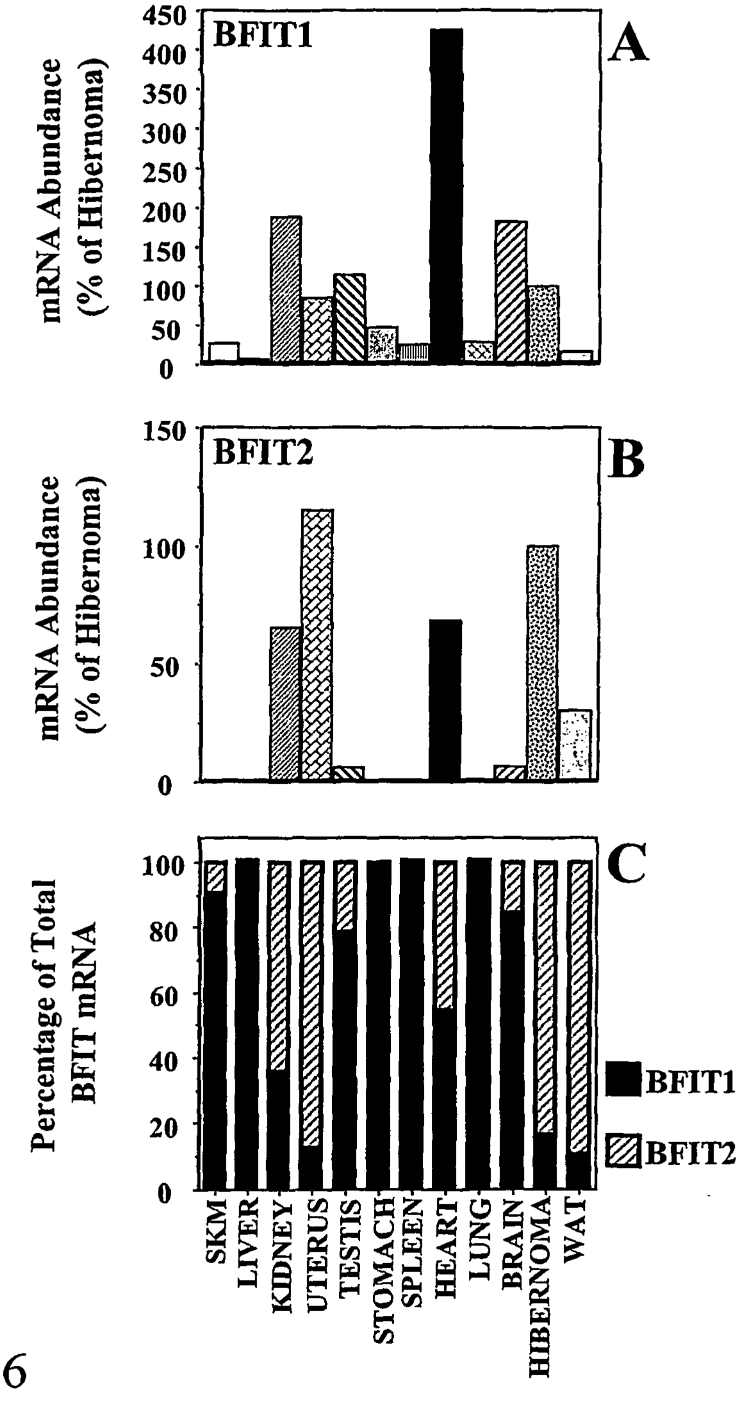

mRNA Abundance
(% of Hibernoma)
450
400
350
300
250
200
150
100
50
0
BFIT1
A
150
100
50
0
BFIT2
B
Percentage of Total
BFIT mRNA
100
80
60
40
20
0
C
BFIT1
BFIT2
SKM
LIVER
KIDNEY
UTERUS
TESTIS
STOMACH
SPLEEN
HEART
LUNG
BRAIN
HIBERNOMA
WAT


Figure 6

Figure 7

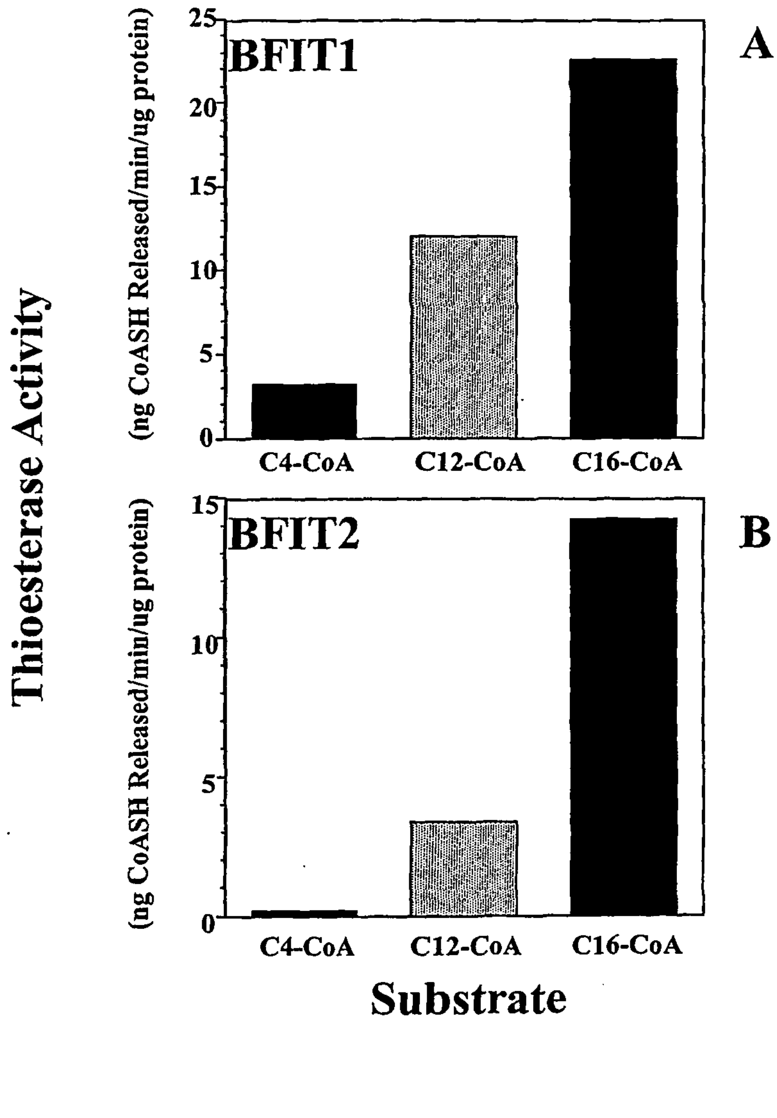
Thioesterase Activity
(ng CoASH Released/min/ug protein)
25
20
15
10
5
0
BFIT1
A
C4-CoA
C12-CoA
C16-CoA
(ng CoASH Released/min/ug protein)
15
10
5
0
BFIT2
B
C4-CoA
C12-CoA
C16-CoA
Substrate

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4816567 A
- US 5545807 A
- US 5545806 A
- US 5569825 A
- US 5625126 A
- US 5633425 A
- US 5661016 A
- WO 9308829 A
- WO 9627011 A
- US 4676980 A
- WO 9100360 A
- WO 92200373 A
- EP 03089 A
- WO 9411026 A
- US 4485045 A
- US 4544545 A
- US 5013556 A
- WO 9106629 A
- WO 9010048 A
- WO 9013641 A
- WO 9104753 A
- WO 9010448 A
- WO 9733551 A
- EP 307247 A
- EP 402226 A
- WO 9100357 A
- US 0201783 W
- US 60263362 B


### Non-patent literature cited in the description


- **OKANO.** Oligodeoxynucleotides as Antisense Inhibitors of Gene Expression. CRC Press, 1988
- **ALAM, J. ; J.L. COOK.** Reporter genes: Application to the study of mammalian gene transcription. *Anal. Biochem.,* 1990, vol. 188, 245-254
- **ALTSCHUL, S.F. ; W. GISH.** Local alignment statistics. *Methods Enzymol.,* 1996, vol. 266, 460-80
- **ALTSCHUL, S.F. ; T.L. MADDEN ; A.A. SCHAFFER ; J. ZHANG et al.** Gapped BLAST and PSI-BLAST: a new generation of protein database search programs. *Nucleic Acids Res.,* 1997, vol. 25, 3389-402
- **AUSTIN, C.P. ; C.L. CEPKO.** Cellular migration patterns in the developing mouse cerebral cortex. *Development,* 1990, vol. 110, 713-732
- **BECHTOLD, N. ; G. PELLETIER.** In planta Agrobacterium-mediated transformation of adult Arabidopsis thaliana plants by vacuum infiltration. *Methods Mol Biol.,* 1998, vol. 82, 259-66
- **BECKER, D.M. ; L. GUARENTE.** High-efficiency transformation of yeast by electroporation. *Methods Enzymol.,* 1991, vol. 194, 182-187
- **BEGGS, J.D.** Transformation of yeast by a replicating hybrid plasmid. *Nature,* 1978, vol. 275, 104-109
- **BERGER, J. ; J. HAUBER ; R. HAUBER ; R. GEIGER et al.** Secreted placental alkaline phosphatase: A powerful new qunatitative indicator of gene expression in eukaryotic cells. *Gene,* 1988, vol. 66, 1-10
- **BODINE, D.M. ; K.T. MCDONAGH ; N.E. SEIDEL ; A.W. NIENHUIS.** Survival and retrovirus infection of murine hematopoietic stem cells in vitro: effects of 5-FU and method of infection. *Exp. Hematol.,* 1991, vol. 19, 206-212
- **CAPECCHI, M.R.** High efficiency transformation by direct microinjection of DNA into cultured mammalian cells. *Cell,* 1980, vol. 22, 479
- **CASE, M.E. ; M. SCHWEIZER ; S.R. KUSHNER ; N.H. GILES.** Efficient transformation of Neurospora crassa by utilizing hybrid plasmid DNA. *Proc Natl Acad Sci U S A.,* 1979, vol. 76, 5259-63
- **CEPKO, C.L. ; B.E. ROBERTS ; R.E. MULLIGAN.** Construction and applications of a highly transmissible murine retrovirus shuttle vector. *Cell,* 1984, vol. 37, 1053-1062
- **CHAGNON, Y.C. ; L. PERUSSE ; M. LAMOTHE ; M. CHAGNON et al.** Suggestive linkages between markers on human 1 p32-p22 and body fat and insulin levels in the Quebec Family Study. *Obes Res.,* 1997, vol. 5, 115-21
- **CHALFIE, M. ; Y. TU ; G. EUSKIRCHEN ; W.W. WARD et al.** Green fluorescent protein as a marker for gene expression. *Science,* 1994, vol. 263, 802-805
- **CHANEY, W.G. ; D.R. HOWARD ; J.W. POLLARD ; S. SALLUSTIO et al.** Highfrequency transfection of CHO cells using Polybrene. *Somatic Cell Mol. Genet.,* 1986, vol. 12, 237

- **CHEN, C. ; H. OKAYAMA.** Calcium phosphate-mediated gene transfer: A highly efficient system for stably transforming cells with plasmid DNA. *BioTechniques,* 1988, vol. 6, 632-638
- **COHEN, S.M.N. ; A.C.Y. CHANG ; L. HSU.** Non-chromosomal antibiotic resistance in bacteria: Genetic transformation of Escherichia coli by R-factor DNA. *Proc. Natl. Acad. Sci. USA.,* 1972, vol. 69, 2110
- **DE LOUVENCOURT, L. ; H. FUKUHARA ; H. HESLOT ; M. WESOLOWSKI.** Transformation of Kluyveromyces lactis by killer plasmid DNA. *J Bacteriol.,* 1983, vol. 154, 737-42
- **DE WET, J.R. ; K.V. WOOD ; M. DELUCA ; D.R. HELINSKI et al.** Sturcture and expression in mammalian cells. *Mol. Cell Biol.,* 1987, vol. 7, 725-737
- **DERVAN, P.B.** Characterization of protein-DNA complexes by affinity cleaving. *Methods Enzymol.,* 1991, vol. 208, 497-515
- **ELROY-STEIN, O. ; B. MOSS.** Cytoplasmic expression system based on constitutive synthesis of bacteriophage T7 RNA polymerase in mammalian cells. *Proc. Natl. Acad. Sci. USA.,* 1990, vol. 87, 6743-6747
- **ESCUDERO, J. ; B. HOHN.** Transfer and integration of T-DNA without cell injury in the host plant. *Plant Cell.,* 1997, vol. 9, 2135-2142
- **FEKETE, D.M. ; C.L. CEPKO.** Retroviral infection coupled with tissue transplantation limits gene transfer in the chick embryo. *Proc. Natl. Acad. Sci. USA.,* 1993, vol. 90, 2350-2354
- **FELGNER, P.L. ; T.R. GADEK ; M. HOLM ; R. ROMAN et al.** Lipofectin: A highly efficient, lipid-mediated DNA/transfection procedure. *Proc. Natl. Acad. Sci. USA.,* 1987, vol. 84, 7413-7417
- **FINER, J.J. ; K.R. FINER ; T. PONAPPA.** Particle bombardment-mediated transformation. *Current Topics in microbiology and immunology.,* 1999, vol. 240, 59-80
- **FLEER, R. ; P. YEH ; N. AMELLAL ; I. MAURY et al.** Stable multicopy vectors for high-level secretion of recombinant human serum albumin by Kluyveromyces yeasts. *Biotechnology (N Y).,* 1991, vol. 9, 968-75
- **FROMM, M. ; L.P. TAYLOR ; V. WALBOT.** Expression of genes transferred into monocot and dicot plant cells by electroporation. *Proc. Natl. Acad. Sci. USA.,* 1985, vol. 82, 5824-5828
- **FUJITA, T. ; H. SHUBIYA ; T. OHASHI ; K. YAMANISHI et al.** Regulation of human interleukin-2 gene: Functional DNA sequences in the 5' flanking region for the gene expression in activated T lymphocytes. *Cell,* 1986, vol. 46, 401-407
- **GALLAGHER, S.R.** GUS protocols: Using the GUS gene as a reporter of gene expression. Academic Press, 1992
- Growth and transformation of Saccharomyces cerevisiae. **GIETZ, R.D. ; R.A. WOODS ; P. MANIVASAKAM ; R.H. SCHIESTL.** Cells: A laboratory manual. Cold Spring Harbor Press, 1998, vol. I
- **GORMAN, C.M. ; L.F. MOFFAT ; B.H. HOWARD.** Recombinant genomes which express chloramphenicol acetyltransferase in mammalian cells. *Mol. Cell. Biol.,* 1982, vol. 2, 1044-1051
- **GRAHAM, F.L. ; A.J. VAN DER EB.** A new technique for the assay of infectivity of human adenovirus 5 DNA. *Virology,* 1973, vol. 52, 456
- **HANAHAN, D.** Studies on transformation of Escherichia coli with plasmids. *J. Mol. Biol.,* 1983, vol. 166, 557-580
- **HANSEN, G. ; M.-D. CHILTON.** Lessons in gene transfer to plants by a gifted microbe. *Curr. Top. Microbiol. Immunol.,* 1999, vol. 240, 21-57
- **HANSEN, G. ; M.S. WRIGHT.** Recent advances in the transformation of plants. *Trends Plant Sci.,* 1999, vol. 4, 226-231
- **HINNEN, A. ; J.B. HICKS ; G.R. FINK.** Transformation of yeast. *Proc. Natl. Acad. Sci. USA.,* 1978, vol. 75, 1929-1933
- **HOFFMAN, F.** Laser microbeams for the manipulation of plant cells and subcellular structures. *Plant Sci.,* 1996, vol. 113, 1-11
- **ISHIKAWA, K. ; T. NAGASE ; M. SUYAMA ; N. MIYAJIMA et al.** Prediction of the coding sequences of unidentified human genes. X. The complete sequences of 100 new cDNA clones from brain which can code for large proteins in vitro. *DNA Res.,* 1998, vol. 5, 169-76
- **ISHIURA, M. ; S. HIROSE ; T. UCHIDA ; Y. HAMADA et al.** Phage particle-mediated gene transfer to cultured mammalian cells. *Molecular and Cellular Biology.,* 1982, vol. 2, 607-616
- **ITO, H. ; Y. FUKUDA ; K. MURATA ; A. KIMURA.** Transformation of intact yeast cells treated with alkali cations. *J. Bacteriol.,* 1983, vol. 153, 163-168
- **KAUFMAN, R.J. ; P. MURTHA ; D.E. INGOLIA ; C.-Y. YEUNG et al.** Selection and amplification of heterologous genes encoding adenosine deaminase in mammalian cells. *Proc. Natl. Acad. Sci. USA.,* 1986, vol. 83, 3136-3140
- **KAWAI, S. ; M. NISHIZAWA.** New procedure for DNA transfection with polycation and dimethyl sulfoxide. *Mol. Cell. Biol.,* 1984, vol. 4, 1172
- **KELLY, J.M. ; M.J. HYNES.** Transformation of Aspergillus niger by the amdS gene of Aspergillus nidulans. *Embo J.,* 1985, vol. 4, 475-9
- **LEDUC, N.** Isolated maize zygotes mimic in vivo embryogenic development and express microinjected genes when cultured in vitro. *Dev. Biol.,* 1996, vol. 10, 190-203

- **LEMISCHKA, I.R. ; D.H. RAULET ; R.C. MULLIGAN.** Developmental potential and dynamic behavior of hematopoietic stem cells. *Cell,* 1986, vol. 45, 917-927
- **LITTLEFIELD, J.W.** Selection of hybrids from matings of fibroblasts in vitro and their presumed recombinants. *Science,* 1964, vol. 145, 709-710
- **LOPATA, M.A. ; D.W. CLEVELAND ; B. SOLLNER-WEBB.** High-level expression of a chloramphenicol acetyltransferase gene by DEAEdextran-mediated DNA traansfection couled with a dimethylsulfoxide or glycerol shock treatment. *Nucleic Acids Research,* 1984, vol. 12, 5707
- Cloning and expression of heterologous genes in insect cells with baculovirus vectors. **LUCKOW, V.A.** Recombinant DNA technology and applications. McGraw-Hill, 1991, 97-152
- **MANDEL, M. ; A. HIGA.** Calcium-dependent bacteriophage DNA infection. *J. Mol. biol.,* 1970, vol. 53, 159-162
- **MILLER, A.D. ; C. BUTTIMORE.** Redesign of retrovirus packaging cell lines to avoid recombination leading to helper virus production. *Mol. Cell biol.,* 1986, vol. 6, 2895-2902
- **MILLER, L.K.** Baculoviruses as gene expression vectors. *Annu. Rev. Microbiol.,* 1988, vol. 42, 177-199
- **NEUMANN, E. ; M. SCHAEFER-RIDDER ; Y. WANG ; P.H. HOFSCHNEIDER.** Gene transfer into mouse lyoma cells by electroporation in high electric fields. *EMBO J.,* 1982, vol. 1, 841-845
- **O'REILLY, D.R. ; L.K. MILLER ; V.A. LUCKOW.** Baculovirus expression vectors. W.H. Freeman and Company, 1992
- **OU-LEE, T.M. ; R. TURGEON ; R. WU.** Uptake and expression of a foreign gene linked to either a plant virus or Drosophila promoter in protoplasts of rice, wheat and sorghum. *Proc. Natl. Acad. Sci. USA.,* 1986, vol. 83, 6815-6819
- **PALMER, T.D. ; R.A. HOCK ; W.R.A. OSBORNE ; A.D. MILLER.** Efficient retrovirus-mediated transfer and expression of a human adenosine deaminase gene in diploid skin fibroblasts from an adenosie-deficient human. *Proc. Natl. Acad. Sci. USA.,* 1987, vol. 84, 1055-1059
- **PEAR, W. ; G. NOLAN ; M. SCOTT ; D. BALTIMORE.** Production of high-titer helper-free retroviruses by transient transfection. *Proc. Natl. Acad. Sci. USA,* 1993, vol. 90, 8392-8396
- **PONTING, C.P. ; L. ARAVIND.** START: a lipid-binding domain in StAR, HD-ZIP and signalling proteins. *Trends Biochem Sci.,* 1999, vol. 24, 130-2
- **POTTER, H.** Electroporation in biology: Methods, applications, and instrumentation. *Analytical Biochemistry,* 1988, vol. 174, 361-373
- **POTTER, H. ; L. WEIR ; P. LEDER.** Enhancer-dependent expression of human kappa immunoglobulin genes introduced into mouse pre-B lymphocytes by electroporation. *Proc. Natl. Acad. Sci. USA.,* 1984, vol. 81, 7161-7165
- **RASSOULZADEGAN, M. ; B. BINETRUY ; F. CUZIN.** High frequency of gene transfer after fusion between bacteria and eukaryotic cells. *Nature,* 1982, vol. 295, 257
- **RHODES, C.A. ; D.A. PIERCE ; I.J. METTLER ; D. MASCARENHAS et al.** Genetically transformed maize plants from protoplasts. *Science,* 1988, vol. 240, 204-207
- **ROSE, J.K. ; L. BUONOCORE ; M. WHITT.** A new cationic liposome reagent mediating nearly quantitative transfection of animal cells. *BioTechniques,* 1991, vol. 10, 520-525
- **SANDRI-GOLDIN, R.M. ; A.L. GOLDIN ; J.C. GLORIOSO ; M. LEVINE.** Highfrequency transfer of cloned herpes simjplex virus type I sequences to mammalian cells by protoplast fusion. *Mol. Cell. Biol.,* 1981, vol. 1, 7453-752
- Plant gene transfer using electrofusion and electroporation. **SAUNDERS, J.A. ; B.F. MATTHEWS ; P.D. MILLER.** Electroporation and electrofusion in cell biology. Plenum Press, 1989, 343-354
- **SCHAFFNER, W.** Direct transfer of cloned genes from bacteria to mammalian cells. *Proc. Natl. Acad. Sci. USA.,* 1980, vol. 77, 2163
- **SELDEN, R.F. ; K. BURKE-HOWIE ; M.E. ROWE ; H.M. GOODMAN et al.** Human growth hormone as a reporter gene in regulation studies employing transient gene expression. *Molecular and Cellular Bilololgy.,* 1986, vol. 6, 3173-3179
- **SHIGEKAWA, K. ; W.J. DOWER.** Electroporation of eukaryotes and prokaryotes: A general approach to the introduction of macomolecules into cells. *BioTechniques,* 1988, vol. 6, 742-751
- Methods of genetic transformations: Electroporation and polyethylene glycol treatment. **SHILLITO, R.** Molecular improvement of cereal crop. Kluwer, 1999, 9-20
- **SHIMKETS, R.A. ; D.G. LOWE ; J.T. TAI ; P. SEHL et al.** Gene expression analysis by transcript profiling coupled to a gene database query. *Nat Biotechnol.,* 1999, vol. 17, 798-803, java/Propub/biotech/nbt0899_798.fulltext java/Propub/biotech/nbt0899_798.abstract.
- **SIMONSEN, C.C. ; A.D. LEVINSON.** Isolation and expression of an altered mouse dihydrofolate reductase cDNA. *Proc. Natl. Acad. Sci. USA,* 1983, vol. 80, 2495-2499
- **SOUTHERN, P.J. ; P. BERG.** Transformation of mammalian cells to antibiotic resistanced with a bacterial gene under control of the SV40 early region promoter. *J. Mol. Appl. Gen.,* 1982, vol. 1, 327-341

- **SREEKRISHNA, K. ; R.H. POTENZ ; J.A. CRUZE ; W.R. MCCOMBIE et al.** High level expression of heterologous proteins in methylotrophic yeast Pichia pastoris. *J Basic Microbiol.,* 1988, vol. 28, 265-78
- **SURWIT, R.S. ; M.N. FEINGLOS ; J. RODIN ; A. SUTHERLAND et al.** Differential effects of fat and sucrose on the development of obesity and diabetes in C57BL/6J and A/J mice. *Metabolism,* 1995, vol. 44, 645-51
- **THOMPSON, J.A.** Maize transformation utilizing silicon carbide whiskers: A review. *Euphytica,* 1995, vol. 85, 75-80
- **TILBURN, J. ; C. SCAZZOCCHIO ; G.G. TAYLOR ; J.H. ZABICKY-ZISSMAN et al.** Transformation by integration in Aspergillus nidulans. *Gene,* 1983, vol. 26, 205-21
- **TOURAEV, A.** Plant male germ line transformation. *Plant J.,* 1997, vol. 12, 949-956
- **TRICK, H.N.** Recent advances in soybean transformation. *Plant Tissue Cult. Biotechnol.,* 1997, vol. 3, 9-26
- **TURNER, D.L. ; E.Y. SNYDER ; C.L. CEPKO.** Lineage-independent determinationh of cell type in the embryonic mouse retina. *Neuron,* 1990, vol. 4, 833-845
- **WEST, D.B. ; J. WAGUESPACK ; B. YORK ; J. GOUDEY-LEFEVRE et al.** Genetics of dietary obesity in AKR/J x SWR/J mice: segregation of the trait and identification of a linked locus on chromosome 4. *Mamm Genome.,* 1994, vol. 5, 546-52
- **WHITT, M.A. ; L. BUONOCORE ; J.K. ROSE ; V. CICCARONE et al.** TransfectACE reagent promotes transient transfection frequencies greater than 90%. *Focus,* 1990, vol. 13, 8-12
- **WIGLER, M. ; A. PELLICER ; S. SILVERSTTEIN ; R. AXEL.** Biochemical transfer of single-copy eucaryotic genes using total cellular DNA as donor. *Cell,* 1978, vol. 14, 725
- **WILLIAMS, D.A. ; I.R. LEMISCHKA ; D.G. NATHAN ; R.C. MULLIGAN.** Introduction of a new genetic material into pluripotent haematopoietic stem cells of the mouse. *Nature,* 1984, vol. 310, 476-480
- **WONG, T.K. ; E. NEUMANN.** Electric field mediated gene transfer. *Biochemical and Biophysical Research Communications.,* 1982, vol. 107, 584-587
- **YELTON, M.M. ; J.E. HAMER ; W.E. TIMBERLAKE.** Transformation of Aspergillus nidulans by using a trpC plasmid. *Proc Natl Acad Sci U S A.,* 1984, vol. 81, 1470-4
- **YU, X.X. ; J.L. BARGER ; B.B. BOYER ; M.D. BRAND et al.** Impact of endotoxin on UCP homolog mRNA abundance, thermoregulation, and mitochondrial proton leak kinetics. *Am J Physiol Endocrinol Metab.,* 2000, vol. 279, E433-46
- **ZHOU, G.** Introduction of exogenous DNA into cotton embryos. *Methods Enzymol.,* 1983, vol. 101, 433-481

SEQUENCE LISTING

<110> Adams, Sean H.
Goddard, Audrey D.
Grimaldi, J. Christopher

<120> BFIT Compositions and Methods of Use

<130> 10716-76

<140> Unknown
<141> 2000-11-27

<160> 23

<170> PatentIn Ver. 2.1

<210> 1
<211> 1857
<212> DNA
<213> Homo sapiens

<400> 1

```
gtggaattgc cctttcaaat gatccagaat gtcggaaatc acctgcgacg gggcttggcc 60
tctgtgttct ccaaccgcac atcccggaag tcagccttac gtgcggggaa cgacagtgcc 120
atggcagacg gcgagggata ccggaacccc acggaggtgc agatgagcca gctggtgctg 180
ccctgccaca ccaaccaacg tggtgagctg agcgtcgggc agctgctcaa gtggattgac 240
accacggctt gcctgtccgc ggagaggcac gctggctgcc cctgtgtcac agcttccatg 300
gatgacatct attttgagca caccattagt gttggacaag tggtgaatat caaggccaag 360
gtgaaccggg ccttcaactc cagcatggag gtgggcatcc aggtggcctc ggaggacctg 420
tgctctgaga agcagtggaa tgtgtgcaag gccttggcca ccttcgtggc ccgccgagag 480
atcaccaagg tgaagctgaa gcagatcacg ccgcggacag aagaggagaa gatggagcac 540
agtgtggcgg ctgagcgccg gcgcatgcgc cttgtctatg cagacaccat caaggacctc 600
ctggccaact gcgccattca gggcgatctg gagagcagag actgtagccg catggtgccg 660
gctgagaaga cccgtgtgga gagtgtggag ctggtcctgc ctccccacgc caatcaccag 720
ggcaacacct ttgggggcca gatcatggcc tggatggaga atgtggccac cattgcagcc 780
agccggctct gccgtgccca ccctacgctg aaggccattg aaatgttcca cttccgaggc 840
ccgtcccagg tcggcgaccg tctggtgctc aaagccatcg tgaacaatgc cttcaaacat 900
agcatggagg tgggcgtgtg cgtggaggcc tatcgccagg aggctgagac ccaccggcgc 960
cacatcaaca gtgcctttat gacctttgtg gtcctggacg cagatgacca gccccagttg 1020
ctgccctgga ttcggcccca gcccggcgat ggtgagcggc ggtaccgaga ggccagtgcc 1080
agaaagaaga tccgcctgga caggaagtac atcgtgtcct gtaagcagac agaggtgccc 1140
ctctccgtcc cctgggaccc tagcaaccag gtgtacctga gctacaataa cgtctcctcc 1200
ttgaagatgc ttgtggccaa ggacaactgg gtgctgtcct cggagatcag tcaggtccgc 1260
ctgtacactc tggaggatga caagttcctc tccttccaca tggagatggt ggtgcatgtg 1320
gatgcagccc aggccttcct gctgctctcg gacctgcgtc agaggccaga gtgggacaag 1380
cactaccgga gcgtggagct agtgcagcag gtagacgagg acgacgccat ctaccacgtc 1440
accagccctg ccctcggagg tcacacaaag ccccaggact tcgtgatcct ggcctcgagg 1500
```

```
cggaagcctt gtgacaatgg ggacccctat gtcatcgcgc tgaggtcggt cacgctgccc 1560
acacaccgag agacgccaga gtacagacgc ggagagaccc tctgctcagg cttctgcctc 1620
tggcgcgagg gggaccagct gaccaagtgc tgctgggtta gggtctccct gactgagctg 1680
gtctcggcaa gtggcttcta ttcctggggg ctcgaatcca ggtcaaaggg tcgcaggagc 1740
gacggttgga atggaaaact agctggagga cacctgagta ctcttaaagc aatccccgtg 1800
gccaaaatca acagccgatt tggatacctt caagacacct gaaaccttat catgagc    1857
```

<210> 2
<211> 607
<212> PRT
<213> Homo sapiens

<400> 2

```
Met Ile Gln Asn Val Gly Asn His Leu Arg Arg Gly Leu Ala Ser Val
1               5                   10                  15

Phe Ser Asn Arg Thr Ser Arg Lys Ser Ala Leu Arg Ala Gly Asn Asp
            20                  25                  30

Ser Ala Met Ala Asp Gly Glu Gly Tyr Arg Asn Pro Thr Glu Val Gln
        35                  40                  45

Met Ser Gln Leu Val Leu Pro Cys His Thr Asn Gln Arg Gly Glu Leu
    50                  55                  60

Ser Val Gly Gln Leu Leu Lys Trp Ile Asp Thr Thr Ala Cys Leu Ser
65                  70                  75                  80

Ala Glu Arg His Ala Gly Cys Pro Cys Val Thr Ala Ser Met Asp Asp
                85                  90                  95

Ile Tyr Phe Glu His Thr Ile Ser Val Gly Gln Val Val Asn Ile Lys
            100                 105                 110

Ala Lys Val Asn Arg Ala Phe Asn Ser Ser Met Glu Val Gly Ile Gln
        115                 120                 125

Val Ala Ser Glu Asp Leu Cys Ser Glu Lys Gln Trp Asn Val Cys Lys
    130                 135                 140

Ala Leu Ala Thr Phe Val Ala Arg Arg Glu Ile Thr Lys Val Lys Leu
145                 150                 155                 160

Lys Gln Ile Thr Pro Arg Thr Glu Glu Glu Lys Met Glu His Ser Val
                165                 170                 175

Ala Ala Glu Arg Arg Arg Met Arg Leu Val Tyr Ala Asp Thr Ile Lys
```

180 185 190

Asp Leu Leu Ala Asn Cys Ala Ile Gln Gly Asp Leu Glu Ser Arg Asp
195 200 205

Cys Ser Arg Met Val Pro Ala Glu Lys Thr Arg Val Glu Ser Val Glu
210 215 220

Leu Val Leu Pro Pro His Ala Asn His Gln Gly Asn Thr Phe Gly Gly
225 230 235 240

Gln Ile Met Ala Trp Met Glu Asn Val Ala Thr Ile Ala Ala Ser Arg
245 250 255

Leu Cys Arg Ala His Pro Thr Leu Lys Ala Ile Glu Met Phe His Phe
260 265 270

Arg Gly Pro Ser Gln Val Gly Asp Arg Leu Val Leu Lys Ala Ile Val
275 280 285

Asn Asn Ala Phe Lys His Ser Met Glu Val Gly Val Cys Val Glu Ala
290 295 300

Tyr Arg Gln Glu Ala Glu Thr His Arg Arg His Ile Asn Ser Ala Phe
305 310 315 320

Met Thr Phe Val Val Leu Asp Ala Asp Asp Gln Pro Gln Leu Leu Pro
325 330 335

Trp Ile Arg Pro Gln Pro Gly Asp Gly Glu Arg Arg Tyr Arg Glu Ala
340 345 350

Ser Ala Arg Lys Lys Ile Arg Leu Asp Arg Lys Tyr Ile Val Ser Cys
355 360 365

Lys Gln Thr Glu Val Pro Leu Ser Val Pro Trp Asp Pro Ser Asn Gln
370 375 380

Val Tyr Leu Ser Tyr Asn Asn Val Ser Ser Leu Lys Met Leu Val Ala
385 390 395 400

Lys Asp Asn Trp Val Leu Ser Ser Glu Ile Ser Gln Val Arg Leu Tyr
405 410 415

Thr Leu Glu Asp Asp Lys Phe Leu Ser Phe His Met Glu Met Val Val
420 425 430

His Val Asp Ala Ala Gln Ala Phe Leu Leu Leu Ser Asp Leu Arg Gln

```
                435                 440                 445

Arg Pro Glu Trp Asp Lys His Tyr Arg Ser Val Glu Leu Val Gln Gln
    450                 455                 460

Val Asp Glu Asp Asp Ala Ile Tyr His Val Thr Ser Pro Ala Leu Gly
465                 470                 475                 480

Gly His Thr Lys Pro Gln Asp Phe Val Ile Leu Ala Ser Arg Arg Lys
                485                 490                 495

Pro Cys Asp Asn Gly Asp Pro Tyr Val Ile Ala Leu Arg Ser Val Thr
            500                 505                 510

Leu Pro Thr His Arg Glu Thr Pro Glu Tyr Arg Arg Gly Glu Thr Leu
        515                 520                 525

Cys Ser Gly Phe Cys Leu Trp Arg Glu Gly Asp Gln Leu Thr Lys Cys
    530                 535                 540

Cys Trp Val Arg Val Ser Leu Thr Glu Leu Val Ser Ala Ser Gly Phe
545                 550                 555                 560

Tyr Ser Trp Gly Leu Glu Ser Arg Ser Lys Gly Arg Arg Ser Asp Gly
                565                 570                 575

Trp Asn Gly Lys Leu Ala Gly Gly His Leu Ser Thr Leu Lys Ala Ile
            580                 585                 590

Pro Val Ala Lys Ile Asn Ser Arg Phe Gly Tyr Leu Gln Asp Thr
        595                 600                 605
```

<210> 3
<211> 1818
<212> DNA
<213> Homo sapiens

<400> 3

```
gtggaattgc cctttgcatc aaatgatcca gaatgtcgga aatcacctgc gacggggctt 60
ggcctctgtg ttctccaacc gcacatcccg gaagtcagcc ttacgtgcgg ggaacgacag 120
tgccatggca gacggcgagg gataccggaa ccccacggag gtgcagatga gccagctggt 180
gctgccctgc cacaccaacc aacgtggtga gctgagcgtc gggcagctgc tcaagtggat 240
tgacaccacg gcttgcctgt ccgcggagag gcacgctggc tgcccctgtg tcacagcttc 300
catggatgac atctattttg agcacaccat tagtgttgga caagtggtga atatcaaggc 360
caaggtgaac cgggccttca actccagcat ggaggtgggc atccaggtgg cctcggagga 420
cctgtgctct gagaagcagt ggaatgtgtg caaggccttg gccaccttcg tggcccgccg 480
agagatcacc aaggtgaagc tgaagcagat cacgccgcgg acagaagagg agaagatgga 540
```

```
gcacagtgtg gcggctgagc gccggcgcat gcgccttgtc tatgcagaca ccatcaagga 600
cctcctggcc aactgcgcca ttcagggcga tctggagagc agagactgta gccgcatggt 660
gccggctgag aagacccgtg tggagagtgt ggagctggtc ctgcctcccc acgccaatca 720
ccagggcaac acctttgggg gccagatcat ggcctggatg gagaatgtgg ccaccattgc 780
agccagccgg ctctgccgtg cccaccctac gctgaaggcc attgaaatgt tccacttccg 840
aggcccgtcc caggtcggcg accgtctggt gctcaaagcc atcgtgaaca atgccttcaa 900
acatagcatg gaggtgggcg tgtgcgtgga ggcctatcgc caggaggctg agacccaccg 960
gcgccacatc aacagtgcct ttatgacctt tgtggtcctg gacgcagatg accagcccca 1020
gttgctgccc tggattcggc cccagcccgg cgatggtgag cggcggtacc gagaggccag 1080
tgccagaaag aagatccgcc tggacaggaa gtacatcgtg tcctgtaagc agacagaggt 1140
gcccctctcc gtcccctggg accctagcaa ccaggtgtac ctgagctaca ataacgtctc 1200
ctccttgaag atgcttgtgg ccaaggacaa ctgggtgctg tcctcggaga tcagtcaggt 1260
ccgcctgtac actctggagg atgacaagtt cctctccttc cacatggaga tggtggtgca 1320
tgtggatgca gcccaggcct tcctgctgct ctcggacctg cgtcagaggc cagagtggga 1380
caagcactac cggagcgtgg agctagtgca gcaggtagac gaggacgacg ccatctacca 1440
cgtcaccagc cctgccctcg gaggtcacac aaagccccag gacttcgtga tcctggcctc 1500
gaggcggaag ccttgtgaca atggggaccc ctatgtcatc gcgctgaggt cggtcacgct 1560
gcccacacac cgagagacgc cagagtacag acgcggagag accctctgct caggcttctg 1620
cctctggcgc gaggggggacc agctgaccaa ggtatcctac tacaaccagg ccaccccagg 1680
tgttctcaac tatgtgacca ccaacgtggc cggcctctcc tctgagttct acaccacctt 1740
caaggcttgt gagcagtttc tcttggacaa ccggaatgat ctggccccca gcctccagac 1800
cctctagatg ccctcagc                                                1818
```

<210> 4
<211> 594
<212> PRT
<213> Homo sapiens

<400> 4

```
Met Ile Gln Asn Val Gly Asn His Leu Arg Arg Gly Leu Ala Ser Val
1               5                   10                  15

Phe Ser Asn Arg Thr Ser Arg Lys Ser Ala Leu Arg Ala Gly Asn Asp
            20                  25                  30

Ser Ala Met Ala Asp Gly Glu Gly Tyr Arg Asn Pro Thr Glu Val Gln
        35                  40                  45

Met Ser Gln Leu Val Leu Pro Cys His Thr Asn Gln Arg Gly Glu Leu
    50                  55                  60

Ser Val Gly Gln Leu Leu Lys Trp Ile Asp Thr Thr Ala Cys Leu Ser
65                  70                  75                  80

Ala Glu Arg His Ala Gly Cys Pro Cys Val Thr Ala Ser Met Asp Asp
                85                  90                  95
```

```
Ile Tyr Phe Glu His Thr Ile Ser Val Gly Gln Val Val Asn Ile Lys
            100                 105                 110

Ala Lys Val Asn Arg Ala Phe Asn Ser Ser Met Glu Val Gly Ile Gln
        115                 120                 125

Val Ala Ser Glu Asp Leu Cys Ser Glu Lys Gln Trp Asn Val Cys Lys
    130                 135                 140

Ala Leu Ala Thr Phe Val Ala Arg Arg Glu Ile Thr Lys Val Lys Leu
145                 150                 155                 160

Lys Gln Ile Thr Pro Arg Thr Glu Glu Glu Lys Met Glu His Ser Val
                165                 170                 175

Ala Ala Glu Arg Arg Arg Met Arg Leu Val Tyr Ala Asp Thr Ile Lys
            180                 185                 190

Asp Leu Leu Ala Asn Cys Ala Ile Gln Gly Asp Leu Glu Ser Arg Asp
        195                 200                 205

Cys Ser Arg Met Val Pro Ala Glu Lys Thr Arg Val Glu Ser Val Glu
    210                 215                 220

Leu Val Leu Pro Pro His Ala Asn His Gln Gly Asn Thr Phe Gly Gly
225                 230                 235                 240

Gln Ile Met Ala Trp Met Glu Asn Val Ala Thr Ile Ala Ala Ser Arg
                245                 250                 255

Leu Cys Arg Ala His Pro Thr Leu Lys Ala Ile Glu Met Phe His Phe
            260                 265                 270

Arg Gly Pro Ser Gln Val Gly Asp Arg Leu Val Leu Lys Ala Ile Val
        275                 280                 285

Asn Asn Ala Phe Lys His Ser Met Glu Val Gly Val Cys Val Glu Ala
    290                 295                 300

Tyr Arg Gln Glu Ala Glu Thr His Arg Arg His Ile Asn Ser Ala Phe
305                 310                 315                 320

Met Thr Phe Val Val Leu Asp Ala Asp Asp Gln Pro Gln Leu Leu Pro
                325                 330                 335

Trp Ile Arg Pro Gln Pro Gly Asp Gly Glu Arg Arg Tyr Arg Glu Ala
            340                 345                 350
```

```
Ser Ala Arg Lys Lys Ile Arg Leu Asp Arg Lys Tyr Ile Val Ser Cys
        355                 360                 365

Lys Gln Thr Glu Val Pro Leu Ser Val Pro Trp Asp Pro Ser Asn Gln
    370                 375                 380

Val Tyr Leu Ser Tyr Asn Asn Val Ser Ser Leu Lys Met Leu Val Ala
385                 390                 395                 400

Lys Asp Asn Trp Val Leu Ser Ser Glu Ile Ser Gln Val Arg Leu Tyr
                405                 410                 415

Thr Leu Glu Asp Asp Lys Phe Leu Ser Phe His Met Glu Met Val Val
            420                 425                 430

His Val Asp Ala Ala Gln Ala Phe Leu Leu Leu Ser Asp Leu Arg Gln
        435                 440                 445

Arg Pro Glu Trp Asp Lys His Tyr Arg Ser Val Glu Leu Val Gln Gln
    450                 455                 460

Val Asp Glu Asp Asp Ala Ile Tyr His Val Thr Ser Pro Ala Leu Gly
465                 470                 475                 480

Gly His Thr Lys Pro Gln Asp Phe Val Ile Leu Ala Ser Arg Arg Lys
                485                 490                 495

Pro Cys Asp Asn Gly Asp Pro Tyr Val Ile Ala Leu Arg Ser Val Thr
            500                 505                 510

Leu Pro Thr His Arg Glu Thr Pro Glu Tyr Arg Arg Gly Glu Thr Leu
        515                 520                 525

Cys Ser Gly Phe Cys Leu Trp Arg Glu Gly Asp Gln Leu Thr Lys Val
    530                 535                 540

Ser Tyr Tyr Asn Gln Ala Thr Pro Gly Val Leu Asn Tyr Val Thr Thr
545                 550                 555                 560

Asn Val Ala Gly Leu Ser Ser Glu Phe Tyr Thr Thr Phe Lys Ala Cys
                565                 570                 575

Glu Gln Phe Leu Leu Asp Asn Arg Asn Asp Leu Ala Pro Ser Leu Gln
            580                 585                 590

Thr Leu
```

<210> 5
<211> 2699
<212> DNA

<213> Mus musculus

<400> 5

ctagagatcc ctcgacctcg acccacgcgt ccgagacccc cccacagcct ggcaacccag 60
caaacggagc agcaatgatt cagaatgtgg gcaaccactt gcgaaggggc ttcgcctcta 120
tgttctctaa tcgcacatcc cggaagtcaa tctcccatcc ggagtctgga gaccctccta 180
ccatggcaga gggtgaagga taccggaacc ccacggaggt gcagatgagc cagctggtac 240
tgccctgcca caccaaccac cgtggggagc tgagcattgg acagttgctc aagtggatcg 300
acaccacagc ctgcctatca gcggagaggc atgctggctg tccctgcgtc acagcctcta 360
tggatgacat ctacttcgac cataccatta gtgtcggcca agtggtgaat atcaaggcca 420
aggtgaaccg ggccttcaac tccagcatgg aggtgggaat ccaggtggtc tctgaggatc 480
tgtgctctga gaagcagtgg agtgtctgca aggccttggc cacctttgtg gcccaccggg 540
agctctccaa ggtgaagctg aagcaggtca tcccattgac cgaggaggag aagactgaac 600
atggggtggc ggctgagcgc cggcgtatgc gactggtcta tgcagacacc atcaaagatc 660
tcctaaccca ctgtgtcatc caggacgatt tggacaagga ctgcagcaat atggtgccag 720
ccgagaagac ccgagtggag agtgtggagc tggtgctgcc tcctcacgcc aatcatcagg 780
gcaatacctt cgggggacag atcatggctt ggatggagaa tgtggccacc attgcagcca 840
gccggctctg tcacgcccac cctacgctca aggccatcga gatgttccat ttccgaggcc 900
cgtctcaggt gggggaccgt ctggtgctca aggccatcgt gaataacgcc tttaagcaca 960
gcatggaggt gggtgtgtgt gtggaggcgt accgccagga agctgagacc cagcgccggc 1020
acatcaacag cgccttcatg accttcgtgg tcctggacaa agatgaccag cctcagaagc 1080
tgccctggat tcgtccccag cctggagagg gtgaacggcg ataccgagaa gccagtgcca 1140
ggaagaagat ccgcctggac aggaaatacc ttgtgtcctg taagcaggca gaagtggccc 1200
tgtctgtccc ctgggaccct agcaaccagg tatacctgag ctactacaac gtgtcctctc 1260
tgaagacgct catggccaag gacaactggg tgctgtccgt ggagatcagc gaggtccgcc 1320
tgtacatcct agaagaggac ttcctctcct ttcacttgga gatggtggta aatgtggatg 1380
ccgcccaggt ctttcagctg ctgtcagacc tgcgcaggag accagagtgg gacaagcatt 1440
accggagtgt ggagctggtg cagcaagtgg atgaggatga cgccatctac cacgtcatca 1500
gccccgccct gagcgggaac accaagcccc aggactttgt gatcctggcc tctaggcgga 1560
agccttgtga caatggggac ccctatgtca ttgccctgag gtcggtcacg ctgcccacgc 1620
accatgagac accggaatac caacgtgggg agactctctg ttcaggcttc tgtctgtggc 1680
gtgaggggga ccagatgact aaggtttctt actacaacca ggccaccccc ggctttctca 1740
actatgtgac caccaatgtg tccggcctgt cctcagaatt ctacaacact ttcaaggctt 1800
gtgagagttt tctgttggac aaccggaatg acctagctcc cagcctccag accctctaga 1860
caccacccat gctgccccag gtcttacaca gtgcgtggaa caaagcagag acatttattc 1920
accttgactc cccagggaag ccttccacac tagatggtcc aatcctactg gatggtcggt 1980
tgctgctcac atctgcctgc aagtctttcc agtactcctg ggatatcctg taatagactc 2040
gggtcctgtc cacggccctg gccgcccaca atccagccca caaatccaca tggctgttcc 2100
cagcagtgct gtggtacact gtgacagtgg ctctagggga ggaggccagg agcctggcca 2160
cagtgttggc tggactctga ctcagtggcc cagcctgcag ctggaaggac acaggttgcc 2220
ggagtccctg acacagctcc agcatatctg tgaccatctg ctcctgataa ccactgtcca 2280
gcatctcttc gggccagccc ggtgccacgg tcacatgggg gaagacttca gccacagctg 2340
tgagcagctc tctgccagct atgtggccag ggaccacaaa actaccatgt gagactgtgg 2400
accccaccca cacaggccag ggcagatggc caagggtaga aaggtgtgct aacgtggcca 2460

```
gggatggccg gagagcttcg ggttccacta tgttcacatg gatgccccag cgcctggggt 2520
gaacagccag acgcagcaga cacgactcca gtgtcagagc agggccacct ggggtgtgta 2580
ggatgggtac agggttccca gcctcaggtt cctggaggcc aatgtccagc aagatcatgc 2640
cttctctgtc tggaagaggc aacagtttgg agatcctgtc atcaaaaaaa aaaaaaaaa  2699
```

<210> 6
<211> 594
<212> PRT
<213> Mus musculus

<400> 6

```
Met Ile Gln Asn Val Gly Asn His Leu Arg Arg Gly Phe Ala Ser Met
1               5                   10                  15

Phe Ser Asn Arg Thr Ser Arg Lys Ser Ile Ser His Pro Glu Ser Gly
            20                  25                  30

Asp Pro Pro Thr Met Ala Glu Gly Glu Gly Tyr Arg Asn Pro Thr Glu
        35                  40                  45

Val Gln Met Ser Gln Leu Val Leu Pro Cys His Thr Asn His Arg Gly
    50                  55                  60

Glu Leu Ser Ile Gly Gln Leu Leu Lys Trp Ile Asp Thr Thr Ala Cys
65                  70                  75                  80

Leu Ser Ala Glu Arg His Ala Gly Cys Pro Cys Val Thr Ala Ser Met
                85                  90                  95

Asp Asp Ile Tyr Phe Asp His Thr Ile Ser Val Gly Gln Val Val Asn
            100                 105                 110

Ile Lys Ala Lys Val Asn Arg Ala Phe Asn Ser Ser Met Glu Val Gly
        115                 120                 125

Ile Gln Val Val Ser Glu Asp Leu Cys Ser Glu Lys Gln Trp Ser Val
    130                 135                 140

Cys Lys Ala Leu Ala Thr Phe Val Ala His Arg Glu Leu Ser Lys Val
145                 150                 155                 160

Lys Leu Lys Gln Val Ile Pro Leu Thr Glu Glu Glu Lys Thr Glu His
                165                 170                 175

Gly Val Ala Ala Glu Arg Arg Arg Met Arg Leu Val Tyr Ala Asp Thr
            180                 185                 190
```

```
Ile Lys Asp Leu Leu Thr His Cys Val Ile Gln Asp Asp Leu Asp Lys
        195                 200                 205

Asp Cys Ser Asn Met Val Pro Ala Glu Lys Thr Arg Val Glu Ser Val
    210                 215                 220

Glu Leu Val Leu Pro Pro His Ala Asn His Gln Gly Asn Thr Phe Gly
225                 230                 235                 240

Gly Gln Ile Met Ala Trp Met Glu Asn Val Ala Thr Ile Ala Ala Ser
                245                 250                 255

Arg Leu Cys His Ala His Pro Thr Leu Lys Ala Ile Glu Met Phe His
            260                 265                 270

Phe Arg Gly Pro Ser Gln Val Gly Asp Arg Leu Val Leu Lys Ala Ile
        275                 280                 285

Val Asn Asn Ala Phe Lys His Ser Met Glu Val Gly Val Cys Val Glu
    290                 295                 300

Ala Tyr Arg Gln Glu Ala Glu Thr Gln Arg Arg His Ile Asn Ser Ala
305                 310                 315                 320

Phe Met Thr Phe Val Val Leu Asp Lys Asp Asp Gln Pro Gln Lys Leu
                325                 330                 335

Pro Trp Ile Arg Pro Gln Pro Gly Glu Gly Glu Arg Arg Tyr Arg Glu
            340                 345                 350

Ala Ser Ala Arg Lys Lys Ile Arg Leu Asp Arg Lys Tyr Leu Val Ser
        355                 360                 365

Cys Lys Gln Ala Glu Val Ala Leu Ser Val Pro Trp Asp Pro Ser Asn
    370                 375                 380

Gln Val Tyr Leu Ser Tyr Tyr Asn Val Ser Ser Leu Lys Thr Leu Met
385                 390                 395                 400

Ala Lys Asp Asn Trp Val Leu Ser Val Glu Ile Ser Glu Val Arg Leu
                405                 410                 415

Tyr Ile Leu Glu Glu Asp Phe Leu Ser Phe His Leu Glu Met Val Val
            420                 425                 430

Asn Val Asp Ala Ala Gln Val Phe Gln Leu Leu Ser Asp Leu Arg Arg
        435                 440                 445
```

Arg Pro Glu Trp Asp Lys His Tyr Arg Ser Val Glu Leu Val Gln Gln
450 455 460

Val Asp Glu Asp Asp Ala Ile Tyr His Val Ile Ser Pro Ala Leu Ser
465 470 475 480

Gly Asn Thr Lys Pro Gln Asp Phe Val Ile Leu Ala Ser Arg Arg Lys
485 490 495

Pro Cys Asp Asn Gly Asp Pro Tyr Val Ile Ala Leu Arg Ser Val Thr
500 505 510

Leu Pro Thr His His Glu Thr Pro Glu Tyr Gln Arg Gly Glu Thr Leu
515 520 525

Cys Ser Gly Phe Cys Leu Trp Arg Glu Gly Asp Gln Met Thr Lys Val
530 535 540

Ser Tyr Tyr Asn Gln Ala Thr Pro Gly Phe Leu Asn Tyr Val Thr Thr
545 550 555 560

Asn Val Ser Gly Leu Ser Ser Glu Phe Tyr Asn Thr Phe Lys Ala Cys
565 570 575

Glu Ser Phe Leu Leu Asp Asn Arg Asn Asp Leu Ala Pro Ser Leu Gln
580 585 590

Thr Leu

<210> 7
<211> 19
<212> DNA
<213> Artificial Sequence

<220>
<223> Description of Artificial Sequence: Mouse BFIT forward primer

<400> 7
tgaaggatac cggaacccc 19

<210> 8
<211> 23
<212> DNA
<213> Artificial Sequence

<220>
<223> Description of Artificial Sequence: Mouse BFIT probe

<400> 8
cggaggtgca gatgagccag ctg 23

<210> 9

<211> 19
<212> DNA
<213> Artificial Sequence

<220>
<223> Description of Artificial Sequence: Mouse BFIT reverse primer

<400> 9
tactgccctg ccacaccaa 19

<210> 10
<211> 20
<212> DNA
<213> Artificial Sequence

<220>
<223> Description of Artificial Sequence: Human BFIT1 forward primer

<400> 10
tgctgggtta gggtctccct 20

<210> 11
<211> 24
<212> DNA
<213> Artificial Sequence

<220>
<223> Description of Artificial Sequence: Human BFIT1 probe

<400> 11
actgagctgg tctcggcaag tggc 24

<210> 12
<211> 19
<212> DNA
<213> Artificial Sequence

<220>
<223> Description of Artificial Sequence: Human BFIT1 reverse primer

<400> 12
tctattcctg ggggctcga 19

<210> 13
<211> 21
<212> DNA
<213> Artificial Sequence

<220>
<223> Description of Artificial Sequence: Human BFIT2 forward primer

<400> 13
tctcttggac aaccggaatg a 21

<210> 14
<211> 20
<212> DNA
<213> Artificial Sequence

<220>
<223> Description of Artificial Sequence: Human BFIT2 probe

<400> 14
tggcccccag cctccagacc 20

<210> 15
<211> 20
<212> DNA
<213> Artificial Sequence

<220>
<223> Description of Artificial Sequence: Human BFIT2 reverse primer

<400> 15
tctagatgcc ctcagtggcc 20

<210> 16
<211> 20
<212> DNA
<213> Artificial Sequence

<220>
<223> Description of Artificial Sequence: Mouse BFIT chromosomal locus forward primer

<400> 16
gtaagaaggg agcctgggag 20

<210> 17
<211> 20
<212> DNA
<213> Artificial Sequence

<220>
<223> Description of Artificial Sequence: Mouse BFIT chromosomal locus reverse primer

<400> 17
tctagaccac cctttctccg 20

<210> 18
<211> 22
<212> DNA
<213> Artificial Sequence

<220>
<223> Description of Artificial Sequence: AB014607 reverse primer

<400> 18
ccagaccctc tagatgccct ca 22

<210> 19
<211> 24
<212> DNA
<213> Artificial Sequence

<220>
<223> Description of Artificial Sequence: AB014607 forward primer

<400> 19
atgatccaga atgtcggaaa tcac 24

<210> 20
<211> 26
<212> DNA
<213> Artificial Sequence

<220>
<223> Description of Artificial Sequence: AB014607 reverse primer

<400> 20
agacacctga aaccttatca tgagcc 26

<210> 21
<211> 25
<212> DNA
<213> Artificial Sequence

<220>
<223> Description of Artificial Sequence: Inverse PCR primer e6.bat.snr1

<400> 21
gccactgagt cagagtccag ccaac 25

<210> 22
<211> 21
<212> DNA
<213> Artificial Sequence

<220>
<223> Description of Artificial Sequence: Inverse PCR primer e6.bat.snf1

<400> 22
ccagcctgca gctggaagga c 21

<210> 23
<211> 47
<212> DNA
<213> Artificial Sequence

<220>
<223> Description of Artificial Sequence: Mouse BFIT specific probe

<400> 23
aaatccacat ggctgttccc agcagtgctg tggtacactg tgacagt 47

SEQUENCE LISTING

<110> Adams, Sean H
Goddard, Audrey D
Grimaldi, J. Christopher
Chui, Clarissa J

<120> BFIT COMPOSITIONS AND METHODS OF USE

<130> 9800081-0014

<140> PCT/US 02/01783
<141> 2002-01-22

<150> US 60/263,362
<151> 2002-01-22

<160> 23

<170> PatentIn version 3.1

<210> 1
<211> 1857
<212> DNA
<213> Homo sapiens

<400> 1

gtggaattgc cctttcaaat gatccagaat gtcggaaatc acctgcgacg gggcttggcc 60
tctgtgttct ccaaccgcac atcccggaag tcagccttac gtgcggggaa cgacagtgcc 120
atggcagacg gcgagggata ccggaacccc acggaggtgc agatgagcca gctggtgctg 180
ccctgccaca ccaaccaacg tggtgagctg agcgtcgggc agctgctcaa gtggattgac 240
accacggctt gcctgtccgc ggagaggcac gctggctgcc cctgtgtcac agcttccatg 300
gatgacatct attttgagca caccattagt gttggacaag tggtgaatat caaggccaag 360
gtgaaccggg ccttcaactc cagcatggag gtgggcatcc aggtggcctc ggaggacctg 420
tgctctgaga agcagtggaa tgtgtgcaag gccttggcca ccttcgtggc ccgccgagag 480
atcaccaagg tgaagctgaa gcagatcacg ccgcggacag aagaggagaa gatggagcac 540
agtgtggcgg ctgagcgccg gcgcatgcgc cttgtctatg cagacaccat caaggacctc 600
ctggccaact gcgccattca gggcgatctg gagagcagag actgtagccg catggtgccg 660
gctgagaaga cccgtgtgga gagtgtggag ctggtcctgc ctccccacgc caatcaccag 720
ggcaacacct ttgggggcca gatcatggcc tggatggaga atgtggccac cattgcagcc 780
agccggctct gccgtgccca ccctacgctg aaggccattg aaatgttcca cttccgaggc 840
ccgtcccagg tcggcgaccg tctggtgctc aaagccatcg tgaacaatgc cttcaaacat 900
agcatggagg tgggcgtgtg cgtggaggcc tatcgccagg aggctgagac ccaccggcgc 960
cacatcaaca gtgcctttat gacctttgtg gtcctggacg cagatgacca gccccagttg 1020
ctgccctgga ttcggcccca gcccggcgat ggtgagcggc ggtaccgaga ggccagtgcc 1080

agaaagaaga tccgcctgga caggaagtac atcgtgtcct gtaagcagac agaggtgccc 1140
ctctccgtcc cctgggaccc tagcaaccag gtgtacctga gctacaataa cgtctcctcc 1200
ttgaagatgc ttgtggccaa ggacaactgg gtgctgtcct cggagatcag tcaggtccgc 1260
ctgtacactc tggaggatga caagttcctc tccttccaca tggagatggt ggtgcatgtg 1320
gatgcagccc aggccttcct gctgctctcg gacctgcgtc agaggccaga gtgggacaag 1380
cactaccgga gcgtggagct agtgcagcag gtagacgagg acgacgccat ctaccacgtc 1440
accagccctg ccctcggagg tcacacaaag ccccaggact tcgtgatcct ggcctcgagg 1500
cggaagcctt gtgacaatgg ggacccctat gtcatcgcgc tgaggtcggt cacgctgccc 1560
acacaccgag agacgccaga gtacagacgc ggagagaccc tctgctcagg cttctgcctc 1620
tggcgcgagg gggaccagct gaccaagtgc tgctgggtta gggtctccct gactgagctg 1680
gtctcggcaa gtggcttcta ttcctggggg ctcgaatcca ggtcaaaggg tcgcaggagc 1740
gacggttgga atggaaaact agctggagga cacctgagta ctcttaaagc aatccccgtg 1800
gccaaaatca acagccgatt tggatacctt caagacacct gaaaccttat catgagc 1857

<210> 2
<211> 607
<212> PRT
<213> Homo sapiens

<400> 2

```
Met Ile Gln Asn Val Gly Asn His Leu Arg Arg Gly Leu Ala Ser Val
1               5                   10                  15

Phe Ser Asn Arg Thr Ser Arg Lys Ser Ala Leu Arg Ala Gly Asn Asp
            20                  25                  30

Ser Ala Met Ala Asp Gly Glu Gly Tyr Arg Asn Pro Thr Glu Val Gln
        35                  40                  45

Met Ser Gln Leu Val Leu Pro Cys His Thr Asn Gln Arg Gly Glu Leu
    50                  55                  60

Ser Val Gly Gln Leu Leu Lys Trp Ile Asp Thr Thr Ala Cys Leu Ser
65                  70                  75                  80

Ala Glu Arg His Ala Gly Cys Pro Cys Val Thr Ala Ser Met Asp Asp
                85                  90                  95

Ile Tyr Phe Glu His Thr Ile Ser Val Gly Gln Val Val Asn Ile Lys
            100                 105                 110

Ala Lys Val Asn Arg Ala Phe Asn Ser Ser Met Glu Val Gly Ile Gln
```

115 120 125

Val Ala Ser Glu Asp Leu Cys Ser Glu Lys Gln Trp Asn Val Cys Lys
130 135 140

Ala Leu Ala Thr Phe Val Ala Arg Arg Glu Ile Thr Lys Val Lys Leu
145 150 155 160

Lys Gln Ile Thr Pro Arg Thr Glu Glu Glu Lys Met Glu His Ser Val
165 170 175

Ala Ala Glu Arg Arg Arg Met Arg Leu Val Tyr Ala Asp Thr Ile Lys
180 185 190

Asp Leu Leu Ala Asn Cys Ala Ile Gln Gly Asp Leu Glu Ser Arg Asp
195 200 205

Cys Ser Arg Met Val Pro Ala Glu Lys Thr Arg Val Glu Ser Val Glu
210 215 220

Leu Val Leu Pro Pro His Ala Asn His Gln Gly Asn Thr Phe Gly Gly
225 230 235 240

Gln Ile Met Ala Trp Met Glu Asn Val Ala Thr Ile Ala Ala Ser Arg
245 250 255

Leu Cys Arg Ala His Pro Thr Leu Lys Ala Ile Glu Met Phe His Phe
260 265 270

Arg Gly Pro Ser Gln Val Gly Asp Arg Leu Val Leu Lys Ala Ile Val
275 280 285

Asn Asn Ala Phe Lys His Ser Met Glu Val Gly Val Cys Val Glu Ala
290 295 300

Tyr Arg Gln Glu Ala Glu Thr His Arg Arg His Ile Asn Ser Ala Phe
305 310 315 320

Met Thr Phe Val Val Leu Asp Ala Asp Asp Gln Pro Gln Leu Leu Pro
325 330 335

Trp Ile Arg Pro Gln Pro Gly Asp Gly Glu Arg Arg Tyr Arg Glu Ala
340 345 350

Ser Ala Arg Lys Lys Ile Arg Leu Asp Arg Lys Tyr Ile Val Ser Cys
355 360 365

Lys Gln Thr Glu Val Pro Leu Ser Val Pro Trp Asp Pro Ser Asn Gln
370 375 380

Val Tyr Leu Ser Tyr Asn Asn Val Ser Ser Leu Lys Met Leu Val Ala
385 390 395 400

Lys Asp Asn Trp Val Leu Ser Ser Glu Ile Ser Gln Val Arg Leu Tyr
405 410 415

Thr Leu Glu Asp Asp Lys Phe Leu Ser Phe His Met Glu Met Val Val
420 425 430

His Val Asp Ala Ala Gln Ala Phe Leu Leu Leu Ser Asp Leu Arg Gln
435 440 445

Arg Pro Glu Trp Asp Lys His Tyr Arg Ser Val Glu Leu Val Gln Gln
450 455 460

Val Asp Glu Asp Asp Ala Ile Tyr His Val Thr Ser Pro Ala Leu Gly
465 470 475 480

Gly His Thr Lys Pro Gln Asp Phe Val Ile Leu Ala Ser Arg Arg Lys
485 490 495

Pro Cys Asp Asn Gly Asp Pro Tyr Val Ile Ala Leu Arg Ser Val Thr
500 505 510

Leu Pro Thr His Arg Glu Thr Pro Glu Tyr Arg Arg Gly Glu Thr Leu
515 520 525

Cys Ser Gly Phe Cys Leu Trp Arg Glu Gly Asp Gln Leu Thr Lys Cys
530 535 540

Cys Trp Val Arg Val Ser Leu Thr Glu Leu Val Ser Ala Ser Gly Phe
545 550 555 560

Tyr Ser Trp Gly Leu Glu Ser Arg Ser Lys Gly Arg Arg Ser Asp Gly
565 570 575

Trp Asn Gly Lys Leu Ala Gly Gly His Leu Ser Thr Leu Lys Ala Ile
580 585 590

Pro Val Ala Lys Ile Asn Ser Arg Phe Gly Tyr Leu Gln Asp Thr
595 600 605

<210> 3
<211> 1818
<212> DNA
<213> Homo sapiens

<400> 3

gtggaattgc cctttgcatc aaatgatcca gaatgtcgga aatcacctgc gacggggctt 60
ggcctctgtg ttctccaacc gcacatcccg gaagtcagcc ttacgtgcgg ggaacgacag 120
tgccatggca gacggcgagg gataccggaa ccccacggag gtgcagatga gccagctggt 180
gctgccctgc cacaccaacc aacgtggtga gctgagcgtc gggcagctgc tcaagtggat 240
tgacaccacg gcttgcctgt ccgcggagag gcacgctggc tgcccctgtg tcacagcttc 300
catggatgac atctattttg agcacaccat tagtgttgga caagtggtga atatcaaggc 360
caaggtgaac cgggccttca actccagcat ggaggtgggc atccaggtgg cctcggagga 420
cctgtgctct gagaagcagt ggaatgtgtg caaggccttg gccaccttcg tggcccgccg 480
agagatcacc aaggtgaagc tgaagcagat cacgccgcgg acagaagagg agaagatgga 540
gcacagtgtg gcggctgagc gccggcgcat gcgccttgtc tatgcagaca ccatcaagga 600
cctcctggcc aactgcgcca ttcagggcga tctggagagc agagactgta gccgcatggt 660
gccggctgag aagacccgtg tggagagtgt ggagctggtc ctgcctcccc acgccaatca 720
ccagggcaac acctttgggg gccagatcat ggcctggatg gagaatgtgg ccaccattgc 780
agccagccgg ctctgccgtg cccaccctac gctgaaggcc attgaaatgt tccacttccg 840
aggcccgtcc caggtcggcg accgtctggt gctcaaagcc atcgtgaaca atgccttcaa 900
acatagcatg gaggtgggcg tgtgcgtgga ggcctatcgc caggaggctg agacccaccg 960
gcgccacatc aacagtgcct ttatgacctt tgtggtcctg gacgcagatg accagcccca 1020
gttgctgccc tggattcggc cccagcccgg cgatggtgag cggcggtacc gagaggccag 1080
tgccagaaag aagatccgcc tggacaggaa gtacatcgtg tcctgtaagc agacagaggt 1140
gcccctctcc gtcccctggg accctagcaa ccaggtgtac ctgagctaca ataacgtctc 1200
ctccttgaag atgcttgtgg ccaaggacaa ctgggtgctg tcctcggaga tcagtcaggt 1260
ccgcctgtac actctggagg atgacaagtt cctctccttc cacatggaga tggtggtgca 1320
tgtggatgca gcccaggcct tcctgctgct ctcggacctg cgtcagaggc cagagtggga 1380
caagcactac cggagcgtgg agctagtgca gcaggtagac gaggacgacg ccatctacca 1440
cgtcaccagc cctgccctcg gaggtcacac aaagccccag gacttcgtga tcctggcctc 1500
gaggcggaag ccttgtgaca atggggaccc ctatgtcatc gcgctgaggt cggtcacgct 1560
gcccacacac cgagagacgc cagagtacag acgcggagag accctctgct caggcttctg 1620
cctctggcgc gagggggacc agctgaccaa ggtatcctac tacaaccagg ccaccccagg 1680
tgttctcaac tatgtgacca ccaacgtggc cggcctctcc tctgagttct acaccacctt 1740
caaggcttgt gagcagtttc tcttggacaa ccggaatgat ctggccccca gcctccagac 1800

cctctagatg ccctcagc 1818

<210> 4
<211> 594

<212> PRT
<213> Homo sapiens

<400> 4

```
Met Ile Gln Asn Val Gly Asn His Leu Arg Arg Gly Leu Ala Ser Val
1               5                   10                  15

Phe Ser Asn Arg Thr Ser Arg Lys Ser Ala Leu Arg Ala Gly Asn Asp
            20                  25                  30

Ser Ala Met Ala Asp Gly Glu Gly Tyr Arg Asn Pro Thr Glu Val Gln
        35                  40                  45

Met Ser Gln Leu Val Leu Pro Cys His Thr Asn Gln Arg Gly Glu Leu
    50                  55                  60

Ser Val Gly Gln Leu Leu Lys Trp Ile Asp Thr Thr Ala Cys Leu Ser
65                  70                  75                  80

Ala Glu Arg His Ala Gly Cys Pro Cys Val Thr Ala Ser Met Asp Asp
                85                  90                  95

Ile Tyr Phe Glu His Thr Ile Ser Val Gly Gln Val Val Asn Ile Lys
            100                 105                 110

Ala Lys Val Asn Arg Ala Phe Asn Ser Ser Met Glu Val Gly Ile Gln
        115                 120                 125

Val Ala Ser Glu Asp Leu Cys Ser Glu Lys Gln Trp Asn Val Cys Lys
    130                 135                 140

Ala Leu Ala Thr Phe Val Ala Arg Arg Glu Ile Thr Lys Val Lys Leu
145                 150                 155                 160

Lys Gln Ile Thr Pro Arg Thr Glu Glu Glu Lys Met Glu His Ser Val
                165                 170                 175

Ala Ala Glu Arg Arg Arg Met Arg Leu Val Tyr Ala Asp Thr Ile Lys
            180                 185                 190

Asp Leu Leu Ala Asn Cys Ala Ile Gln Gly Asp Leu Glu Ser Arg Asp
        195                 200                 205

Cys Ser Arg Met Val Pro Ala Glu Lys Thr Arg Val Glu Ser Val Glu
```

210 215 220

Leu Val Leu Pro Pro His Ala Asn His Gln Gly Asn Thr Phe Gly Gly
225 230 235 240

Gln Ile Met Ala Trp Met Glu Asn Val Ala Thr Ile Ala Ala Ser Arg
245 250 255

Leu Cys Arg Ala His Pro Thr Leu Lys Ala Ile Glu Met Phe His Phe
260 265 270

Arg Gly Pro Ser Gln Val Gly Asp Arg Leu Val Leu Lys Ala Ile Val
275 280 285

Asn Asn Ala Phe Lys His Ser Met Glu Val Gly Val Cys Val Glu Ala
290 295 300

Tyr Arg Gln Glu Ala Glu Thr His Arg Arg His Ile Asn Ser Ala Phe
305 310 315 320

Met Thr Phe Val Val Leu Asp Ala Asp Asp Gln Pro Gln Leu Leu Pro
325 330 335

Trp Ile Arg Pro Gln Pro Gly Asp Gly Glu Arg Arg Tyr Arg Glu Ala
340 345 350

Ser Ala Arg Lys Lys Ile Arg Leu Asp Arg Lys Tyr Ile Val Ser Cys
355 360 365

Lys Gln Thr Glu Val Pro Leu Ser Val Pro Trp Asp Pro Ser Asn Gln
370 375 380

Val Tyr Leu Ser Tyr Asn Asn Val Ser Ser Leu Lys Met Leu Val Ala
385 390 395 400

Lys Asp Asn Trp Val Leu Ser Ser Glu Ile Ser Gln Val Arg Leu Tyr
405 410 415

Thr Leu Glu Asp Asp Lys Phe Leu Ser Phe His Met Glu Met Val Val
420 425 430

His Val Asp Ala Ala Gln Ala Phe Leu Leu Leu Ser Asp Leu Arg Gln
435 440 445

Arg Pro Glu Trp Asp Lys His Tyr Arg Ser Val Glu Leu Val Gln Gln
450 455 460

Val Asp Glu Asp Asp Ala Ile Tyr His Val Thr Ser Pro Ala Leu Gly
465 470 475 480

Gly His Thr Lys Pro Gln Asp Phe Val Ile Leu Ala Ser Arg Arg Lys
485 490 495

Pro Cys Asp Asn Gly Asp Pro Tyr Val Ile Ala Leu Arg Ser Val Thr
500 505 510

Leu Pro Thr His Arg Glu Thr Pro Glu Tyr Arg Arg Gly Glu Thr Leu
515 520 525

Cys Ser Gly Phe Cys Leu Trp Arg Glu Gly Asp Gln Leu Thr Lys Val
530 535 540

Ser Tyr Tyr Asn Gln Ala Thr Pro Gly Val Leu Asn Tyr Val Thr Thr
545 550 555 560

Asn Val Ala Gly Leu Ser Ser Glu Phe Tyr Thr Thr Phe Lys Ala Cys
565 570 575

Glu Gln Phe Leu Leu Asp Asn Arg Asn Asp Leu Ala Pro Ser Leu Gln
580 585 590

Thr Leu

<210> 5
<211> 2699
<212> DNA
<213> Mus musculus

<400> 5

ctagagatcc ctcgacctcg acccacgcgt ccgagacccc cccacagcct ggcaacccag 60
caaacggagc agcaatgatt cagaatgtgg gcaaccactt gcgaaggggc ttcgcctcta 120
tgttctctaa tcgcacatcc cggaagtcaa tctcccatcc ggagtctgga gaccctccta 180
ccatggcaga gggtgaagga taccggaacc ccacggaggt gcagatgagc cagctggtac 240
tgccctgcca caccaaccac cgtggggagc tgagcattgg acagttgctc aagtggatcg 300
acaccacagc ctgcctatca gcggagaggc atgctggctg tccctgcgtc acagcctcta 360
tggatgacat ctacttcgac cataccatta gtgtcggcca agtggtgaat atcaaggcca 420
aggtgaaccg ggccttcaac tccagcatgg aggtgggaat ccaggtggtc tctgaggatc 480
tgtgctctga gaagcagtgg agtgtctgca aggccttggc cacctttgtg gcccaccggg 540
agctctccaa ggtgaagctg aagcaggtca tcccattgac cgaggaggag aagactgaac 600
atggggtggc ggctgagcgc cggcgtatgc gactggtcta tgcagacacc atcaaagatc 660

tcctaaccca ctgtgtcatc caggacgatt tggacaagga ctgcagcaat atggtgccag 720
ccgagaagac ccgagtggag agtgtggagc tggtgctgcc tcctcacgcc aatcatcagg 780
gcaatacctt cgggggacag atcatggctt ggatggagaa tgtggccacc attgcagcca 840
gccggctctg tcacgcccac cctacgctca aggccatcga gatgttccat ttccgaggcc 900
cgtctcaggt gggggaccgt ctggtgctca aggccatcgt gaataacgcc tttaagcaca 960
gcatggaggt gggtgtgtgt gtggaggcgt accgccagga agctgagacc cagcgccggc 1020
acatcaacag cgccttcatg accttcgtgg tcctggacaa agatgaccag cctcagaagc 1080
tgccctggat tcgtccccag cctggagagg gtgaacggcg ataccgagaa gccagtgcca 1140
ggaagaagat ccgcctggac aggaaatacc ttgtgtcctg taagcaggca gaagtggccc 1200
tgtctgtccc ctgggaccct agcaaccagg tatacctgag ctactacaac gtgtcctctc 1260
tgaagacgct catggccaag gacaactggg tgctgtccgt ggagatcagc gaggtccgcc 1320
tgtacatcct agaagaggac ttcctctcct ttcacttgga gatggtggta aatgtggatg 1380
ccgcccaggt ctttcagctg ctgtcagacc tgcgcaggag accagagtgg gacaagcatt 1440
accggagtgt ggagctggtg cagcaagtgg atgaggatga cgccatctac cacgtcatca 1500
gccccgccct gagcgggaac accaagcccc aggactttgt gatcctggcc tctaggcgga 1560
agccttgtga caatggggac ccctatgtca ttgccctgag gtcggtcacg ctgcccacgc 1620
accatgagac accggaatac caacgtgggg agactctctg ttcaggcttc tgtctgtggc 1680
gtgaggggga ccagatgact aaggtttctt actacaacca ggccaccccc ggctttctca 1740
actatgtgac caccaatgtg tccggcctgt cctcagaatt ctacaacact ttcaaggctt 1800
gtgagagttt tctgttggac aaccggaatg acctagctcc cagcctccag accctctaga 1860
caccacccat gctgccccag gtcttacaca gtgcgtggaa caaagcagag acatttattc 1920
accttgactc cccagggaag ccttccacac tagatggtcc aatcctactg gatggtcggt 1980
tgctgctcac atctgcctgc aagtctttcc agtactcctg ggatatcctg taatagactc 2040
gggtcctgtc cacggccctg gccgcccaca atccagccca caaatccaca tggctgttcc 2100
cagcagtgct gtggtacact gtgacagtgg ctctagggga ggaggccagg agcctggcca 2160
cagtgttggc tggactctga ctcagtggcc cagcctgcag ctggaaggac acaggttgcc 2220
ggagtccctg acacagctcc agcatatctg tgaccatctg ctcctgataa ccactgtcca 2280
gcatctcttc gggccagccc ggtgccacgg tcacatgggg gaagacttca gccacagctg 2340
tgagcagctc tctgccagct atgtggccag ggaccacaaa actaccatgt gagactgtgg 2400
accccaccca cacaggccag ggcagatggc caagggtaga aaggtgtgct aacgtggcca 2460
gggatggccg gagagcttcg ggttccacta tgttcacatg gatgccccag cgcctggggt 2520

gaacagccag acgcagcaga cacgactcca gtgtcagagc agggccacct ggggtgtgta 2580

ggatgggtac agggttccca gcctcaggtt cctggaggcc aatgtccagc aagatcatgc 2640

cttctctgtc tggaagaggc aacagtttgg agatcctgtc atcaaaaaa aaaaaaaaa 2699

<210> 6
<211> 594
<212> PRT
<213> Mus musculus

<400> 6

Met Ile Gln Asn Val Gly Asn His Leu Arg Arg Gly Phe Ala Ser Met
1 5 10 15

Phe Ser Asn Arg Thr Ser Arg Lys Ser Ile Ser His Pro Glu Ser Gly
20 25 30

Asp Pro Pro Thr Met Ala Glu Gly Glu Gly Tyr Arg Asn Pro Thr Glu
35 40 45

Val Gln Met Ser Gln Leu Val Leu Pro Cys His Thr Asn His Arg Gly
50 55 60

Glu Leu Ser Ile Gly Gln Leu Leu Lys Trp Ile Asp Thr Thr Ala Cys
65 70 75 80

Leu Ser Ala Glu Arg His Ala Gly Cys Pro Cys Val Thr Ala Ser Met
85 90 95

Asp Asp Ile Tyr Phe Asp His Thr Ile Ser Val Gly Gln Val Val Asn
100 105 110

Ile Lys Ala Lys Val Asn Arg Ala Phe Asn Ser Ser Met Glu Val Gly
115 120 125

Ile Gln Val Val Ser Glu Asp Leu Cys Ser Glu Lys Gln Trp Ser Val
130 135 140

Cys Lys Ala Leu Ala Thr Phe Val Ala His Arg Glu Leu Ser Lys Val
145 150 155 160

Lys Leu Lys Gln Val Ile Pro Leu Thr Glu Glu Glu Lys Thr Glu His
165 170 175

Gly Val Ala Ala Glu Arg Arg Arg Met Arg Leu Val Tyr Ala Asp Thr
180 185 190

Ile Lys Asp Leu Leu Thr His Cys Val Ile Gln Asp Asp Leu Asp Lys

195 200 205

Asp Cys Ser Asn Met Val Pro Ala Glu Lys Thr Arg Val Glu Ser Val
210 215 220

Glu Leu Val Leu Pro Pro His Ala Asn His Gln Gly Asn Thr Phe Gly
225 230 235 240

Gly Gln Ile Met Ala Trp Met Glu Asn Val Ala Thr Ile Ala Ala Ser
245 250 255

Arg Leu Cys His Ala His Pro Thr Leu Lys Ala Ile Glu Met Phe His
260 265 270

Phe Arg Gly Pro Ser Gln Val Gly Asp Arg Leu Val Leu Lys Ala Ile
275 280 285

Val Asn Asn Ala Phe Lys His Ser Met Glu Val Gly Val Cys Val Glu
290 295 300

Ala Tyr Arg Gln Glu Ala Glu Thr Gln Arg Arg His Ile Asn Ser Ala
305 310 315 320

Phe Met Thr Phe Val Val Leu Asp Lys Asp Asp Gln Pro Gln Lys Leu
325 330 335

Pro Trp Ile Arg Pro Gln Pro Gly Glu Gly Glu Arg Arg Tyr Arg Glu
340 345 350

Ala Ser Ala Arg Lys Lys Ile Arg Leu Asp Arg Lys Tyr Leu Val Ser
355 360 365

Cys Lys Gln Ala Glu Val Ala Leu Ser Val Pro Trp Asp Pro Ser Asn
370 375 380

Gln Val Tyr Leu Ser Tyr Tyr Asn Val Ser Ser Leu Lys Thr Leu Met
385 390 395 400

Ala Lys Asp Asn Trp Val Leu Ser Val Glu Ile Ser Glu Val Arg Leu
405 410 415

Tyr Ile Leu Glu Glu Asp Phe Leu Ser Phe His Leu Glu Met Val Val
420 425 430

Asn Val Asp Ala Ala Gln Val Phe Gln Leu Leu Ser Asp Leu Arg Arg
435 440 445

Arg Pro Glu Trp Asp Lys His Tyr Arg Ser Val Glu Leu Val Gln Gln
450 455 460

Val Asp Glu Asp Asp Ala Ile Tyr His Val Ile Ser Pro Ala Leu Ser
465 470 475 480

Gly Asn Thr Lys Pro Gln Asp Phe Val Ile Leu Ala Ser Arg Arg Lys
485 490 495

Pro Cys Asp Asn Gly Asp Pro Tyr Val Ile Ala Leu Arg Ser Val Thr
500 505 510

Leu Pro Thr His His Glu Thr Pro Glu Tyr Gln Arg Gly Glu Thr Leu
515 520 525

Cys Ser Gly Phe Cys Leu Trp Arg Glu Gly Asp Gln Met Thr Lys Val
530 535 540

Ser Tyr Tyr Asn Gln Ala Thr Pro Gly Phe Leu Asn Tyr Val Thr Thr
545 550 555 560

Asn Val Ser Gly Leu Ser Ser Glu Phe Tyr Asn Thr Phe Lys Ala Cys
565 570 575

Glu Ser Phe Leu Leu Asp Asn Arg Asn Asp Leu Ala Pro Ser Leu Gln
580 585 590

Thr Leu

<210> 7
<211> 19
<212> DNA
<213> artificial sequence

<220>
<223> primer oligonucleotide

<400> 7
tgaaggatac cggaacccc 19

<210> 8
<211> 23
<212> DNA
<213> Artificial sequence

<220>
<223> probe oligonucleotide

<400> 8
cggaggtgca gatgagccag ctg 23

<210> 9
<211> 19

<212> DNA
<213> Artificial

<220>
<223> primer oligonucleotide

<400> 9
tactgccctg ccacaccaa 19

<210> 10
<211> 20
<212> DNA
<213> Artificial sequence

<220>
<223> primer oligonucleotide

<400> 10
tgctgggtta gggtctccct 20

<210> 11
<211> 24
<212> DNA
<213> Artificial

<220>
<223> probe oligonucleotide

<400> 11
actgagctgg tctcggcaag tggc 24

<210> 12
<211> 19
<212> DNA
<213> Artificial

<220>
<223> primer oligonucleotide

<400> 12
tctattcctg ggggctcga 19

<210> 13
<211> 21
<212> DNA
<213> Artificial

<220>
<223> primer oligonucleotide

<400> 13
tctcttggac aaccggaatg a 21

<210> 14
<211> 20
<212> DNA
<213> artificial

<220>
<223> probe oligonucleotide

<400> 14
tggcccccag cctccagacc 20

<210> 15
<211> 20
<212> DNA
<213> Artificial

<220>
<223> primer oligonucleotide

<400> 15
tctagatgcc ctcagtggcc 20

<210> 16
<211> 20
<212> DNA
<213> Artificial

<220>
<223> primer oligonucleotide

<400> 16
gtaagaaggg agcctgggag 20

<210> 17
<211> 20
<212> DNA
<213> Artificial

<220>
<223> primer oligonucleotide

<400> 17
tctagaccac cctttctccg 20

<210> 18
<211> 22
<212> DNA
<213> Artificial

<220>
<223> primer oligonucleotide

<400> 18
ccagaccctc tagatgccct ca 22

<210> 19
<211> 24
<212> DNA
<213> Artificial

<220>
<223> primer oligonucleotide

<400> 19
atgatccaga atgtcggaaa tcac 24

<210> 20
<211> 26
<212> DNA
<213> Artificial

<220>
<223> primer oligonucleotide

<400> 20
agacacctga aaccttatca tgagcc 26

<210> 21
<211> 25
<212> DNA
<213> Artificial

<220>
<223> primer oligonucleotide

<400> 21
gccactgagt cagagtccag ccaac 25

<210> 22
<211> 21
<212> DNA
<213> Artificial

<220>
<223> primer oligonucleotide

<400> 22
ccagcctgca gctggaagga c 21

<210> 23
<211> 47
<212> DNA
<213> Artificial

<220>
<223> probe oligonucleotide

<400> 23
aaatccacat ggctgttccc agcagtgctg tggtacactg tgacagt 47